# EUROPÄISCHE PATENTSCHRIFT

(11) **EP 1 926 727 B1**
(45) Veröffentlichungstag und Bekanntmachung des Hinweises auf die Patenterteilung: **14.09.2011**
(21) Anmeldenummer: 06762589.7
(22) Anmeldetag: 14.07.2006
(51) Int. Cl.: C07D 405/04, A61K 31/445, A61P 9/00

(54) **4-CHROMENONYL-1,4-DIHYDROPYRIDINCARBONITRILE UND IHRE VERWENDUNG**
4-CHROMENONYL-1,4-DIHYDROPYRIDINECARBONITRILES AND THE USE THEREOF
4-CHROMENONYL-1,4-DIHYDROPYRIDINCARBONITRILES ET LEUR UTILISATION

(30) Priorität: 22.07.2005 DE 102005034264
(43) Veröffentlichungstag der Anmeldung: 04.06.2008
(73) Patentinhaber: Bayer Pharma Aktiengesellschaft, 13353 Berlin (DE)
(72) Erfinder: KUHL, Alexander, 58093 Hagen (DE); KOLKHOF, Peter, 42113 Wuppertal (DE); HECKROTH, Heike, 51519 Odenthal (DE); SCHLEMMER, Karl-Heinz, 42113 Wuppertal (DE); FLAMME, Ingo, 51580 Reichshof (DE); FIGUEROA PEREZ, Santiago, 51373 Leverkusen (DE); GIELEN-HAERTWIG, Heike, 40789 Monheim (DE); GROSSER, Rolf, 51373 Leverkusen (DE); ERGÜDEN, Jens-Kerim, 42489 Wülfrath (DE); LANG, Dieter, 42553 Velbert (DE)
(86) Internationale Anmeldenummer: PCT/EP2006/006905
(87) Internationale Veröffentlichungsnummer: WO 2007/009670

(56) Entgegenhaltungen:
- EP-A2- 0 123 112
- EP-A2- 0 223 744
- DE-A1- 3 311 003
- BUDRIESI R ET AL: "1,4-Dihydropyridine derivatives as calcium channel modulators: the role of 3-methoxy-flavone moiety" BIOORGANIC & MEDICINAL CHEMISTRY, ELSEVIER SCIENCE LTD, GB, Bd. 13, Nr. 10, 16. Mai 2005 (2005-05-16), Seiten 3423-3430, XP004859479 ISSN: 0968-0896

## Beschreibung

Die vorliegende Anmeldung betrifft neue 4-Chromenonyl-1,4-dihydropyridincarbonitrile, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären Erkrankungen.

Aldosteron spielt eine Schlüsselrolle in der Aufrechterhaltung der Flüssigkeits- und Elektrolythomöostase, indem es im Epithel des distalen82 Nephrons die Natriumretention und Kaliumsekretion fördert, was zur Konstanthaltung des Extrazellulärvolumens und damit zur Blutdruckregulation beiträgt. Daneben entfaltet Aldosteron direkte Effekte auf die Struktur und Funktion des Herz- und Gefäßsystems, wobei die dafür zugrunde liegenden Mechanismen noch nicht erschöpfend geklärt sind [R.E. Booth, J.P. Johnson, J.D. Stockand, Adv. Physiol. Educ. 26 (1), 8-20 (2002)].

Aldosteron ist ein Steroidhormon, das in der Nebennierenrinde gebildet wird. Seine Produktion wird indirekt ganz wesentlich in Abhängigkeit von der Nierendurchblutung reguliert. Jede Abnahme der Nierendurchblutung führt in der Niere zu einer Ausschüttung des Enzyms Renin in den Blutkreislauf. Dieses wiederum aktiviert die Bildung von Angiotensin II, das einerseits verengend auf die arteriellen Blutgefäße wirkt, andererseits aber auch die Bildung von Aldosteron in der Nebennierenrinde stimuliert. Somit fungiert die Niere als Blutdruck- und damit indirekter Volumen-Sensor im Blutkreislauf und wirkt über das Renin-Angiotensin-Aldosteron-System kritischen Volumenverlusten entgegen, indem einerseits der Blutdruck gesteigert wird (Angiotensin II-Wirkung), andererseits durch verstärkte Rückresorption von Natrium und Wasser in der Niere der Füllungszustand des Gefäßsystems wieder ausgeglichen wird (Aldosteron-Wirkung).

Dieses Regelsystem kann in vielfältiger Weise krankhaft gestört sein. So führt eine chronische Minderdurchblutung der Nieren (z.B. infolge einer Herzinsuffizienz und des hierdurch verursachten Blutrückstaus im Venensystem) zu einer chronisch überhöhten Ausschüttung von Aldosteron. Diese wiederum zieht eine Expansion des Blutvolumens nach sich und verstärkt hiermit die Herzschwäche durch ein Volumenüberangebot an das Herz. Eine Stauung von Blut in den Lungen mit Atemnot und Ödembildung in den Extremitäten sowie Aszites und Pleuraergüsse können die Folge sein; die Nierendurchblutung sinkt weiter ab. Überdies führt die übersteigerte Aldosteron-Wirkung zu einer Minderung der Kalium-Konzentration im Blut und in der Extrazellulärflüssigkeit. In ohnehin vorgeschädigten Herzmuskeln kann die Unterschreitung eines kritischen Mindestwertes tödlich endende Herzrhythmusstörungen auslösen. Hierin dürfte eine der Hauptursachen des häufig bei Patienten mit Herzinsuffizienz eintretenden *plötzlichen Herztodes* zu suchen sein.

Zusätzlich wird Aldosteron auch für eine Reihe der typischerweise bei Herzinsuffizienten zu beobachtenden Umbauprozesse des Herzmuskels verantwortlich gemacht. Somit ist der *Hyperaldosteronismus* eine entscheidende Komponente in der Pathogenese und Prognose der Herzinsuffizienz, die ursprünglich durch unterschiedliche Schädigungen, wie z.B. einen Herzinfarkt, eine Herzmuskelentzündung oder Bluthochdruck, ausgelöst werden kann. Diese Annahme wird durch die Tatsache erhärtet, dass in umfangreichen klinischen Studien in Patientenkollektiven mit chronischer Herzinsuffizienz bzw. nach akutem Myokardinfarkt durch Anwendung von Aldosteron-Antagonisten die Gesamtmortalität deutlich gesenkt wurde [B. Pitt, F. Zannad, W.J. Remme et al., N. Engl. J. Med. 341, 709-717 (1999); B. Pitt, W. Remme, F. Zannad et al., N. Engl. J. Med. 348, 1309-1321 (2003)]. Dies konnte unter anderem durch eine Senkung der Inzidenz des plötzlichen Herztodes erreicht werden.

Neueren Studien zufolge findet man auch bei einem nicht unerheblichen Teil der Patienten, die unter einer essentiellen Hypertonie leiden, eine unphysiologische Erhöhung der Aldosteron-Konzentration im Plasma [N.M. Kaplan, The current epidemic of primary aldosteronism: Causes and consequences, J. Hypertens. 22, 863-869 (2004)]. Worin die Ursache dieses Hyperaldosteronismus liegt und ob die Betroffenen eine spezielle Risikogruppe bezüglich des Versterbens am plötzlichen Herztod oder der Entwicklung einer Herzinsuffizienz darstellen, ist nicht bekannt. Es ist jedoch anzunehmen, dass ein im Zusammenhang mit einer essentiellen Hypertonie diagnostizierter Hyperäldosteronismus den Ansatzpunkt für eine kausale und prophylaktisch sinnvolle Therapie bietet.

Ein anderes typischerweise mit Erhöhung der Aldosteron-Konzentration im Plasma einhergehendes Krankheitsbild ist die fortgeschrittene Leberzirrhose. Die Ursache der Aldosteronerhöhung liegt hier vorwiegend im infolge der Leberfunktionsstörung eingeschränkten Abbau des Aldosterons. Volumenüberlastung, Ödeme und Hypokaliämie sind die typischen Folgen, die in der klinischen Praxis durch Aldosteron-Antagonisten erfolgreich gelindert werden können.

Weitaus seltener als die oben aufgeführte Formen des Hyperaldosteronismus sind solche Krankheitsbilder, bei denen die Störung entweder in den Hormon-produzierenden Zellen der Nebenniere selbst zu finden ist oder deren Anzahl oder Masse durch eine Hyperplasie oder Wucherung vermehrt ist. Adenome oder diffuse Hyperplasien der Nebennierenrinde sind die häufigste Ursache des auch als *Conn-Syndrom* bezeichneten primären Hyperaldosteronismus. Auch hier steht neben der chirurgischen Entfernung des krankhaften Gewebes die medikamentöse Therapie mit Aldosteron-Antagonisten im Vordergrund [H.A. Kühn, und J. Schirmeister (Hrsg.), Innere Medizin, 4. Aufl., Springer Verlag, Berlin, 1982].

Die Wirkungen von Aldosteron werden über den in den Zielzellen intrazellulär lokalisierten Mineralokorticoid-Rezeptor vermittelt. Die bislang verfügbaren Aldosteron-Antagonisten besitzen wie Aldosteron selbst eine Steroid-Grundstruktur. Begrenzt wird die Anwendbarkeit derartiger steroidaler Antagonisten durch ihre Wechselwirkungen mit den Rezeptoren anderer Steroidhormone, die zum Teil zu erheblichen Nebenwirkungen wie Gynäkomastie und Impotenz und zum Abbrechen der Therapie führen [M.A. Zaman, S. Oparil, D.A. Calhoun, Nature Rev. Drug Disc. 1, 621-636 (2002)].

Die Anwendung wirkstarker, nicht-steroidaler und für den Mineralokorticoid-Rezeptor selektiver Antagonisten bietet die Möglichkeit, dieses Nebenwirkungsprofil zu umgehen und dadurch einen deutlichen Therapievorteil zu erzielen.

Aufgabe der vorliegenden Erfindung ist die Bereitstellung neuer Verbindungen, die als selektive Mineralokorticoid-Rezeptor-Antagonisten für die Behandlung von Erkrankungen, insbesondere von kardiovaskulären Erkrankungen, eingesetzt werden können.

Chromon- und Thiochromon-substituierte 1,4-Dihydropyridine werden in DE 3 311 003-A1 und DE 3 311 005-A1 als Cardiotonika und Antihypotonika beschrieben. Koronarwirksame 4-Aryl-1,4-dihydropyridin-Derivate sind in DE 2 003 146 offenbart. In EP 0 223 744-A2 werden 2-Phenylchromon-substituierte 1,4-Dihydropyridindiester als Calcium-Antagonisten beansprucht. Über 4-Xanthenonyl-1,4-dihydropyridine mit Calcium-antagonistischer Aktivität wird in Arzneim. Forsch. 42 (6), 797-801 (1992) berichtet.

Gegenstand der vorliegenden Erfindung sind Verbindungen der allgemeinen Formel (I) 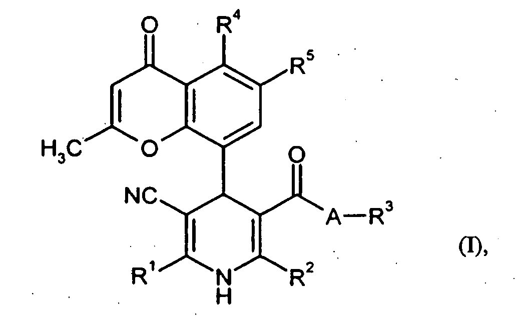 in welcher
- R¹ und R²: gleich oder verschieden sind und unabhängig voneinander für (C₁-C₄)-Alkyl, Trifluor- methyl, Cyclopropyl, oder Cyclobutyl stehen,
- A: für eine Bindung oder für O steht,
- R³: für (C₃-C₇)-Cycloalkyl oder für (C₁-C₆)-Alkyl, das mit (C₂-C₇)-Cycloalkyl oder ein- bis dreifach mit Fluor substituiert sein kann, steht,
- R⁴: für Wasserstoff, Halogen, Cyano, Nitro, Trifluormethyl, (C₁-C₄)-Alkyl oder (C₁-C₄)- Alkoxy steht
und
- R⁵: für Wasserstoff oder Fluor steht,
sowie ihre Salze, Solvate und Solvate der Salze.

Erfindungsgemäße Verbindungen sind die Verbindungen, der Formel (I) und deren Salze; Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
(C₃-C₇)-Cycloalkyl und (C₃-C₅)-Cycloalkyl stehen im Rahmen der Erfindung für eine gesättigte monocyclische Cycloalkylgruppe mit 3 bis 7 bzw. 3 bis 5 Kohlenstoffatomen. Bevorzugt ist ein Cycloalkylrest mit 3 bis 5 Kohlenstoffatomen. Beispielhaft und vorzugsweise seien genannt: Cyclopropyl, Cyclobutyl, Cyclopentyl, Cyclohexyl und Cycloheptyl.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, n-Propoxy, Isopropoxy, n-Butoxy und tert.-Butoxy.
Halogen schließt im Rahmen der Erfindung Fluor, Chlor, Brom und Iod ein. Bevorzugt sind Fluor oder Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- R¹ und R²: gleich oder verschieden sind und für Methyl oder Trifluormethyl stehen,
- A: für eine Bindung oder für O steht,
- R³: für (C₃-C₅)-Cycloalkyl oder für (C₁-C₆)-Alkyl, das mit (C₃-C₅)-Cycloalkyl oder ein- bis dreifach mit Fluor substituiert sein kann, steht,
- R⁴: für Wasserstoff, Fluor, Chlor, Cyano, Nitro oder Methyl steht
und
- R⁵: für Wasserstoff oder Fluor steht,
sowie ihre Salze, Solvate und Solvate der Salze.

Besonders bevorzugt sind Verbindungen der Formel (I), in welcher
- R¹: für Methyl oder Trifluormethyl steht,
- R²: für Methyl steht,
- A: für O steht,
- R³: für Ethyl, 2,2,2-Trifluorethyl, n-Propyl, Isopropyl, 1-(Trifluormethyl)-ethyl, tert.-Butyl, Cyclobutyl, Cyclopentyl, Cyclopropylmethyl oder Cyclobutylmethyl steht,
- R⁴: für Wasserstoff, Fluor, Chlor oder Nitro steht
und
- R⁵: für Wasserstoff oder Fluor steht,
sowie ihre Salze, Solvate und Solvate der Salze.

Besonders bevorzugt sind auch Verbindungen der Formel (I), in welcher
- R¹: für Methyl oder Trifluormethyl steht,
- R²: für Methyl steht,
- A: für eine Bindung steht,
- R³: für Isobutyl, Isopentyl, Cyclobutylmethyl, Cyclopentylmethyl, 2-(Cyclopropyl)-ethyl, 2- (Cyclobutyl)-ethyl oder 2-(Cyclopentyl)-ethyl steht,
- R⁴: für Wasserstoff, Fluor, Chlor oder Nitro steht
und
- R⁵: für Wasserstoff oder Fluor steht,
sowie ihre Salze, Solvate und Solvate der Salze.

Von besonderer Bedeutung sind Verbindungen der Formel (I-A) 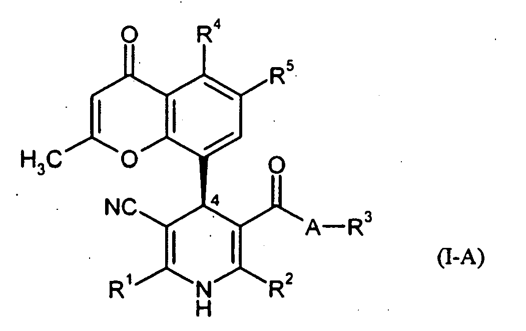 mit S-Konfiguration in Position 4 des Dihydropyridin-Rings,
in welcher A, R¹, R², R³, R⁴ und R⁵ jeweils die oben angegebenen Bedeutungen aufweisen,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man Verbindungen der Formel (II) 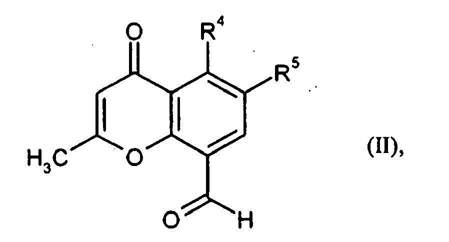 in welcher R⁴ und R⁵ jeweils die oben angegebenen Bedeutungen haben,
entweder
[A] in einem einstufigen Verfahren (Eintopf-Reaktion) mit einer Verbindung der Formel (III) 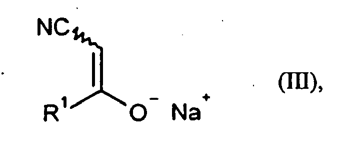 in welcher R¹ die oben angegebenen Bedeutungen hat,
   und einer Verbindung der Formel (IV) 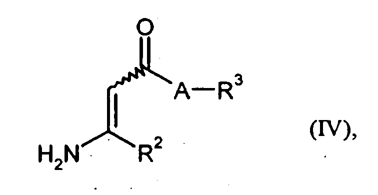 in welcher A, R² und R³ jeweils die oben angegebenen Bedeutungen haben, umsetzt oder
[B] in einem einstufigen Verfahren (Eintopf-Reaktion) mit einer Verbindung der Formel (V) 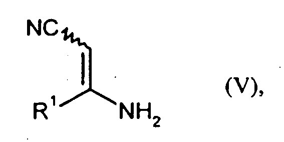 in welcher R¹ die oben angegebenen Bedeutungen hat,
   und einer Verbindung der Formel (VI) 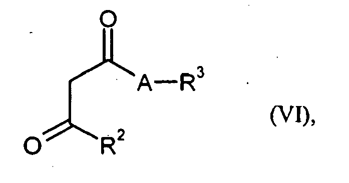 in welcher A, R² und R³ jeweils die oben angegebenen Bedeutungen haben, umsetzt
   oder
[C] in einem zweistufigen Verfahren zunächst mit einer Verbindung der Formel (III) in Verbindungen der Formel (VII) 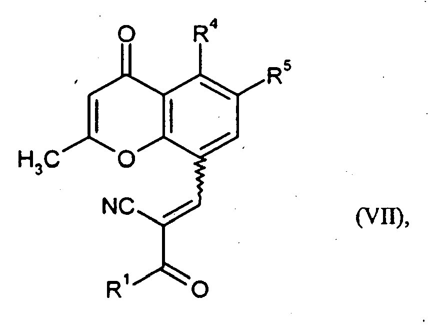 in welcher R¹, R⁴ und R⁵ jeweils die oben angegebenen Bedeutungen haben,
   überführt und diese dann in einem zweiten Schritt mit einer Verbindung der Formel (IV) umsetzt
   oder
[D] in einem zweistufigen Verfahren zunächst mit einer Verbindung der Formel (VI) in Verbindungen der Formel (VII) 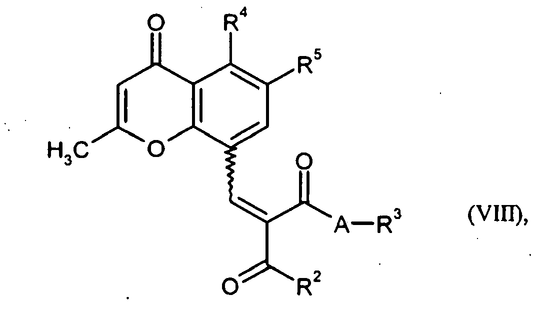 in welcher A, R², R³, R⁴ und R⁵ jeweils die oben angegebenen Bedeutungen haben,
   überführt und diese dann in einem zweiten Schritt mit einer Verbindung der Formel (V) umsetzt,
die resultierenden Verbindungen der Formel (I) gegebenenfalls nach dem Fachmann bekannten Methoden in ihre Enantiomere und/oder Diastereomere trennt und die Verbindungen der Formel (I) bzw. (I-A) gegebenenfalls mit den entsprechenden (i) Lösungsmitteln und/oder (ii) Basen oder Säuren in ihre Solvate, Salze und/oder Solvate der Salze überfährt.

In diesen Verfahrensvarianten können gegebenenfalls für die Gruppierung -C(O)-A-R³, worin A für O steht, zunächst auch leicht spaltbare Carbonsäureester eingesetzt werden, die dann nach dem Fachmann bekannten Methoden gespalten und mit den entsprechenden Alkoholen zu den Verbindungen der Formel (I) umgesetzt werden.

Die Umsetzungen nach Verfahren [A] und [B] sowie der zweiten Stufe der Verfahren [C] und [D] erfolgen im Allgemeinen in inerten Lösungsmitteln, gegebenenfalls in Gegenwart einer Säure oder Base, in einem Temperaturbereich von +20°C bis zum Siedepunkt des Lösungsmittels bei Normaldruck.

Inerte Lösungsmittel hierfür sind beispielsweise Alkohole wie Methanol, Ethanol, n-Propanol, Isopropanol, n-Butanol oder tert.-Butanol, oder andere Lösungsmittel wie Acetonitril, Tetrahydrofuran, Dioxan, 1,2-Dimethoxyethan, Toluol oder Eisessig. Bevorzugt werden die Umsetzungen in Ethanol oder Isopropanol bei der jeweiligen Rückfluss-Temperatur unter Normaldruck durchgeführt.

Die Umsetzungen nach Verfahren [A] und [B] werden bevorzugt in Gegenwart einer Säure wie beispielsweise Essigsäure, Trifluoressigsäure, p-Toluolsulfonsäure, Methansulfonsäure oder Tetrabutylammoniumhydrogensulfat durchgerührt; besonders bevorzugt ist der Zusatz von Essigsäure. Die Umsetzungen gemäß der zweiten Stufe der Verfahren [C] und [D] können gegebenenfalls unter Zusatz einer Base durchgerührt werden. Hierfür eignen sich beispielsweise Alkali- oder Erdalkalicarbonate wie Natrium-, Kalium- oder Cäsiumcarbonat, oder Alkoholate wie Natrium- oder Kaliummethanolat, Natrium- oder Kaliumethanolat oder Natrium- oder Kalium-tert.-butylat. Bevorzugt ist Kalium-tert.-butylat.

Die Umsetzungen gemäß der ersten Stufe der Verfahren [C] und [D] erfolgen im Allgemeinen in inerten Lösungsmitteln, gegebenenfalls in Gegenwart einer Base und/oder Säure, in einem Temperaturbereich von +20°C bis zum Siedepunkt des Lösungsmittels bei Normaldruck.

Als inerte Lösungsmittel eignen sich hierbei beispielsweise Halogenkohlenwasserstoffe wie Dichlormethan, Trichlormethan, Tetrachlormethan, Trichlorethan oder 1,2-Dichlorethan, oder andere Lösungsmittel wie Acetonitril, Pyridin, Benzol, Toluol, Chlorbenzol oder Hexan. Bevorzugt erfolgen die Umsetzungen in Dichlormethan oder Toluol bei der jeweiligen Rückfluss-Temperatur unter Normaldruck.

Die Umsetzungen gemäß der ersten Stufe der Verfahren [C] und [D] werden bevorzugt in Gegenwart einer Säure in Kombination mit Piperidin oder Pyridin als Base und/oder einem wasserentziehendem Mittel, wie beispielsweise Molekularsieb, durchgeführt. Als Säuren eignen sich beispielsweise Essigsäure oder p-Toluolsulfonsäure. Besonders bevorzugt ist eine Reaktionsfiihrung unter Zusatz von Piperidiniumacetat in Verbindung mit Molekularsieb.

Die Verbindungen der Formel (II) sind literaturbekannt oder können in Analogie zu literaturbekannten Verfahren beispielsweise durch Ozonolyse von Verbindungen der Formel (IX) 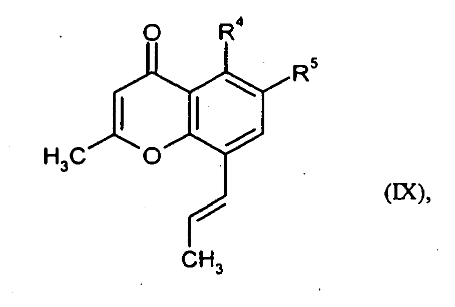 in welcher R⁴ und R⁵ jeweils die oben angegebenen Bedeutungen haben,
oder durch einfache oder doppelte Bromierung von Verbindungen der Formel (X) 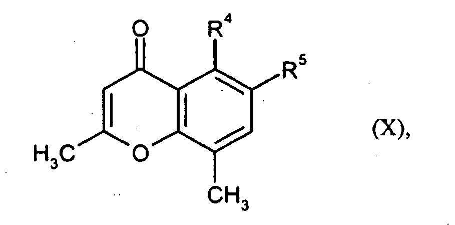 in welcher R⁴ und R⁵ jeweils die oben angegebenen Bedeutungen haben,
zu Verbindungen der Formel (XI) bzw. (XII) 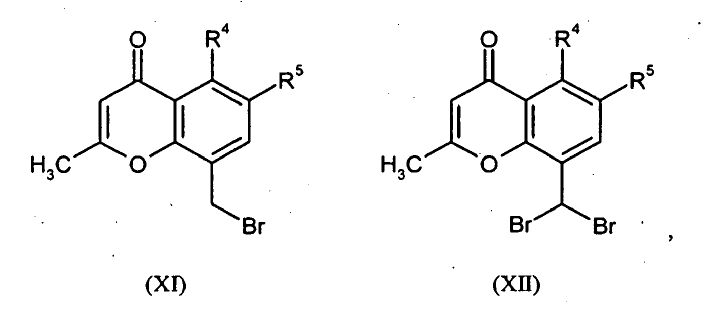 in welchen R⁴ und R⁵ jeweils die oben angegebenen Bedeutungen haben,
und nachfolgende Umsetzung mit *N*-Methylmorpholin-*N*-oxid hergestellt werden. Die Ausgangsverbindungen der Formeln (IX) und (X) sind literaturbekannt oder nach literaturbekannten Verfahren erhältlich [vgl. z.B. zu (IX) und der Umsetzung (IX) → (II): *a)* S.G. Jagadeesh et al., Synth. Commun. 31 (10), 1547-15.57 (2001); *b)* DE 3 311 005-A1 und dort zitierte Literatur; zu (X) und der Umsetzung (X) → (XI)/(XII) → (II): *a)* P. Babin et al., Tetrahedron 37, 1131-1139 (1981); *b)* H.J. Bestmann, G. Schade, Chem. Lett., 997-998 (1983); c) J.I. Ubeda et al., Heterocycles 38 2677-2690 (1994); *d)* R.J. Chambers et al., Bioorg. Med. Chem. Lett. 8, 3577-3582 (1998); siehe auch Schemata 1-3].

Die Verbindungen der Formeln (III), (IV), (V) und (VI) sind kommerziell erhältlich, literaturbekannt oder können nach literaturbekannten Methoden hergestellt werden [zur Herstellung von (IV), worin A für eine Bindung steht, siehe z.B. C. Kashima et al., Bull. Chem. Soc. Jpn. 46, 310-313 (1973); zur Synthese von 1,4-Dihydropyridinen vgl. auch D. M. Stout, A. I. Meyers, Chem. Rev. 1982, 82, 223-243; H. Meier et al., Liebigs Ann. Chem. 1977, 1888; H. Meier et al., Liebigs Ann. Chem. 1977, 1895 und H. Meier et al., Liebigs Ann. Chem. 1976, 1762].

Die Herstellung der erfindungsgemäßen Verbindungen kann durch die folgenden Syntheseschemata veranschaulicht werden: 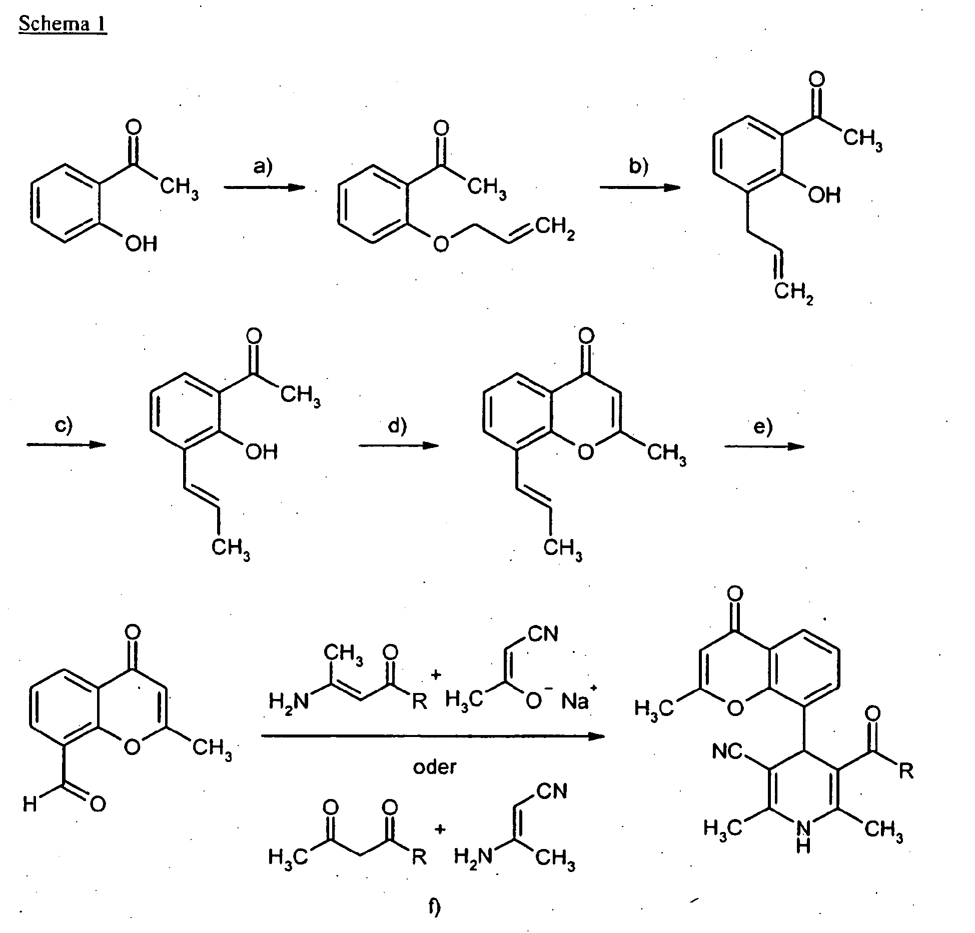 [a): Allylbromid, Kaliumcarbonat, cat. Kaliumiodid, Aceton, Rückfluss; b): 230°C, 4 h; c): Bis-(benzonitril)dichlorpalladium(II), Toluol, 120°C, 16 h; d): Acetylchlorid, Natriumhydrid, THF, 10-25°C, 16 h; e): 1. Ozon, Dichlormethan, -60°C, 30 min; 2. Dimethylsulfid; f): Eisessig, 2-Propanol, Rückfluss, 4-10 h]. 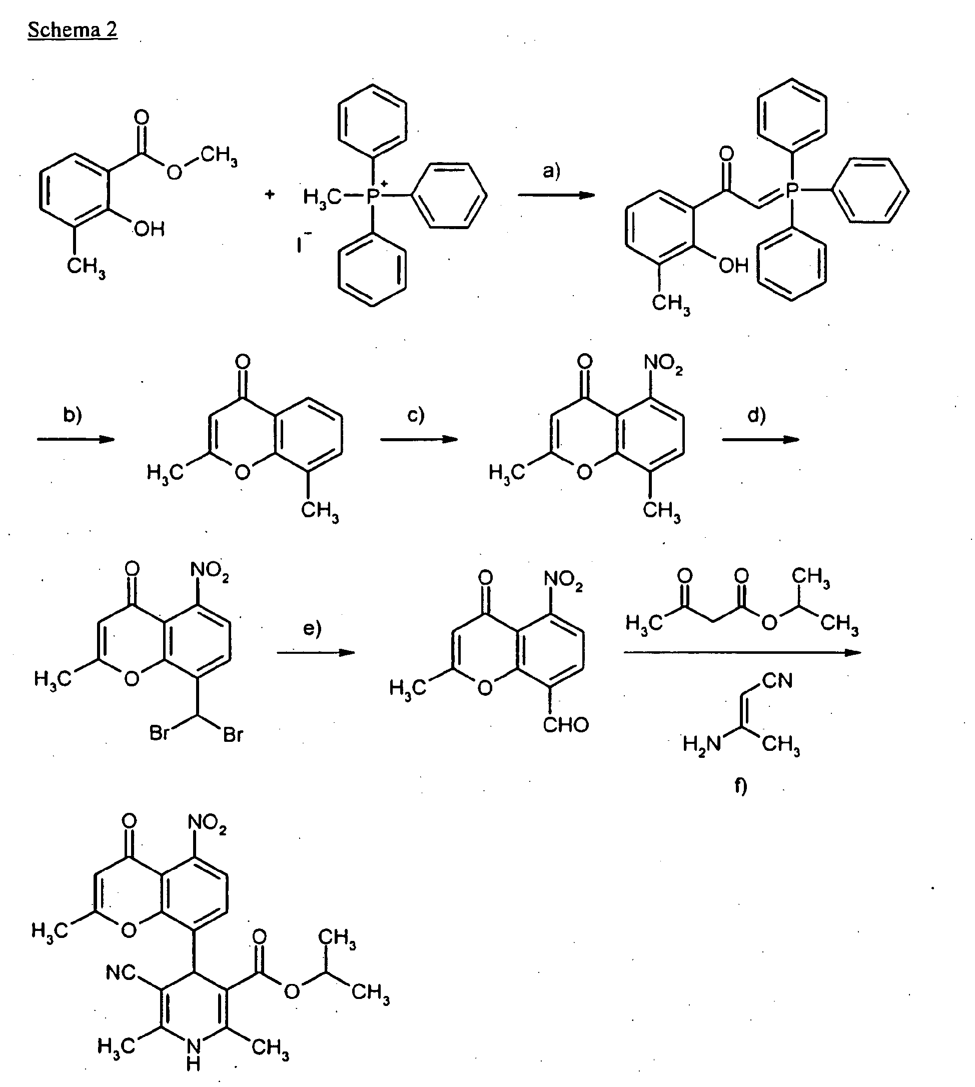 [a): n-Butyllithium, THF, 60°C, 3 h; b): Essigsäureanhydrid, Pyridin, Rückfluss, 6 h; c): konz. H₂SO₄, HNO₃ 0°C, 1 h; d): *N*-Bromsuccinimid, AIBN, Tetrachlorkohlenstoff, Rückfluss; e): *N-*Methylmorpholin-*N*-oxid, Acetonitril, Rückfluss; f): Eisessig, 2-Propanol, Rückfluss, 6 h]. 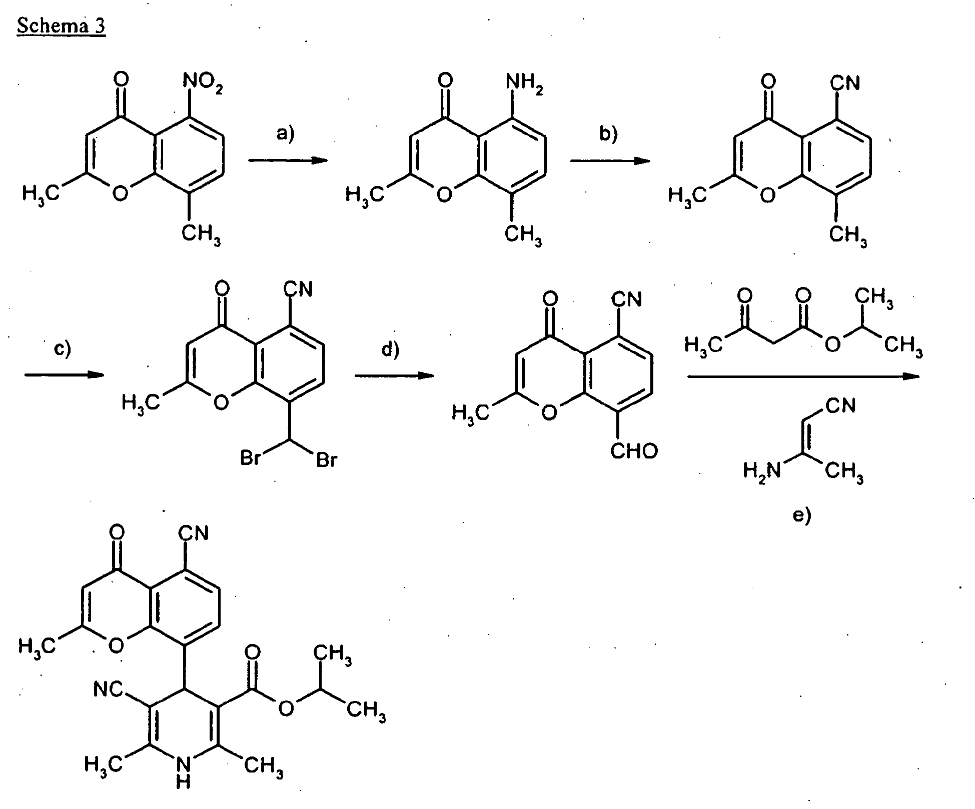 [a): Zinn(II)chlorid-Dihydrat, Ethylacetat, 70°C; b): 1. Natriumnitrit, Schwefelsäure, 0°C, 1.5 h; 2. Kupfer(I)cyanid, Natriumcyanid, Wasser/Ethylacetat, 0°C, 45 min; c): *N*-Bromsuccinimid, AIBN, Tetrachlorkohlenstoff, Rückfluss; d): *N*-Methylmorpholin-N-oxid, Acetonitril, Rückfluss; e): Eisessig, 2-Propanol, Rückfluss, 6 h]. 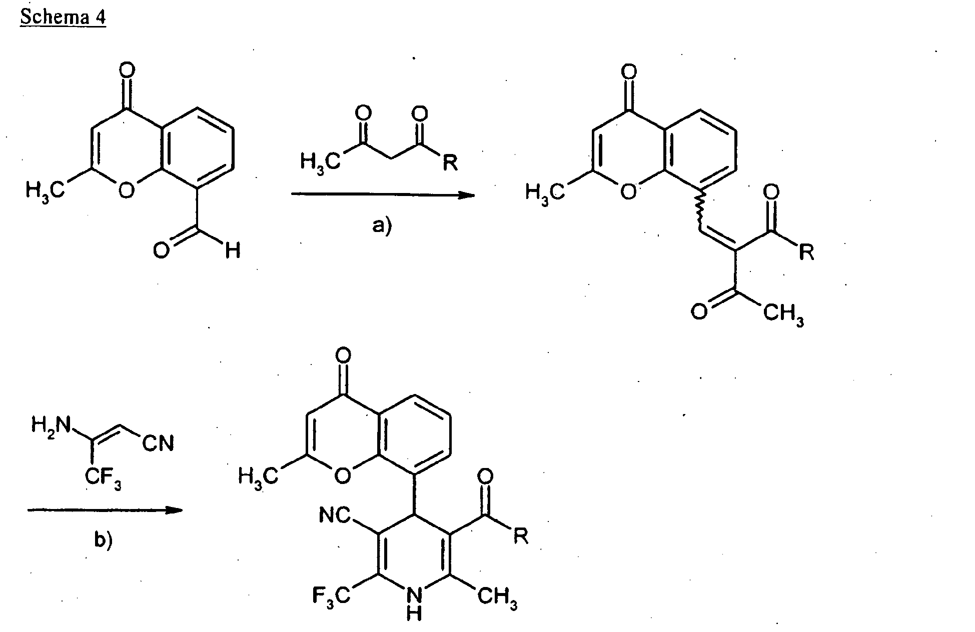 [a): Eisessig / Piperidin, Dichlormethan, Rückfluss, 12 h; b): Kalium-*tert*.-butylät, 2-Propanol, Rückfluss, 12 h).

Die erfindungsgemäßen Verbindungen wirken als Antagonisten des Mineralokorticoid-Rezeptors und zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen sind geeignet für die Prophylaxe und/oder Behandlung von verschiedenen Erkrankungen und krankheitsbedingten Zuständen, insbesondere von Erkrankungen, die durch eine Erhöhung der Aldosteron-Konzentration im Plasma gekennzeichnet sind oder mit ihr einhergehen. Beispielsweise seien genannt: idiopathischer primärer Hyperaldosteronismus, Hyperaldosteronismus bei Nebennierenhyperplasie und/oder Nebennierenadenomen und/oder Nebennierencarzinomen, Hyperaldosteronismus bei Leberzirrhose, Hyperaldosteronismus bei Herzinsuffizienz sowie Hyperaldosteronismus bei essentieller Hypertonie.

Die erfindungsgemäßen Verbindungen sind aufgrund ihres Wirkmechanismus ferner geeignet für die Prophylaxe des plötzlichen Herztodes bei Patienten, die unter einem erhöhten Risiko stehen, an einem plötzlichen Herztod zu versterben. Dies sind insbesondere Patienten, die z.B. an einer der folgenden Erkrankungen leiden: Hypertonie, Herzinsuffizienz, koronare Herzerkrankung, stabile und instabile Angina pectoris, myokardiale Ischämie, Myokardinfarkt, Schock, Arteriosklerose, atriale und ventrikuläre Arrhythmie, transitorische und ischämische Attacken, Hirnschlag, entzündliche kardiovaskuläre Erkrankungen, periphere und kardiale Gefäßerkrankungen, periphere Durchblutungsstörungen, pulmonale Hypertonie, Spasmen der Koronararterien und peripherer Arterien, Thrombosen, thromboembolische Erkrankungen sowie Vaskulitis.

Die erfindungsgemäßen Verbindungen können ferner verwendet werden für die Prophylaxe und/oder Behandlung von Ödembildung wie zum Beispiel pulmonales Ödem, renales Ödem oder Herzinsuffizienz-bedingtes Ödem, und von Restenosen wie nach Thrombolysetherapien, percutantransluminalen Angioplastien (PTA) und transluminalen Koronarangioplastien (PTCA), Herztransplantationen und Bypass-Operationen.

Weiterhin eignen sich die erfindungsgemäßen Verbindungen zur Verwendung als Diuretikum und bei Elektrolytstörungen wie zum Beispiel Hyperkalzämie.

Außerdem können die erfindungsgemäßen Verbindungen eingesetzt werden für die Prophylaxe und/oder Behandlung von Diabetes mellitus und diabetischen Folgeerkrankungen wie z.B. Neuropathie und Nephropathie, von akutem und chronischem Nierenversagen sowie der chronischen Niereninsuffizienz.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehreren weitere Wirkstoffe, insbesondere zur Behandlung und/oder Prävention der zuvor genannten Erkrankungen. Als geeignete Kombinationswirkstoffe seien beispielhaft und vorzugsweise genannt: ACE-Inhibitoren, Renin-Inhibitoren, Angiotensin II-Rezeptor-Antagonisten, Beta-Blocker, Acetylsalicylsäure, Diuretika, Kalium-Supplements, Calcium-Antagonisten, Statine, Digitalis (Digoxin)-Derivate, Calcium-Sensitizer wie Levosimendan, Nitrate, Antikoagulantien, Antiarrhythmika, Vasodilatatoren sowie Thrombolytika.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überfährt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis.0.5 .mg/kg Körpergewicht zur Erzielung wirksamer. Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittetverhättnisse, Verdünnungsverhältnisse und Konzentrationsangaben von flüssig/flüssig-Lösungen.beziehen sich jeweils auf das Volumen.

### A. Beispiele

### Abkürzungen und Akronyme:

- Ac: Acetyl
- AIBN: 2,2'-Azobis-2-methylpropannitril
- cat.: katalytisch
- CI: Chemische Ionisation (bei MS)
- DC: Dünnschichtchromatographie
- DMF: Dimethylformamid
- DMSO: Dimethylsulfoxid
- d. Th.: der Theorie (bei Ausbeute)
- ee: Enantiomerenüberschuss
- ESI: Elektrospray-Ionisation (bei MS)
- GC-MS: Gaschromatographie-gekoppelte Massenspektroskopie
- h: Stunde(n)
- HPLC: Hochdruck-, Hochleistungsflüssigchromatographie
- konz.: konzentriert
- LC-MS: Flüssigchromatographie-gekoppelte Massenspektroskopie
- min: Minute(n)
- MS: Massenspektroskopie
- NMR: Kernresonanzspektroskopie
- R_{f}: Rententionsindex (bei DC)
- Rₑ: Retentionszeit (bei HPLC)
- RT: Raumtemperatur
- THF: Tetrahydrofuran

### LC-MS-, GC-MS- und HPLC-Methoden:

### Methode 1 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4 (HPLC, Enantiomerentrennung):

Säule: 670 mm x 40 mm, chirale Kieselgel-Phase basierend auf dem Selektor Poly(*N*-methacryloyl-L-leucin-*tert.*-butylamid); Eluent: Essigsäureethylester; Temperatur: 24°C; Fluss: 80 ml/min; UV-Detektion: 280 nm.

### Methode 5 (HPLC, Enantiomerentrennung):

Säule: 670 mm x 40 mm, chirale Kieselgel-Phase basierend auf dem Selektor Poly(*N*-methacryloyl-L-leucin-tert.-butylamid); Eluent: Essigsäureethylester; Temperatur: 24°C; Fluss: 50 ml/min; UV-Detektion: 254 nm.

### Methode 6 (HPLC, Enantiomerentrennung):

Säule: 670 mm x 40 mm, chirale Kieselgel-Phase basierend auf dem Selektor Poly(*N*-methacryloyl-D-leucin-*tert.*-butylamid); Eluent: Essigsäureethylester; Temperatur: 24°C; Fluss: 80 ml/min; UV-Detektion: 280 nm.

### Methode 7 (HPLC, Enantiomerentrennung):

Säule: 250 mm x 4.6 mm, chirale Kieselgel-Phase basierend auf dem Selektor Poly(*N*-methacryloyl-L-leucin-*tert.*-butylamid); Eluent: Isohexan/Essigsäureethylester 2:1; Temperatur: 24°C; Fluss: 2 ml/min; UV-Detektion: 270 nm.

### Methode 8 (GC-MS):

Instrument: Micromass GCT, GC 6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Ausführungsbeispiele:

Sofern strukturell möglich und falls nicht anders angegeben, liegen die Alkene, die als Ausgangs- oder Zwischenverbindungen verwendet werden, als E/Z-Gemische vor.

### Allgemeine Vorschrift zur Herstellung von 3-Aminocrotonsäureestern:

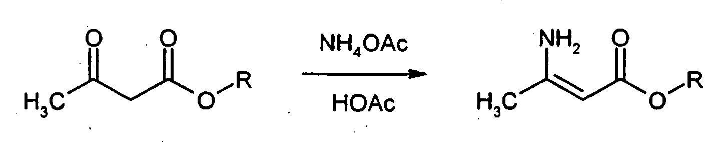

Zu einer Lösung des entsprechenden Acetessigsäureesters in Toluol werden 2 Äquivalente Ammoniumacetat und 0.9 Äquivalente Eisessig gegeben und das Gemisch am Wasserabscheider über Nacht unter Rückfluss gerührt. Nach dem Abkühlen wird die Reaktionslösung mit Essigsäureethylester verdünnt und nacheinander mit Natriumhydrogencarbonat- und Natriumchlorid-Lösung gewaschen. Die organische Phase wird über Magnesiumsulfat getrocknet und eingeengt. Der Rückstand wird ohne weitere Aufreinigung eingesetzt.

### Beispiel 1

### tert.-Butyl 5-cyano-2,6-dimethyl-4-(2-methyl-4-oxo-4H-chroinen-8-yl)-1,4-dihydropyridin-3-carboxylat

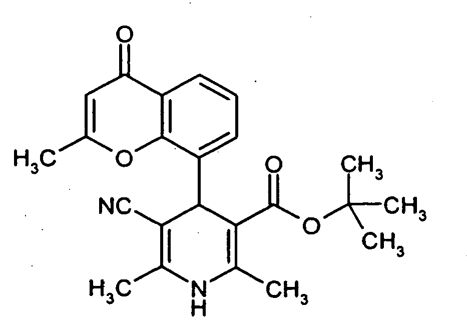

### Stufe 1a):

### 1-[2.(Allyloxy)phenyl]ethanon

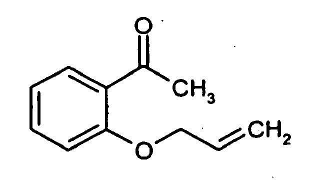

542 g (3.9 mol) 2-Hydroxyacetophenon werden mit 592 g (4.9 mol) Allylbromid, 1000 g (7.2 mol) Kaliumcarbonat und 13.2 g (79 mmol) Kaliumiodid in 2.4 Liter Aceton 24 h lang zum Rückfluss erhitzt. Nach Abkühlen auf Raumtemperatur wird filtriert und das Lösungsmittel im Vakuum entfernt. Der Rückstand wird in Toluol gelöst und mit 10%-iger Natronlauge und Wasser gewaschen. Nach Einengen werden 689 g (98% d. Th.) der Titelverbindung erhalten.
¹H-NMR (300 MHz, CDCl₃): δ = 2.68 (s, 3H), 4.68 (dd, 2H), 5.89 (dd, 2H), 6.09 (m, 1H), 6.99 (dd, 2H), 7.44 (m, 1H), 7.71 (d, 1H).

### Stufe 1b):

### 1-(3-Allyl-2-hydroxyphenyl)ethanon

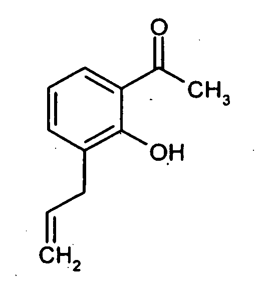

160 g (0.9 mol) 1-[2-(Allyloxy)phenyl]ethanon werden im Metallbad 4 h lang bei 230-240°C gerührt. Nach Abkühlen auf Raumtemperatur wird das Produkt über einen Dünnschichtverdampfer bei 140°C und 0.4 mbar destilliert. Es werden 155 g (97% d. Th.) der Titelverbindung erhalten.
¹H-NMR (300 MHz, CDCl₃): δ = 2.68 (s, 3H), 3.44 (d, 2H), 5.09 (m, 2H), 6.01 (m, 1H), 6.85 (t, 1H), 7.3 (dd, 1H), 7.62 (dd, 1H), 12.61 (s, 1H).

### Stufe 1c:

### 1-{ 2-Hydroxy-3-[(1E)-prop-1-en-1-yl]phenyl}ethanon

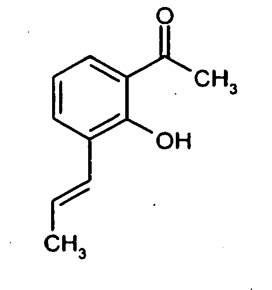

40 g (227 mmol) 1-(3-Allyl-2-hydroxyphenyl)ethanon werden in 120 ml Toluol gelöst und mit 2.17 g (5.6 mmol) Bis(benzonitrii)dichlorpalladium(II) versetzt. Die Reaktionsmischung wird über Nacht auf 120°C erhitzt. Nach Abkühlen auf Raumtemperatur wird über Kieselgur filtriert und das Lösungsmittel im Vakuum entfernt. Es werden 20.9 g (95% d.Th.) der Titelverbindung erhalten, welche ohne weitere Reinigung in der nächsten Stufe umgesetzt wird.
LC-MS (Methode 1): Rₜ = 2.36 min; [M+H]⁺ = 177
¹H-NMR (300 MHz, CDCl₃): δ =1.91 (dd, 3H), 2.63 (s, 3H), 6.32 (m, 1H), 6.73 (dd, 1H), 6.85 (t, 1H), 7.59 (m, 2H), 12.74 (s, 1H).

### Stufe 1d:

### 2-Methyl-8-[(1E)-prop-1-en-1-yl]-4H-chromen-4-on

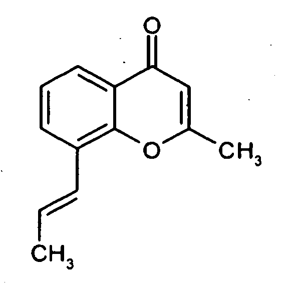

12.52 g (313.2 mmol) 60%-iges Natriumhydrid (Suspension in Mineralöl) werden unter Argon bei 10°C in 300 ml absolutem THF vorgelegt. Zu der Suspension werden 18.4 g (104.4 mmol) 1-{2-Hydroxy-3-[(1*E*)-prop-1-en-1-yl]phenyl}ethanon langsam zugetropft. Nach 15 min werden 9 g (114.9 mmol) Acetylchlorid zugegeben. Die Reaktionsmischung wird über Nacht bei Raumtemperatur gerührt. Man hydrolysiert mit 300 ml Wasser und extrahiert mehrfach mit Ethylacetat. Nach Waschen der organischen Phase mit gesättigter Natriumchlorid-Lösung wird über Natriumsulfat getrocknet. Anschließend wird das Lösungsmittel im Vakuum entfernt. Der Rückstand wird in 200 ml Methanol aufgenommen und mit 50 ml 20%-iger Salzsäure 30 min auf 80°C erhitzt. Anschließend wird das Lösungsmittel im Vakuum entfernt und der Rückstand mit 400 ml Wasser versetzt. Es wird mehrfach mit Dichlormethan extrahiert. Nach Trocknen der organischen Phase über Magnesiumsulfat wird das Lösungsmittel im Vakuum entfernt und der Rückstand mittels Säulenchromatographie gereinigt (Laufmittel: Dichlormethan/Methanol 98:2). Es werden 10.5 g (50.2% d. Th.) der Titelverbindung als gelbes Öl erhalten.
LC-MS (Methode 3): Rₜ = 2.07 min; [M+H]⁺ = 201
¹H-NMR (300 MHz, CDCl₃): δ = 1.98 (dd, 3H), 2.43 (s, 3H), 6.18 (s, 1H), 6.40 (m, 1.H), 6.85 (dd, 1H), 7.31 (t, 1H), 7.72 (dd, 1H), 8.05 (dd, 1H).

### Stufe 1e):

### 2-Methyl-4-oxo-4H-chromen-8-carbaldehyd

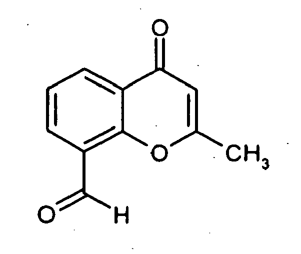

18.5 g (62.8 mmol) 2-Methyl-8-[(1*E*)-prop-1-en-1-yl]-4*H*-chromen-4-on werden in 400 ml Dichlormethan gelöst und auf -60°C abgekühlt. In die Reaktionslösung wird 30 min lang Ozon eingeleitet. Anschließend wird die Reaktionsmischung mit Dimethylsulfid versetzt. Nach Erwärmen auf Raumtemperatur wird das Lösungsmittel im Vakuum entfernt und der Rückstand mit wenig Methanol aufgeschlämmt. Nach Filtration wird der verbleibende Feststoff aus Diethylether umkristallisiert. Es werden 9.1 g (77.4% d. Th.) der Titelverbindung erhalten.
LC-MS (Methode 1): Rₜ = 1.31 min; [M+H]⁺ = 189
¹H-NMR (300 MHz, CDCl₃): δ = 2.48 (s, 3H), 6.27 (s, 1H), 7.5 (m, 1H), 8.21 (dd, 1H), 8.46 (dd, 1H), 10.67 (s, 1H).

### Stufe 1f):

### Natrium 1-cyanoprop-1-en-2-olat

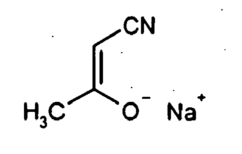

6.9 g (300.9 mmol) Natrium werden unter Argon langsam in 300 ml absolutes Methanol eingetragen. Nachdem sich das Natrium vollständig gelöst hat, werden innerhalb von 5 min 25 g (300.9 mmol) 5-Methylisoxazol zugetropft. Man rührt über Nacht bei Raumtemperatur. Nach Entfernen des Lösungsmittels im Vakuum verbleibt das Produkt als farbloser Feststoff. Es werden 31 g (99% d. Th.) erhalten, welche ohne weitere Reinigung eingesetzt werden.
¹H-NMR (400 MHz, DMSO-d₆): δ = 3.18 (s, 1H), 1.51 (s, 3H).

### Stufe 1g):

### tert.-Butyl 5-cyano-2,6-dimethyl-4-(2-methyl-4-oxo-4H-chromen-8-yl)-1,4-dihydropyridin-3-carboxylat

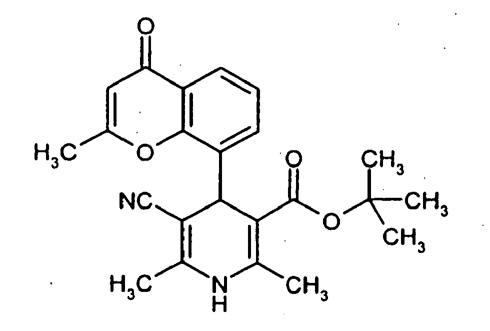

100 mg (0.53 mmol) 2-Methyl-4-oxo-4*H*-chromen-8-carbaldehyd werden mit 55.8 mg (0.53 mmol) Natrium-1-cyanoprop-1-en-2-olat, 83.5 mg (0.53 mmol) 3-Aminocrotonsäure-tert.-butylester und 31.9 mg (0.53 mmol) Essigsäure in 5 ml 2-Propanol gelöst und unter Argon 4 h lang unter Rückfluß erhitzt. Das Lösungsmittel wird im Vakuum entfernt und der Rückstand mittels präparativer HPLC gereinigt. Es werden 89 mg (42% d. Th.) der Titelverbindung als gelber Feststoff erhalten.
LC-MS (Methode 2): Rₜ = 2.31 min; [M+H]⁺ = 393
¹H-NMR (300 MHz, DMSO-d₆): δ= 1.07.(s, 9H), 1.97 (s, 3H), 2.31 (s, 3H), 2.39 (s, 3H), 5.12 (s, 1H), 6.27 (s, 1H), 7.43 (t, 1H), 7.53 (dd, 1H), 7.88 (dd, 1H), 9.18 (s, 1H).

### Beispiel 2

### Cyclopentyl 5-cyano-2,6-dimethyl-4-(2-methyl-4-oxo-4H-chromen-8-yl)-1,4-dihydropyridin-3-carboxylat

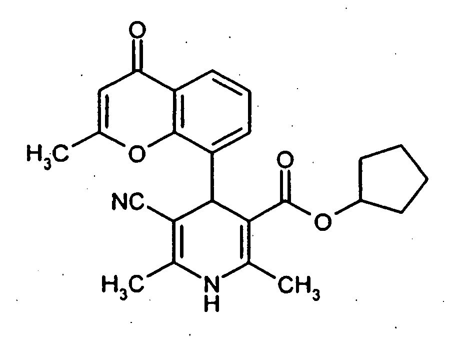

100 mg (0.53 mmol) 2-Methyl-4-oxo-4*H*-chromen-8-carbaldehyd werden mit 55.8 mg (0.53 mmol) Natrium-1-cyanoprop-1-en-2-olat, 89.9 mg (0.53 mmol) 3-Aminocrotonsäurecyclopentylester und 31.9 mg (0.53 mmol) Essigsäure in 5 ml 2-Propanol gelöst und unter Argon 4 h lang unter Rückfluß erhitzt. Das Lösungsmittel wird im Vakuum entfernt und der Rückstand mittels präparativer HPLC gereinigt. Es werden 35 mg (16% d. Th.) der Titelverbindung als gelber Feststoff erhalten.
LC-MS (Methode 2): Rₜ = 2.32 min; [M+H]⁺ = 405
¹H-NMR (300 MHz, DMSO-d₆): δ = 0.89 (m, 2H), 1.42 (m, 4H), 1.63 (m, 2H), 1.97 (s, 3H), 2.33 (s, 3H), 2.39 (s, 3H), 4.89 (m, 1H), 5.14 (s, 1H), 6.28 (s, 1H), 7.42 (t, 1H), 7.52 (dd, 1H), 7, 7.87 (dd, 1H), 9.27 (s, 1H).

### Beispiel 3

### Cyclobutyl 5-cyano-2,6-dimethyl-4-(2-methyl-4-oxo-4H-chromen-8-yl)-1,4-dihydropyridin-3-carboxylat

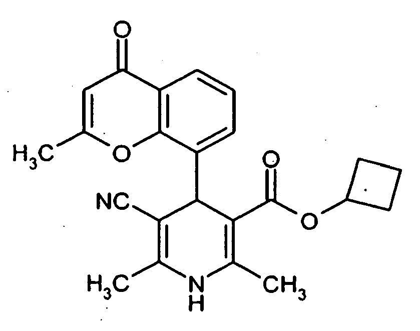

### Stufe 3a):

### Cyclobutyl-3-oxobutanoat

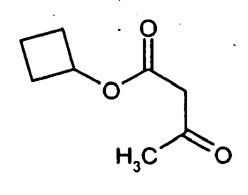

11.82 g (83.21 mmol) 2,2,6-Trimethyl-1,3-dioxin-4-on und 6 g (83.21 mmol) Cyclobutanol werden in Toluol (25 ml) unter Argon 4 h lang unter Rückfluss gerührt. Anschließend wird das Lösungsmittel im Vakuum entfernt. Man erhält 13 g eines gelben Öls, das ohne weitere Reinigung eingesetzt wird.
¹H-NMR (400 MHz, CDCl₃): δ = 1.53 (m, 1H), 1.80 (m, 1H), 2.09 (m, 2H), 2.28 (s, 3H), 2.47 (m, 2H), 3.42 (s, 2H), 5.03 (m, 1H).

### Stufe 3b):

### Cyclobutyl 5-cyano-2,6-dimethyl-4-(2-methyl-4-oxo-4H-chromen-8-yl)-1,4-dihydropyridin-3-carboxylat

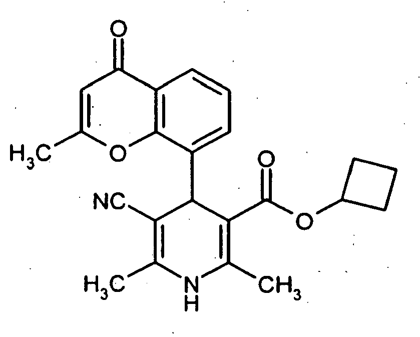

60 mg (0.32 mmol) 2-Methyl-4-oxo-4*H*-chromen-8-carbaldehyd werden mit 49.7 mg (0.32 mmol) Cyclobutyl-3-oxobutanoat, 26.2 mg (0.32 mmol) 3-Aminocrotonsäurenitril und 19.1 mg (0.32 mmol) Essigsäure in 5 ml 2-Propanol gelöst und unter Argon 4 h lang unter Rückfluß erhitzt. Das Lösungsmittel wird im Vakuum entfernt und der Rückstand mittels präparativer HPLC gereinigt. Es werden 64 mg (5 1 % d. Th.) der Titelverbindung als gelber Feststoff erhalten.
LC-MS (Methode 1): Rₜ = 2.02 min; [M+H]⁺ = 391
¹H-NMR (300 MHz, DMSO-d₆): δ = 1.35 (m, 1H), 1.45 (m, 2H), 1.77 (m, 1H), 1.95 (m, 1H); 2.00 (s, 3H), 2.14 (m, 1H), 2.31 (s, 3H), 2.40 (s, 3H), 4.7 (q, 1H), 5.13 (s, 1H), 6.28 (s, 1H), 7.42 (t, 1H), 7.57 (dd, 1H), 7.89 (dd, 1H), 9.32 (s, 1H).

### Beispiel 4

### Propyl 5-cyano-2,6-dimethyl-4-(2-methyl-4-oxo-4H-chromen-8-yl)-1,4-dihydropyridin-3-carboxylat

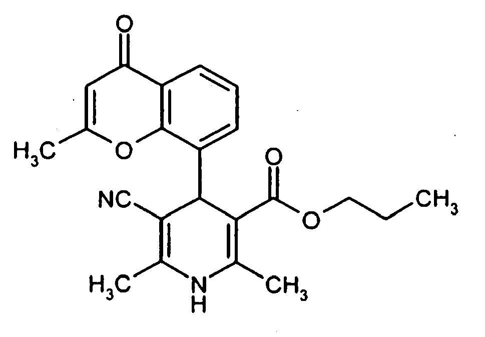

700 mg (3.7 mmol) 2-Methyl-4-oxo-4*H*-chromen-8-carbaldehyd werden mit 536 mg (3.7 mmol) Propyt-3-oxobutanoat, 205 mg (3.7 mmol) 3-Aminocrotonsäurenitril und 223 mg (3.7 mmol) Essigsäure in 20 ml 2-Propanol gelöst und unter Argon 16 h lang unter Rückfluß erhitzt. Das Lösungsmittel wird im Vakuum entfernt und der Rückstand mittels präparativer HPLC gereinigt. Es werden 460 mg (32% d. Th.) der Titelverbindung als gelber Feststoff erhalten.
LC-MS (Methode 1 ): Rₜ = 1.92 min; [M+H]⁺ = 379
¹H-NMR (300 MHz, DMSO-d₆): δ = 0.55 (t, 3H), 1.28 (m, 2H), 1.99 (s, 3H), 2.34 (s, 3H), 2.39 (s. 3H), 3.77 (m, 2H), 5.16 (s, 1H), 6.27 (s, 1H), 7.41 (t, 1H), 7.52 (dd, 1H), 7.89 (dd, 1H), 9.32 (s, 1H).

### Beispiel 5

### 2,2,2-Trifluorethyl 5-cyano-2,6-dimethyl-4-(2-methyl-4-oxo-4H-chromen-8-yl)-1,4-dihydropyridin-3-carboxylat

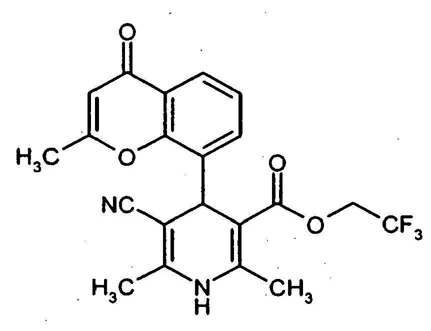

### Stufe 5a:

### 2,2,2-Trifluorethyl 3-oxobutanoat

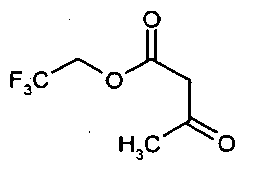

Die Titelverbindung wird in Analogie zu Beispiel 3 (Stufe 3a) ausgehend von 2,2,6-Trimethyl-1,3-dioxin-4-on und 2,2,2-Trifluorethanol hergestellt.

### Stufe 5b):

### 2,2,2-Trifluorethyl 5-cyano-2,6-dimethyl-4-(2-methyl-4-oxo-4H-chromen-8-yl)-1,4-dihydropyridin-3-carboxylat

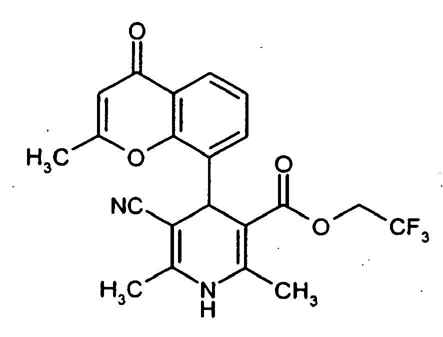

60 mg (0.32 mmol) 2-Methyl-4-oxo-4*H*-chromen-8-carbaldehyd werden mit 60.1 mg (0.32 mmol) 2,2,2-Trifluorethyl-3-oxobutanoat, 26.2 mg (0.32 mmol) 3-Aminocrotonsäurenitril und 19.1 mg (0.32 mmol) Essigsäure in 5 ml 2-Propanol gelöst und unter Argon 4 h lang unter Rückfluß erhitzt. Das Lösungsmittel wird im Vakuum entfernt und der Rückstand mittels präparativer HPLC gereinigt. Es werden 103 mg (77% d. Th.) der Titelverbindung als gelber Feststoff erhalten.
LC-MS (Methode 3): Rₜ = 2.17 min; [M+H]⁺ = 419
¹H-NMR (300 MHz, DMSO-d₆): δ = 2.01 (s, 3H), 2.36 (s, 3H), 2.37 (s, 3H), 4.5 (m, 2H), 5.16 (s, 1H), 6.26 (s, 1H), 7.40 (t, 1H), 7.53 (dd, 1H), 7.87 (dd, 1H), 9.56 (s, 1H).

### Beispiel 6

### Methyl 5-cyano-2,6-dimethyl-4-(2-methyl-4-oxo-4H-chromen-8-yl)-1,4-dihydropyridin-3-carboxylat

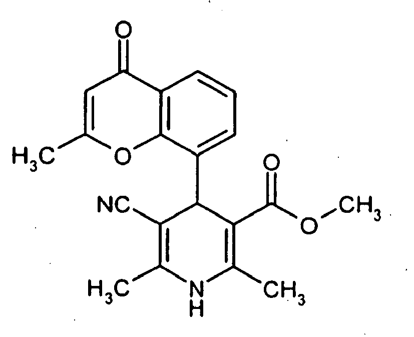

100 mg (0.53 mmol) 2-Methyl-4-oxo-4*H*-chromen-8-carbaldehyd werden mit 61.7 mg (0.53 mmol) Methyl-3-oxobutanoat, 43.6 mg (0.53 mmol) 3-Aminocrotonsäurenitril und 31.9 mg (0.53 mmol) Essigsäure in 5 ml 2-Propanol gelöst und unter Argon 4 h lang unter Rückfluß erhitzt. Nach Abkühlen auf Raumtemperatur wird das auskristallisierte Produkt abfiltriert und mit 2-Propanol und Diethylether gewaschen. Es werden 97 mg (52% d. Th.) der Titelverbindung als gelber Feststoff erhalten.
LC-MS (Methode 3): Rₜ = 1.88 min; [M+H]⁺ = 351
¹H-NMR (300 MHz, DMSO-d₆): δ = 2.00 (s, 3H), 2.32 (s, 3H), 2.40 (s, 3H), 3.42 (s, 3H), 5.09 (s, 1H), 6.27 (s, 1H), 7.40 (t, 1H), 7.49 (dd, 1H), 7.87 (dd, 1H), 9.38 (s, 1H).

### Beispiel 7

### Ethyl 5-cyano-2,6-dimethyl-4-(2-methyl-4-oxo-4H-chromen-8-yl)-1,4-dihydropyridin-3-carboxylat

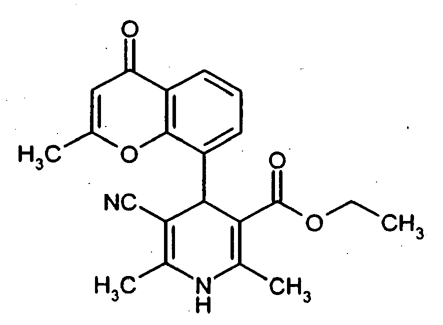

100 mg (0.53 mmol) 2-Methyl-4-oxo-4*H*-chromen-8-carbaldehyd werden mit 69.1 mg (0.53 mmol) Ethyl-3-oxobutanoat, 43.6 mg (0.53 mmol) 3-Aminocrotonsäurenitril und 31.9 mg (0.53 mmol) Essigsäure in 5 ml 2-Propanol gelöst und unter Argon 4 h lang unter Rückfluß erhitzt. Nach Abkühlen auf Raumtemperatur wird das auskristallisierte Produkt abfiltriert und mit 2-Propanol und Diethylether gewaschen. Es werden 43 mg (22% d. Th.) der Titelverbindung als gelber Feststoff erhalten.
LC-MS (Methode 3): Rₜ = 2.02 min; [M+H]⁺ = 365
¹H-NMR (300 MHz, DMSO-d₆): δ = 0.89 (t, 3H), 2.00 (s, 3H), 2.32 (s, 3H), 2.39 (s, 3H), 3.84 (q, 2H), 5.12 (s, 1H), 6.27 (s, 1H), 7.41 (t, 1H), 7.53 (dd, 1H), 7.82 (dd, 1H), 9.33 (s, 1H).

### Beispiel 8

### Cyclobutyl 5-cyano-4-(5-fluor-2-methyl-4-oxo-4H-chromen-8-yl)-2,6-dimethyl-1,4-dihydropyridin-3-carboxylat

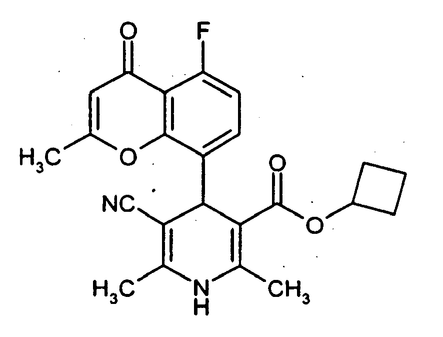

### Stufe 8a):

### 5-Fluor-2-methyl-4-oxo-4H-chromen-8-carbaldehyd

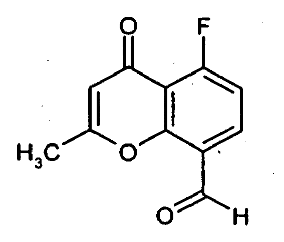

Die Titelverbindung wird in Analogie zu Beispiel 1, Stufe a-e, ausgehend von 1-(2-Fluor-6-hydroxyphenyl)ethanon erhalten.
LC-MS (Methode 3): Rₜ = 1.47 min; [M+H]⁺ = 207
¹H-NMR (300 MHz, CDCl₃): δ = 2.45 (t, 3H), 6.21 (s, 1H), 7.15 (dd, 1H), 8.20 (dd, 1H), 10.57 (s, 1H).

### Stufe 8b):

### Cyclobutyl 5-cyano-4-(5-fluor-2-methyl-4-oxo-4H-chromen-8-yl)-2,6-dimethyl-1,4-dihydropyridin-3-carboxylat

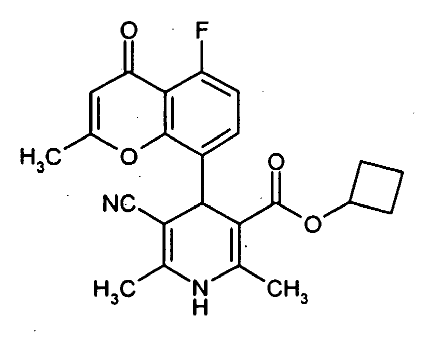

Eine Lösung von 100 mg (0.49 mmol) 5-Fluor-2-methyl-4-oxo-4*H*-chromen-8-carbaldehyd in 5 ml 2-Propanol wird mit 50.96 mg (0.49 mmol) Natrium-1-cyanoprop-1-en-2-olat, 75.28 mg (0.49 mmol) Cyclobutyl-3-aminobut-2-enoat und 0.04 ml (0.73 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die beigefarbenen Kristalle werden abgesaugt und im Vakuumtrockenschrank bei 40°C getrocknet. Es werden 120.5 mg (60.8% d. Th.) der Titelverbindung erhalten.
LC-MS (Methode 1): R₁ = 1.98 min;
MS (ESIpos): m/z = 409 [M+H]⁺
¹H-NMR (300 MHz, DMSO-d₆): δ = 9.51 (1H, s), 7.56 (1H, dd), 7.2 (1H, dd), 6.23 (1H, s), 5.08 (1H, s), 4.71 (1H, m), 2.37 (3H, s), 2.30 (3H, s), 2.15 (1H, m), 2.01 (3H, s), 1.79 (1H, m), 1.63-1.31 (4H, m).

### Beispiel 9

### Isopropyl 5-cyano-4-(5-fluor-2-methyl-4-oxo-4H-chromen-8-yl)-2,6-dimethyl-1,4-dihydropyridin-3-carboxylat

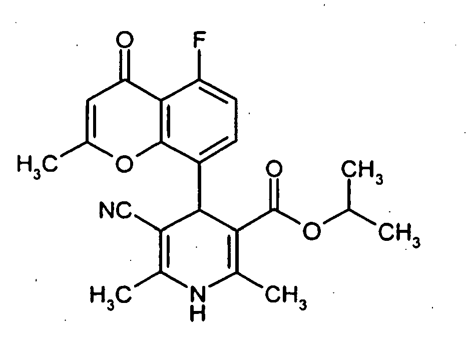

Eine Lösung von 100 mg (0.49 mmol) 5-Fluor-2-methyl-4-oxo-4*H*-chromen-8-carbaldehyd in 5 ml 2-Propanol wird mit 50.96 mg (0.49 mmol) Natrium-1-cyanoprop-1-en-2-olat, 69.45 mg (0.49 mmol) 3-Aminocrotonsäureisopropylester und 0.04 ml (0.73 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die beigefarbenen Kristalle werden abgesaugt und im Vakuumtrockenschrank bei 40°C getrocknet. Es werden 88.1 mg (45.8% d. Th.) der Titelverbindung erhalten.
LC-MS (Methode 1): Rₜ = 1.9 min;
MS (ESIpos): m/z = 397 [M+H]⁺
¹H-NMR (300 MHz, DMSO-d₆): δ = 9.41 (1H, s), 7.53 (1H, dd), 7.19 (1H, dd), 6.23 (1H, s), 5.08 (1H, s), 4.65 (1H, m), 2.36 (3H, s), 2.31 (3H, s), 2.0 (3H, s), 1.06 (3H, d), 0.7 (3H, d).

### Beispiel 10

### Propyl 5-cyano-4-(5-fluor-2-methyl-4-oxo-4H-chromen-8-yl)-2,6-dimethyl-1,4-dihydropyridin-3-carboxylat

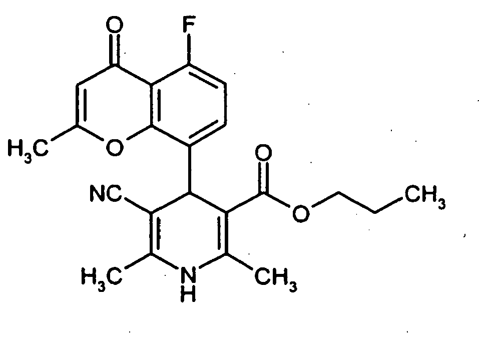

Eine Lösung von 100 mg (0.49 mmol) 5-Fluor-2-methyl-4-oxo-4*H*-chromen-8-carbaldehyd in 5 ml 2-Propanol wird mit 50.96 mg (0.49 mmol) Natrium-1-cyanoprop-1-en-2-olat, 69.45 mg (0.49 mmol) 3-Aminocrotonsäurepropylester und 0.04 ml (0.73 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die beigefarbenen Kristalle werden abgesaugt und im Vakuumtrockenschrank bei 40°C getrocknet. Es werden 112.9 mg (58.7% d. Th.) der Titelverbindung erhalten.
LC-MS (Methode 1): Rₜ = 1.92 min;
MS (ESIpos): m/z = 397 [M+H]⁺
¹H-NMR (300 MHz, DMSO-d₆): δ = 9.45 (1H, s), 7.50 (1H, dd), 7.18 (1H, dd), 6.22 (1H, s), 5.1 (1H, s), 3.77 (2H, m), 2.36 (3H, s), 2.33 (3H, s), 1.99 (3H, s), 1.31 (2H, m), 0.58 (3H, t).

### Beispiel 11

### Ethyl 5-cyano-4-(5-fluor-2-methyl-4-oxo-4H-chromen-8-yl)-2,6-dimethyl-1,4-dihydropyridin-3-carboxylat

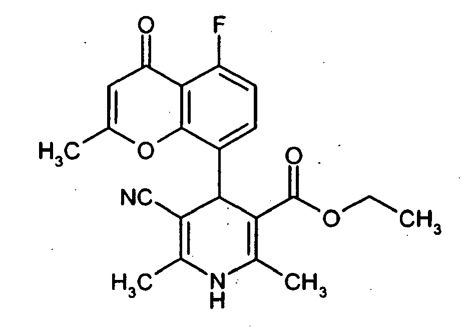

Eine Lösung von 100 mg (0.49 mmol) 5-Fluor-2-methyl-4-oxo-4*H*-chromen-8-carbaldehyd in 5 ml 2-Propanol wird mit 50.96 mg (0.49 mmol) Natrium-1-cyanoprop-1-en-2-olat, 62.65 mg (0.49 mmol) 3-Aminocrotonsäureethylester und 0.04 ml (0.73 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die beigefarbenen Kristalle werden abgesaugt und im Vakuumtrockenschrank bei 40°C getrocknet. Es werden 120.7 mg (65.1% d. Th.) der Titelverbindung erhalten.
LC-MS (Methode 1): Rₜ = 1.77 min;
MS (ESIpos): m/z = 383 [M+H]⁺
¹H-NMR (400 MHz, DMSO-d₆): δ = 9.54 (1H, s), 7.51 (1H, dd), 7.18 (1H, dd), 6.22 (1H, s), 5.07 (1H, s), 3.85 (2H, q), 2.36 (3H, s), 2.31 (3H, s), 2.0 (3H, s), 0.92 (3H, t).

### Beispiel 12

### Methyl 5-cyano-4-(5-fluor-2-methyl-4-oxo-4H-chromen-8-yl)-2,6-dimethyl-1,4-dihydropyridin-3-carboxylat

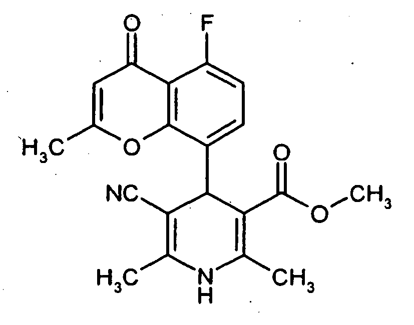

Eine Lösung von 100 mg (0.49 mmol) 5-Fluor-2-methyl-4-oxo-4*H*-chromen-8-carbaldehyd in 5 ml 2-Propanol wird mit 50.96 mg (0.49 mmol) Natrium-1-cyanoprop-1-en-2-olat, 55.84 mg (0.49 mmol) 3-Aminocrotonsäuremethylester und 0.04 ml (0.73 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die beigefarbenen Kristalle werden abgesaugt und im Vakuumtrockenschrank bei 40°C getrocknet. Es werden 128.3 mg (71.8% d. Th.) der Titelverbindung erhalten.
LC-MS (Methode 2): Rₜ = 1.89 min;
MS (ESIpos): m/z = 369 [M+H]⁺
¹H-NMR (300 MHz, DMSO-d₆): δ = 9.61 (1H, s), 7.48 (1H, dd), 7.16 (1H, dd), 6.21 (1H, s), 5.04 (1H, s), 3.43 (3H, s), 2.37 (3H, s), 2.32 (3H, s), 2.01 (3H, s).

### Beispiel 13

### 5-(Cyclobutylacetyl)-4-(5-fluor-2-methyl-4-oxo-4H-chromen-8-yl)-2,6-dimethyl-1,4-dihydropyridin-3-carbonitril

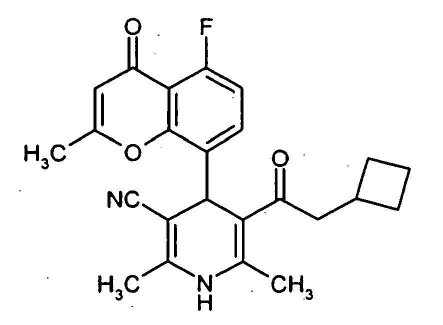

### Stufe 13a):

### 4-Amino-1-cyclobutylpent-3-en-2-on

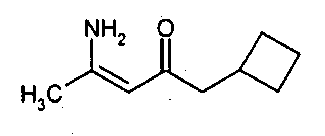

Die Herstellung erfolgt analog zu Beispiel 14 (Stufe 14a) ausgehend von 5-(Cyclobutylmethyl)-3-methylisoxazol [erhältlich analog zu C. Kashima et al., Bull. Chem. Soc. Jpn. 46, 310-313 (1973)].
GC-MS (Methode 8): Rₜ = 7.03 min; MS (CIpos): m/z = 154 [M+H]⁺.

### Stufe 13b):

### 5-(Cyclobutylacetyl)-4-(5-fluor-2-methyl-4-oxo-4H-chromen-8-yl)-2,6-dimethyl-1,4-dihydropyridin-3-carbonitril

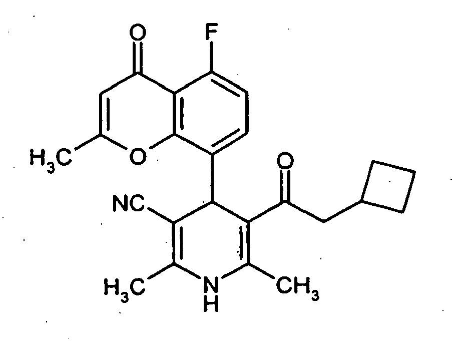

Eine Lösung von 100 mg (0.49 mmol) 5-Fluor-2-methyl-4-oxo-4*H*-chromen-8-carbaldehyd in 5 ml 2-Propanol wird mit 50.96 mg (0.49 mmol) Natrium-1-cyanoprop-1-en-2-olat, 92.90 mg (0.49 mmol) 4-Amino-1-cyclobutylpent-3-en-2-on und 0.04 ml (0.67 mmol) Essigsäure versetzt und 3 h lang unter Rückfluß gerührt. Nach dem Abkühlen wird der Ansatz eingeengt. Der erhaltene Rückstand wird über eine Analogix-Kartusche (F12M) gereinigt (Laufmittel: Cyclohexan/Essigsäureethylester 2:1). Es werden 40.4 mg (20.5% d. Th.) der Titelverbindung als gelbe Kristalle erhalten.
LC-MS (Methode 2): Rₜ = 2.23 min;
MS (ESIpos): m/z = 407 [M+H]⁺
¹H-NMR (300 MHz, DMSO-d₆): δ = 9.29 (1H, s), 7.45 (1H, dd), 7.17 (1H, dd), 6.22 (1H, s), 5.18 (1H, s), 2.71 (1H, dd), 2.45 (1H, dd), 2.39 (3H, s), 2.37 (1H, m), 2.30 (1H, m), 2.29 (3H, s), 1.99 (3H, s), 1.96-1.79 (2H, m), 1.78-1.59 (2H, m), 1.52-1.29 (2H, m).

### Beispiel 14

### 4-(5-Fluor-2-methyl-4-oxo-4H-chromen-8-yl)-2,6-dimethyl-5-(4-methylpentanoyl)-1,4-dihydropyridin-3-carbonitril

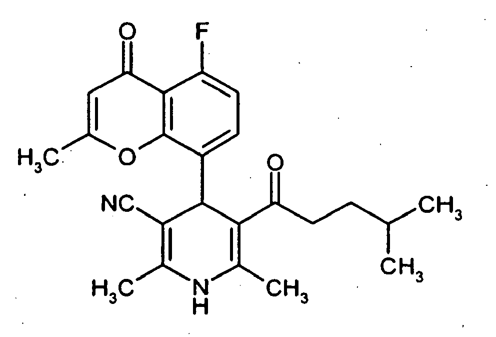

### Stufe 14a):

### 2-Amino-7-methyloct-2-en-4-on

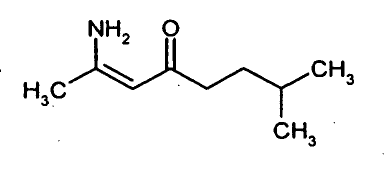

3-Methyl-5-(3-methylbutyl)isoxazol (3.90 g, 25.5 mmol) [Synthese analog C. Kashima et al., Bull. Chem. Soc. Jpn. 46, 310-313 (1973)] wird in 80 ml Ethanol vorgelegt, Platin(IV)oxid-Katalysator (390 mg, 1.72 mmol) zugegeben und der Ansatz anschließend unter Wasserstoff-Normaldruck für 2 h hydriert (leicht exotherme Reaktion). Der Katalysator wird abfiltriert, das Filtrat eingeengt und der Rückstand über eine Biotage-Kartusche 40M (Laufmittel: Isohexan/Essigsäureethylester 3:1) chromatographisch gereinigt. Die Produktfraktionen werden eingeengt. Man erhält als Rückstand ein Öl, das nach kurzer Zeit kristallisiert. Man trocknet im Vakuum und erhält 3.41 g (86% d. Th.) der Titelverbindung.
¹H-NMR (400 MHz, CDCl₃): δ = 9.71 (br. s, 1H), 5.02 (s, 1H), 4.95 (br. s, 1H), 2.26 (m, 2H), 1.91 (s, 3H), 1.63-1.42 (m, 3H), 0.89 (d, 6H)
GC-MS (Methode 8): Rₜ = 6.21 min; MS (CIpos): m/z = 156 (M+H]⁺.

### Stufe 14b):

### 4-(5-Fluor-2-methyl-4-oxo-4H-chromen-8-yl)-2,6-dimethyl-5-(4-methylpentanoyl)-1,4-dihydropyridin-3-carbonitril

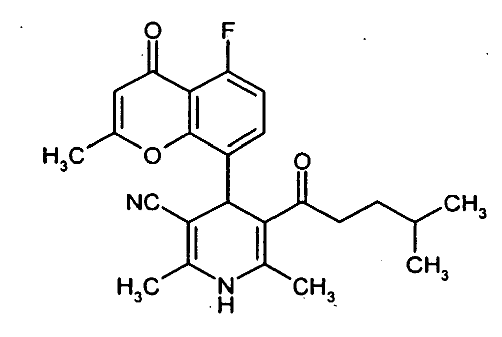

Eine Lösung von 100 mg (0.49 mmol) 5-Fluor-2-methyl-4-oxo-4*H*-chromen-8-carbaldehyd in 5 ml 2-Propanol wird mit 50.96 mg (0.49 mmol) Natrium-1-cyanoprop-1-en-2-olat, 75.3 mg (0.49 mmol) 2-Amino-7-methyloct-2-en-4-on und 0.04 ml (0.73 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die gelben Kristalle werden abgesaugt und über eine Analogix-Kartusche (F12M) weiter gereinigt (Laufmittel: Cyclohexan/Essigsäureethylester 2:1). Es werden 75.2 mg (38.1% d. Th.) der Titelverbindung erhalten.
LC-MS (Methode 2):Rₜ= 2.31 min;
MS (ESIpos): m/z = 409 [M+H]⁺
¹H-NMR (300 MHz, DMSO-d₆): δ = 9.3 (1H, s), 7.45 (1H, dd), 7.19 (1H, dd), 6.22 (1H, s), 5.12 (1H, s), 2.5-2.4 (1H, m), 2.38 (3H, s), 2.3 (3H, s), 2.2-2.09 (1H, m), 2.0 (3H, s), 1.37-1.0 (3H, m).

### Beispiel 15

### 5-(3-Cyclobutylpropanoyl)-4-(5-fluor-2-methyl-4-oxo-4H-chromen-8-yl)-2,6-dimethyl-1,4-dihydropyridin-3-carbonitril

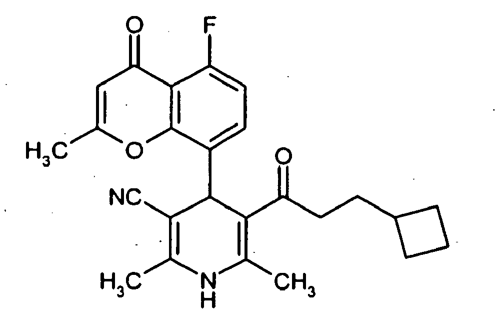

### Stufe 15a):

### 5-Amino-1-cyclobutylhex-4-en-3-on

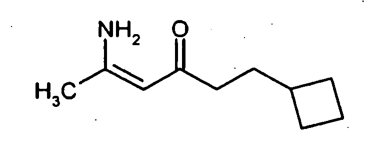

Die Herstellung erfolgt analog zu Beispiel 14 (Stufe 14a) ausgehend von 5-(2-Cyclobutylethyl)-3-methylisoxazol (erhältlich analog zu C. Kashima et al., Bull. Chem. Soc. Jpn. 46, 310-313 (1973)].
GC-MS (Methode 8): Rₜ = 7.82 min; MS (CIpos): m/z = 168 [M+H]⁺.

### Stufe 15b):

### 5-(3-Cyclobutylpropanoyl)-4-(5-fluor-2-methyl-4-oxo-4H-chromen-8-yl)-2,6-dimethyl-1,4-dihydropyridin-3-carbonitril

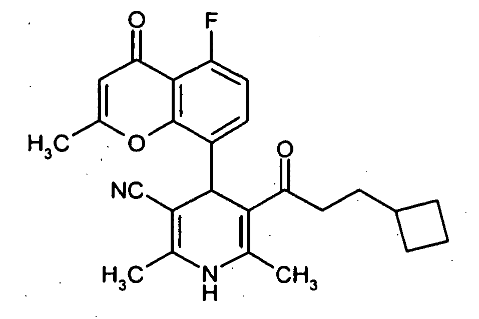

Eine Lösung von 100 mg (0.49 mmol) 5-Fluor-2-methyl-4-oxo-4*H*-chromen-8-carbaldehyd in 5 ml 2-Propanol wird mit 50.96 mg (0.49 mmol) Natrium-1-cyanoprop-1-en-2-olat, 81.12 mg (0.49 mmol) 5-Amino-1-cyclobutylhex-4-en-3-on und 0.04 ml (0.73 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die gelben Kristalle werden abgesaugt und über eine Analogix-Kartusche (F12M) weiter gereinigt (Laufmittel: Cyclohexan/Essigsäureethylester 2:1). Es werden 60.2 mg (29.5% d. Th.) der Titelverbindung erhalten.
LC-MS (Methode 2): Rₜ = 2.38 min;
MS (ESIpos): m/z = 421 [M+H]⁺
¹H-NMR (300 MHz, DMSO-d₆): δ = 9.3 (1H, s), 7.46 (1H, dd), 7.19 (1H, dd), 6.24 (1H, s), 5.2 (1H, s), 2.38 (3H, s), 2.3 (3H, s), 2.14-1.95 (4H, m), 1.85-1.6 (5H, m), 1.46-1.22 (5H, m).

### Beispiel 16

### 2,6-Dimethyl-4-(2-methyl-4-oxo-4H-chromen-8-yl)-5-(4-methylgentanoyl)-1,4-dihydropyridin-3-carbonitril

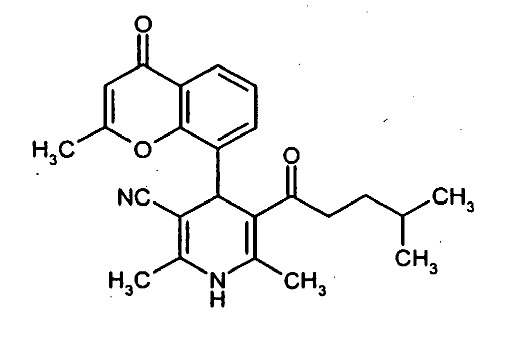

100 mg (0.53 mmol) 2-Methyl-4-oxo-4*H*-chromen-8-carbaldehyd werden mit 55.8 mg (0.53 mmol) Natrium-1-cyanoprop-1-en-2-olat, 82.4 mg (0.53 mmol) 2-Amino-7-methyloct-2-en-4-on (Beispiel 14, Stufe a) und 31.9 mg (0.53 mmol) Essigsäure in 5 ml 2-Propanol gelöst und unter Argon 6 h lang unter Rückfluß erhitzt. Das Lösungsmittel wird im Vakuum entfernt und der Rückstand mittels präparativer HPLC gereinigt. Es werden 47 mg (22% d. Th.) der Titelverbindung als gelber Feststoff erhalten.
LC-MS (Methode 1): Rₜ = 2.23 min; [M+H]⁺ = 391
¹H-NMR (300 MHz, DMSO-d₆): δ = 0.66 (m, 6H), 0.85 (m, 1H), 1.07 (m, 1H), 1.26 (m, 2H), 1.99 (s, 3H), 2.14 (m, 1H), 2.30 (s, 3H), 2.41 (s, 3H), 5.27 (s, 1H), 6.28 (s, 1H), 7.41 (t, 1H), 7.49 (dd, 1H), 7.88 (dd, 1H), 9.28 (s, 1H).

### Beispiel 17

### Cyclobutyl 5-cyano-4-(5,6-difluor-2-methyl-4-oxo-4H-chromen-8-yl)-2,6-dimethyl-1,4-dihydropyridin-3-carboxylat

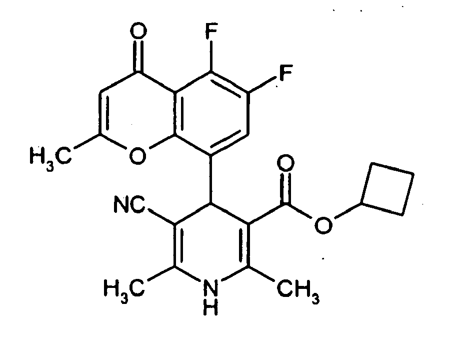

### Stufe 17a):

### 5,6-Difluor-2-methyl-4-oxo-4H-chromen-8-carbaldehyd

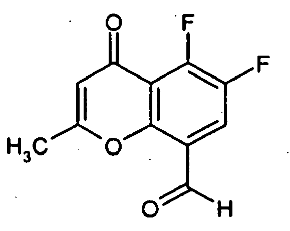

Die Titelverbindung wird in Analogie zu Beispiel 1, Stufe a-e, ausgehend von 1-(2,3-Difluor-6-hydroxyphenyl)ethanon erhalten.
LC-MS (Methode 1): Rₜ = 1.42 min; [M+H]⁺ = 225
¹H-NMR (300 MHz, CDCl₃): δ = 2.48 (s, 3H), 6.20 (s, 1H), 8.03 (dd, 1H), 10.56 (s, 1H).

### Stufe 17b):

### Cyclobutyl 5-cyano-4-(5,6-difluor-2-methyl-4-oxo-4H-chromen-8-yl)-2,6-dimethyl-1,4-dihydropyridin-3-carboxylat

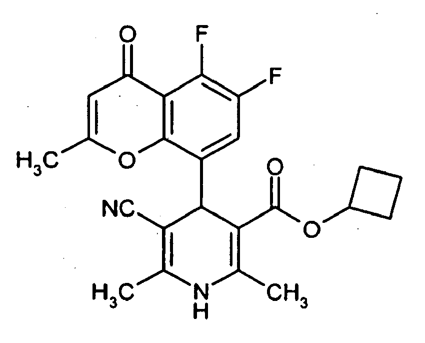

Eine Lösung von 100 mg (0.45 mmol) 5,6-Difluor-2-methyl-4-oxo-4*H*-chromen-8-carbaldehyd in 5 ml 2-Propanol wird mit 46.9 mg (0.45 mmol) Natrium-1-cyanoprop-1-en-2-olat, 69.23 mg (0.45 mmol) 3-Aminocrotonsäurecyclobutylester und 0.04 ml (0.67 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die gelben Kristalle werden abgesaugt und im Vakuumtrockenschrank bei 40°C getrocknet. Es werden 104.4 mg (54.9% d. Th.) der Titelverbindung erhalten.
LC-MS (Methode 3): Rₜ = 2.15 min;
MS (ESIpos): m/z = 427 [M+H]⁺
¹H-NMR (400 MHz, DMSO-d₆): δ = 9.45 (1H, s), 7.69 (1H, t), 6.26 (1H, s), 5.11 (1H, s), 4.79-4.68 (1H, m), 2.37 (3H, s), 2.31 (3H, s), 2.01 (3H, s), 2.23-2.11 (1H, m), 1.89-1.75 (1H, m), 1.65-1.42 (4H, m).

### Beispiel 18

### Cyclobutyl(4S)-5-cyano-4-(5,6-difluor-2-methyl-4-oxo-4H-chromen-8-yl)-2,6-dimethyl-1,4-dihydropyridin-3-carboxylat

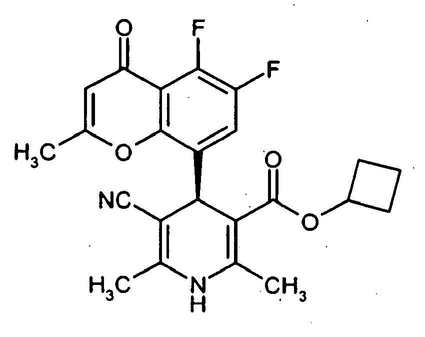

380 mg (0.89 mmol) racemisches Cyclobutyl 5-cyano-4-(5,6-difluor-2-methyl-4-oxo-4*H*-chromen-8-yl)-2,6-dimethyl-1,4-dihydropyridin-3-carboxylat (Beispiel 17) werden mittels präparativer HPLC an chiraler Phase (Methode 4) in die Enantiomeren getrennt:

### Enantiomer 1 (mit 4R-Konfiguration):

Ausbeute: 150 mg
Rₜ= 1.71 min.

### Enantiomer 2 (mit 4S-Konfiguration):

Ausbeute: 145.1 mg
Rₜ = 2.26 min; >99.5% ee
¹H-NMR (300 MHz, DMSO-d₆): δ = 9.39 (1H, s), 7.69 (1H, t), 6.26 (1H, s); 5.11 (1H, s), 4.8-4.66 (1H, m), 2.36 (3H, s), 2.31 (3H, s), 2.25-2.09 (1H, m), 2.00 (3H, s), 1.89-1.72 (1H, m), 1.65-1.38 (4H, m)
LC-MS (Methode 1): Rₜ = 2.1 min;
MS (ESIpos): m/z = 427 [M+H]⁺.

### Beispiel 19

### 5-(Cyclobutylacetyl)-4-(5,6-difluor-2-methyl-4-oxo-4H-chromen-8-yl)-2,6-dimethyl-1,4-dihydropyridin-3-carbonitril

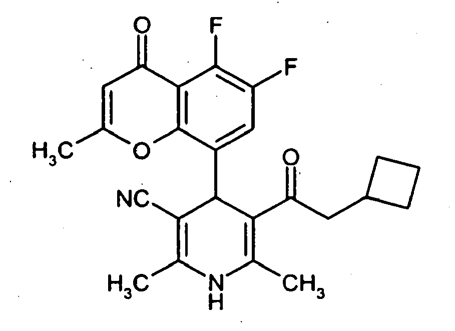

Eine Lösung von 100 mg (0.45 mmol) 5,6-Difluor-2-methyl-4-oxo-4H-chromen-8-carbaldehyd in 5 ml 2-Propanol wird mit 46.9 mg (0.45 mmol) Natrium-1-cyanoprop-1-en-2-olat, 85.44 mg (0.45 mmol) 4-Amino-1-cyclobutylpent-3-en-2-on (Beispiel 13, Stufe a) und 0.04 ml (0.67 mmol) Essigsäure versetzt und 3 h lang unter Rückfluß gerührt. Nach dem Abkühlen wird der Ansatz eingeengt. Der Rückstand wird über eine Analogix-Kartusche (F12M) gereinigt (Laufmittel: Cyclohexan/Essigsäureethylester 2:1). Es werden 70.9 mg (37.44% d. Th.) der Titelverbindung als gelbe Kristalle erhalten.
¹H-NMR (300 MHz, CDCl₃): δ = 7.15 (1H, dd), 6.15 (2H, s), 5.32 (1H, s), 2.5 (3H, s), 2.43 (3H, s), 2.37-2.2 (2H, m), 2.1 (3H, s), 2.1-1.92 (2H, m), 1.92-1.55 (3H, m), 1.6-1.4 (2H, m)
LC-MS (Methode 1): Rₜ = 2.11 min;
MS (ESIpos): m/z = 425 [M+H]⁺.

### Beispiel 20

### 5-(3-Cyclobutylpropanoyl)-4-(5,6-difluor-2-methyl-4-oxo-4H-chromen-8-yl)-2,6-dimethyl-1,4-dihydropyridin-3-carbonitril

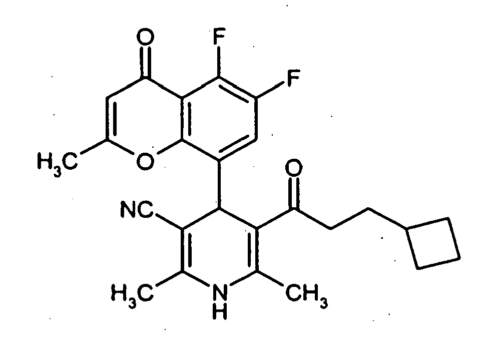

Eine Lösung von 100 mg (0.45 mmol) 5,6-Difluor-2-methyl-4-oxo-4H-chromen-8-carbaldehyd in 5 ml 2-Propanol wird mit 46.9 mg (0.45 mmol) Natrium-1-cyanoprop-1-en-2-olat, 74.61 mg (0.45 mmol) 5-Amino-1-cyclobutylhex-4-en-3-on (Beispiel 15, Stufe a) und 0.04 ml (0.67 mmol) Essigsäure versetzt und 3 h lang unter Rückfluß gerührt. Nach dem Abkühlen wird der Ansatz eingeengt. Der Rückstand wird über eine Analogix-Kartusche (F12M) gereinigt (Laufmittel: Cyclohexan/Essigsäureethylester 2:1). Es werden 71.2 mg (36.4% d. Th.) der Titelverbindung als gelbe Kristalle erhalten.
¹H-NMR (300 MHz, CDCl₃): δ = 7.14 (1H, dd), 6.15 (1H, s), 5.98 (1H, s), 5.32 (1H, s), 2.47 (3H, s), 2.44 (3H, s), 2.42-2.27 (2H, m), 2.1 (3H, s), 2.1-1.98 (1H, m), 1.98-1.68 (5H, m), 1.58-1.4 (3H, m)
LC-MS (Methode 1): Rₜ = 2.26 min;
MS (ESIpos): m/z = 439 [M+H]⁺.

### Beispiel 21

### 4-(5,6-Difluor-2-methyl-4-oxo-4H-chromen-8-yl)-2,6-dimethyl-5-(3-methylbutanoyl)-1,4-dihydropyridin-3-carbonitril

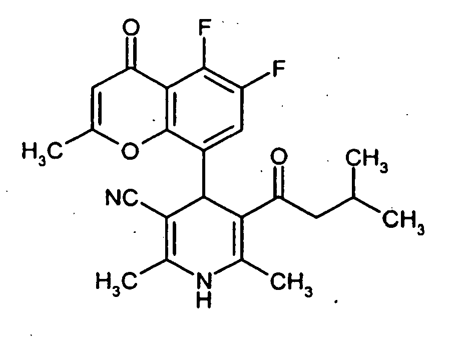

### Stufe 21a):

### 2-Amino-6-methylhept-2-en-4-on

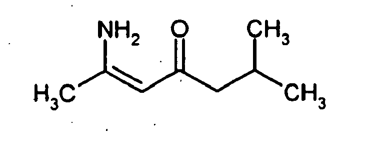

Die Herstellung erfolgt analog zu Beispiel 14 (Stufe 14a) ausgehend von 4.50 g (32.3 mmol) 5-Isobutyl-3-methylisoxazol [erhältlich analog zu C. Kashima et al., Bull. Chem. Soc. Jpn. 46, 310-313 (1973)].
Ausbeute: 4.02 g (88% d. Th.)
GC-MS (Methode 8): Rₜ = 5.30 min; MS (CIpos): m/z = 142 [M+H]⁺.

### Stufe 21b):

### 4-(5,6-Difluor-2-methyl-4-oxo-4H-chromen-8-yl)-2,6-dimethyl-5-(3-methylbutanoyl)-1,4-dihydropyridin-3-carbonitril

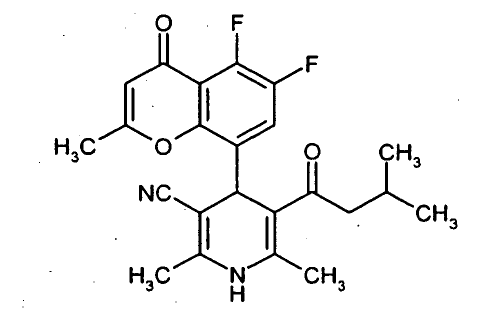

Eine Lösung von 100 mg (0.45 mmol) 5,6-Difluor-2-methyl-4-oxo-4*H*-chromen-8-carbaldehyd in 5 ml 2-Propanol wird mit 46.9 mg (0.45 mmol) Natrium-1-cyanoprop-1-en-2-olat, 63 mg (0.45 mmol) 2-Amino-6-methylhept-2-en-4-on und 0.04 ml (0.67 mmol) Essigsäure versetzt und 3 h lang unter Rückfluß gerührt. Nach dem Abkühlen wird der Ansatz eingeengt. Der Rückstand wird über eine Analogix-Kartusche (F12M) gereinigt (Laufmittel: Cyclohexan/Essigsäureethylester 2:1). Es werden 20.9 mg (11.36% d. Th.) der Titelverbindung als weiße Kristalle erhalten.
¹H-NMR (300 MHz, CDCl₃): δ = 7.2-7.1 (1H, m), 6.15 (1H, s), 5.9 (1H, s), 5.3 (1H, s), 2.48 (3H, s), 2.41 (3H, s), 2.38-2.28 (1H, m), 2.12-1.96 (5H, m), 0.88 (3H, d), 0.78 (3H, d)
LC-MS (Methode 2): Rₜ = 2.34 min;
MS (ESIpos): m/z = 413 [M+H]⁺.

### Beispiel 22

### 4-(5,6-Difluor-2-methyl-4-oxo-4H-chromen-8-yl)-2,6-dimethyl-5-(4-methylpentanoyl)-1,4-dihydropyridin-3-carbonitril

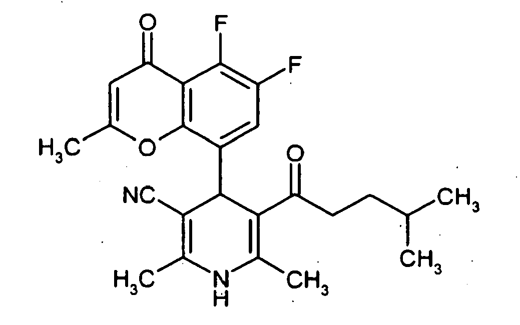

Eine Lösung von 100 mg (0.45 mmol) 5,6-Difluor-2-methyl-4-oxo-4*H*-chromen-8-carbaldehyd in 5 ml 2-Propanol wird mit 46.9 mg (0.45 mmol) Natrium-1-cyanoprop-1-en-2-olat, 69.25 mg (0.45 mmol) 2-Amino-7-methyloct-2-en-4-on (Beispiel 14, Stufe a) und 0.04 ml (0.67 mmol) Essigsäure versetzt und 3 h lang unter Rückfluß gerührt. Nach dem Abkühlen wird der Ansatz eingeengt. Der Rückstand wird über eine Analogix-Kartusche (F12M) gereinigt (Laufmittel: Cyclohexan/Essigsäureethylester 2:1). Es werden 88.1 mg (46.36% d. Th.) der Titelverbindung als gelbe Kristalle erhalten.
¹H-NMR (300 MHz, CDCl₃): δ = 7.22-7.12 (1H, m), 6.15 (1H, s), 6.1 (1H, d), 5.34 (1H, s), 2.47 (3H, s), 2.44 (3H, s), 2.2-2.08 (4H, m), 1.48-1.15 (4H, m), 0.8 (6H, d)
LC-MS (Methode 3): Rₜ = 2.17 min;
MS (ESIpos): m/z = 427 [M+H]⁺.

### Beispiel 23

### Propyl 5-cyano-4-(5,6-difluor-2-methyl-4-oxo-4H-chromen-8-yl)-2,6-dimethyl-1,4-dihydropyridin-3-carboxylat

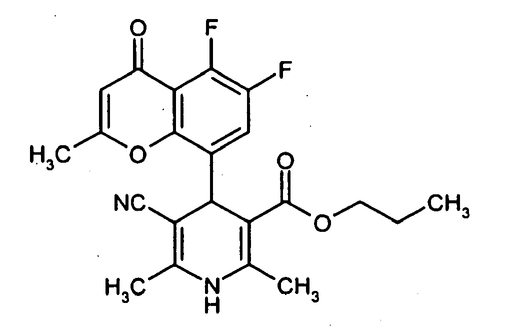

Eine Lösung von 100 mg (0.45 mmol) 5,6-Difluor-2-methyl-4-oxo-4*H*-chromen-8-carbaldehyd in 5 ml 2-Propanol wird mit 46.9 mg (0.45 mmol) Natrium-1-cyanoprop-1-en-2-olat, 63.88 mg (0.45 mmol) 3-Aminocrotonsäurepropylester und 0.04 ml (0.67 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die beigefarbenen Kristalle werden abgesaugt und im Vakuumtrockenschrank bei 40°C getrocknet. Es werden 74.6 mg (40.35% d. Th.) der Titelverbindung erhalten.
¹H-NMR (400 MHz, DMSO-d₆): δ = 9.47 (1H, s), 7.61 (1H, t), 6.25 (1H, s), 5.12 (1H, s), 3.87-3.72 (2H, m), 2.36 (3H, s), 2.31 (3H, s), 1.99 (3H, s), 1.39-1.28 (2H, m), 0.6 (3H, t)
LC-MS (Methode 3): Rₜ = 2.11 min;
MS (ESIpos): m/z = 415 [M+H]⁺.

### Beispiel 24

### Isopropyl 5-cyano-4-(5,6-difluor-2-methyl-4-oxo-4H-chromen-8-yl)-2,6-dimethyl-1,4-dihydropyridin-3-carboxylat

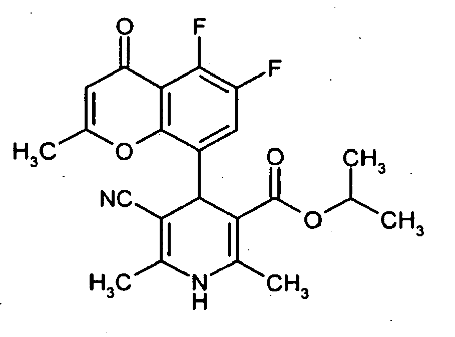

Eine Lösung von 100 mg (0.45 mmol) 5,6-Difluor-2-methyl-4-oxo-4*H*-chromen-8-carbaldehyd in 5 ml 2-Propanol wird mit 46.9 mg (0.45 mmol) Natrium-1-cyanoprop-1-en-2-olat, 63.88 mg (0.45 mmol) 3-Aminocrotonsäureisopropylester und 0.04 ml (0.67 mmol) Essigsäure versetzt und 3 h lang unter Rückfluß gerührt. Nach dem Abkühlen wird der Ansatz eingeengt. Der Rückstand wird in Dichlormethan aufgenommen und mit Wasser gewaschen. Die organische Phase wird über Natriumsulfat getrocknet und eingeengt. Der erhaltene Rückstand wird aus Diethylether kristallisiert. Die beigefarbenen Kristalle werden abgesaugt und im Vakuumtrockenschrank bei 40°C getrocknet. Es werden 105.9 mg (57.3% d. Th.) der Titelverbindung erhalten.
¹H-NMR (400 MHz, DMSO-d₆): δ = 9.34 (1H, s), 7.65 (1H, t), 6.26 (1H, s), 5.11 (1H, s), 4.76-4.63 (1H, m), 2.37 (3H, s), 2.31 (3H, s), 2.0 (3H, s), 1.07 (3H, d), 0.74 (3H, d)
LC-MS (Methode 3): Rₜ = 2.1 min;
MS (ESIpos): m/z = 415 [M+H]⁺.

### Beispiel 25

### Propyl 5-cyano-2-methyl-4-(2-methyl-4-oxo-4H-chromen-8-yl)-6-(trifluormethyl)-1,4-dihydropyridin-3-carboxylat

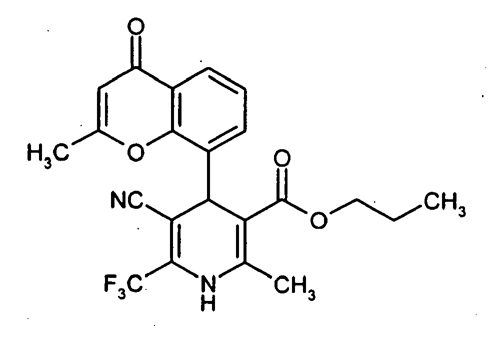

### Stufe 25a):

### Propyl 2-[(2-methyl-4-oxo-4H-chromen-8-yl)methylen]-3-oxobutanoat

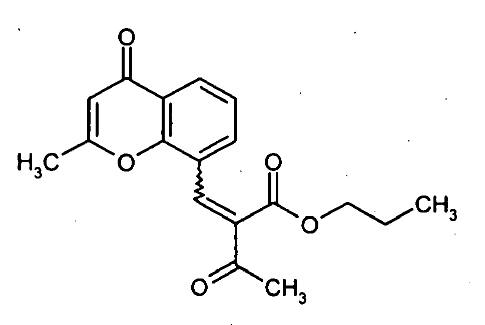

2-Methyl-4-oxo-4*H*-chromen-8-carbaldehyd (2 g, 10.62 mmol) und Acetessigsäurepropylester (1.53 g, 10.62 mmol) werden in 500 ml Dichlormethan gelöst und nach Zugabe von Eisessig (0.76 ml, 13.28 mmol) und Piperidin (0.1 ml, 1.06 mmol) 18 h lang am Wasserabscheider unter Rückfluss erhitzt. Nach dem Abkühlen wird das Reaktionsgemisch mit 50 ml Dichlormethan verdünnt, mit 20 ml Natriumchlorid-Lösung gewaschen, die organische Phase über Natriumsulfat getrocknet und das Lösungsmittel im Vakuum entfernt. Man erhält 3.3 g (99% d. Th.) der Titelverbindung als E/Z-Isomerengemisch.
LC-MS (Methode 1): Rₜ = 1.92 und 2.06 min; MS (ESIpos): m/z = 315 [M+H]⁺.

### Stufe 25b):

### Propyl 5-cyano-2-methyl-4-(2-methyl-4-Oxo-4H-chromen-8-yl)-6-(trifluormethyl)-1,4-dihydropyridin-3-carboxylat

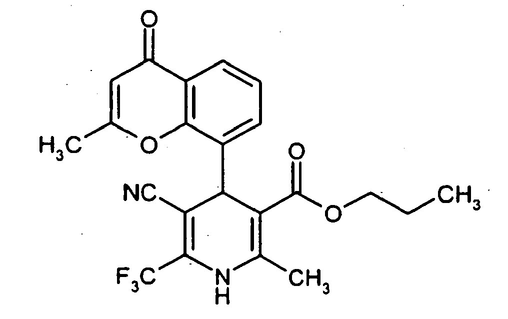

Eine Lösung von 295 mg (0.94 mmol) Propyl 2-[(2-methyl-4-oxo-4*H*-chromen-8-yl)methylen]-3-oxobutanoat in 20 ml 2-Propanol wird mit 127.71 mg (0.94 mmol) 3-Amino-4,4,4-trifluorbut-2-en-nitril [Darstellung analog K. Krespan, J. Org. Chem. 34, 42-45 (1969)] und 15.8 mg (0.14 mmol) Kalium-*tert*.-butylat versetzt und 12 h lang unter Rückfluss gerührt. Nach dem Abkühlen wird der Ansatz eingeengt. Der Rückstand wird über eine Analogix-Kartusche (F12M) gereinigt (Laufmittel: Cyclohexan/Essigsäureethylester 2:1). Nach Einengen der Produktfraktionen wird der Rückstand aus Diethylether kristallisiert. Es werden 88.4 mg (20.7% d. Th.) der Titelverbindung als weiße Kristalle erhalten.
¹H-NMR (400 MHz, CDCl₃): δ = 8.13 (1H, d), 7.48 (1H, d), 7.38 (1H, t), 6.28 (1H, s), 6.21 (1H, s), 5.49 (1H, s), 3.9 (2H, t), 2.5 (3H, s), 2.43 (3H, s), 1.48-1.35 (2H, m), 0.7 (3H, t)
LC-MS (Methode 3): Rₜ = 2.34 min;
MS (ESIpos): m/z = 433 [M+H]⁺

### Beispiel 26

### Propyl (4S)-5-cyano-2-methyt-4-(2-methy)-4-oxo-4H-chromen-8-yl)-6-(trifluormethyl)-1,4-dihydropyridin-3-carboxylat

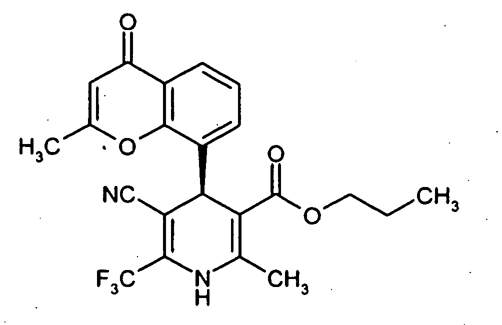

827 mg (1.9 mmol) racemisches Propyl 5-cyano-2-methyl-4-(2-methyl-4-oxo-4*H*-chromen-8-yl)-6-(trifluormethyl)-1,4-dihydropyridin-3-carboxylat (Beispiel 25) werden mittels präparativer HPLC an chiraler Phase (Methode 5) in die Enantiomeren getrennt:

### Enantiomer 1 (mit 4S-Konfiguration):

Ausbeute: 371 mg
Rₜ = 3.85 min; >98.1 % ee
¹H-NMR (400 MHz, CDCl₃): δ = 8.13 (1H, d), 7.48 (1H, d), 7.38 (1H, t), 6.35 (1H, s), 6.21 (1H, s), 5.5 (1H, s), 3.92 (2H, t), 2.53 (3H, s), 2.45 (3H, s), 1.48-1.38 (2H, m), 0.7 (3H, t)
LC-MS (Methode 3): Rₜ = 2.30 min;
MS (ESIpos): m/z = 433 [M+H]⁺.

### Enantiomer 2 (mit 4R-Konfiguration):

Ausbeute: 388 mg
Rₜ = 4.75 min.

### Beispiel 27

### Cyclobutyl 5-cyano-2-methyl-4-(2-methyl-4-oxo-4H-chromen-8-yl)-6-(trifluormethyl)-1,4-dihydropyridin-3-carboxylat

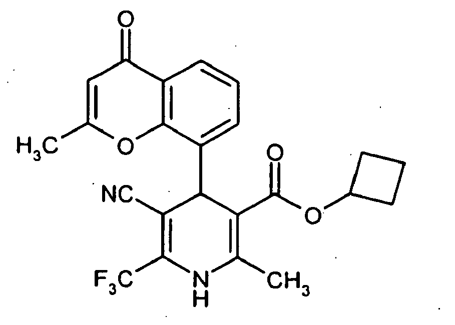

### Stufe 27a):

### Cyclobutyl 2-[(2-methyl-4-oxo-4H-chromen-8-yl)methylen]-3-oxobutanoat

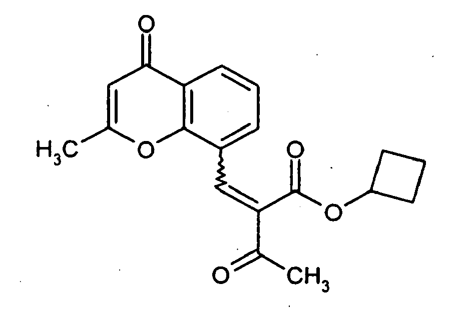

Die Titelverbindung wird in Analogie zu Beispiel 25 (Stufe 25a) ausgehend von 2-Methyl-4-oxo-4H-chromen-8-carbaldehyd (2 g, 10.62 mmol) und Acetessigsäurecyclobutylester (1.66 g, 10.62 mmol) hergestellt. Man erhält 3.4 g (98% d. Th.) der Titelverbindung als *E*/*Z*-Isomerengemisch.
LC-MS (Methode 3): Rₜ = 2.15 und 2.29 min; MS (ESIpos): m/z = 327 [M+H]⁺.

### Stufe 27b):

### Cyclobutyl 5-cyano-2-methyl-4-(2-methyl-4-oxo-4H-chromen-8-yl)-6-(trifluormethyl)-1,4-dihydropyridin-3-carboxylat

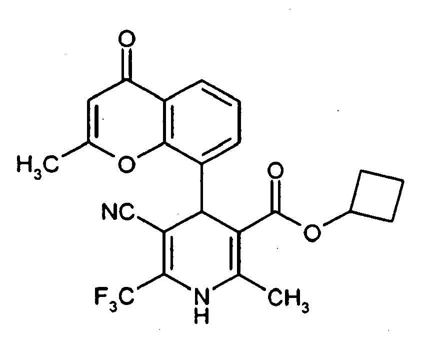

Eine Lösung von 345 mg (1.06 mmol) Cyclobutyl 2-[(2-methyl-4-oxo-4*H*-chromen-8-yl)-methylen]-3-oxobutanoat in 30 ml 2-Propanol wird mit 143.85 mg (1.06 mmol) 3-Amino-4,4,4-trifluorbut-2-ennitril [Darstellung analog K. Krespan, J. Org. Chem. 34,42-45 (1969)] und 17.8 mg (0.16 mmol) Kalium-*tert*.-butylat versetzt und 12 h lang unter Rückfluß gerührt. Nach dem Abkühlen wird der Ansatz eingeengt. Der Rückstand wird über eine Analogix-Kartusche (F12M) gereinigt (Laufmittel: Cyclohexan/Essigsäureethylester 2:1). Nach Einengen der Produktfraktionen wird der Rückstand aus Diethylether kristallisiert. Es werden 50.9 mg (10.8% d. Th.) der Titelverbindung als weiße Kristalle erhalten.
¹H-NMR (300 MHz, CDCl₃): δ = 8.15 (1H, d), 7.5 (1H, d), 7.38 (1H, t), 6.23-6.19 (2H, m), 5.49 (1H, s), 4.9-4.78 (1H, m), 2.5 (3H, s), 2.45 (3H, s), 2.32-2.2 (1H, m), 2.2-2.04 (1H, m), 1.92-1.75 (1H, m), 1.7-1-.48 (3H, m)
LC-MS (Methode 2): Rₜ = 2.55 min;
MS (ESIpos): m/z = 445 [M+H]⁺.

### Beispiel 28

### Cyclobutyl (4S)-5-cyano-2-methyl-4-(2-methyl-4-oxo-4H-chromen-8-yl)-6-(trifluormethyl)-1,4-dihydropyridin-3-carboxylat

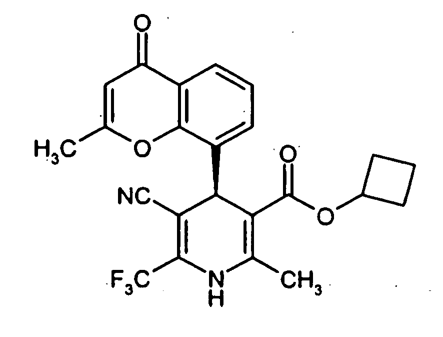

497 mg (1.1 mmol) racemisches Cyclobutyl 5-cyano-2-methyl-4-(2-methyl-4-oxo-4*H*-chromen-8-yl)-6-(trifluormethyl)-1,4-dihydropyridin-3-carboxylat (Beispiel 27) werden mittels präparativer HPLC an chiraler Phase (Methode 6) in die Enantiomeren getrennt:

### Enantiomer 1 (mit 4R-Konfiguration):

Ausbeute: 252 mg
Rₜ = 4.00 min.

### Enantiomer 2 (mit 4S-Konfiguration):

Ausbeute: 245 mg
Rₜ = 5.17 min; >99.5% ee
¹H-NMR (300 MHz, CDCl₃): δ = 8.15 (1H, d), 7.5 (1H, d), 7.38 (1H, t), 6.25 (1H, s), 6.22 (1H, s), 5.48 (1H, s), 4.89-4.79 (1H, m), 2.5 (3H, s), 2.45 (3H, s), 2.3-2.2 (1H, m), 2.15-2.08 (1H, m), 1.9-1.79 (1H, m), 1.68-1.6 (1H, m), 1.6-1.49 (2H, m)
LC-MS (Methode 3): Rₜ = 2.35 min;
MS (ESIpos): m/z = 445 [M+H]⁺.

### Beispiel 29

### Ethyl 5-cyano-2-methyl-4-(2-methyl-4-oxo-4H-chromen-8-yl)-6-(trifluormethyl)-1,4-dihydropyridin-3-carboxylat

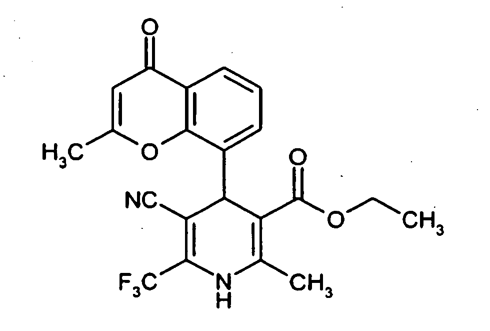

### Stufe 29a):

### Ethyl 2-[(2-methyl-4-oxo-4H-chromen-8-yl)methylen]-3-oxobutanoat

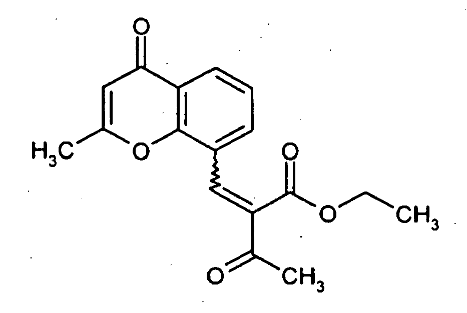

Die Titelverbindung wird in Analogie zu Beispiel 25 (Stufe 25a) ausgehend von 2-Methyl-4-oxo-4H-chromen-8-carbaldehyd (200 mg, 1.062 mmol) und Acetessigsäureethylester (138 mg, 1.062 mmol) hergestellt. Man erhält 309 mg (97% d. Th.) der Titelverbindung als E/Z-Isomerengemisch.
LC-MS (Methode 3): Rₜ = 1.94 min; MS (ESIpos): m/z = 301 [M+H]⁺.

### Stufe 29b):

### Ethyl 5-cyano-2-methyl-4-(2-methyl-4-oxo-4H-chromen-8-yl)-6-(trifluormethyl)-1,4-dihydropyridin-3-carboxylat

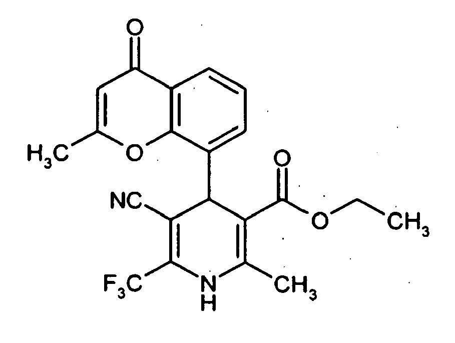

Eine Lösung von 310 mg (1.03 mmol) Ethyl 2-[(2-methyl-4-oxo-4*H*-chromen-8-yl)methylen]-3-oxobutanoat in 30 ml 2-Propanol wird mit 140.47 mg (1.03 mmol) 3-Amino-4,4,4-trifluorbut-2-en-nitril [Darstellung analog K. Krespan, J. Org. Chem. 34, 42-45 (1969)] und 17.38 mg (0.15 mmol) Kalium-*tert*.-butylat versetzt und 12 h lang unter Rückfluss gerührt. Nach dem Abkühlen wird der Ansatz eingeengt. Der Rückstand wird über eine Analogix-Kartusche (F12M) gereinigt (Laufmittel: Cyclohexan/Essigsäureethylester 2:1). Nach Einengen der Produktfraktionen wird der Rückstand aus Diethylether kristallisiert. Es werden 50.9 mg (10.8% d. Th.) der Titelverbindung als weiße Kristalle erhalten.
¹H-NMR (300 MHz, CDCl₃): δ = 8.15 (1H, d), 7.5 (1H, d), 7.38 (1H, t), 6.28 (1H, s), 6.21 (1H, s), 5.48 (1H, s), 4.0 (2H, q), 2.5 (3H, s), 2.44 (3H; s), 1.04 (3H, t)
LC-MS (Methode 2): Rₜ = 2.26 min;
MS (ESIpos): m/z = 419 [M+H]⁺.

### Beispiel 30

### Ethyl (4S)-5-cyano-2-methyl-4-(2-methyl-4-oxo-4H-chromen-8-yl)-6-(trifluormethyl)-1,4-dihydropyridin-3-carboxylat

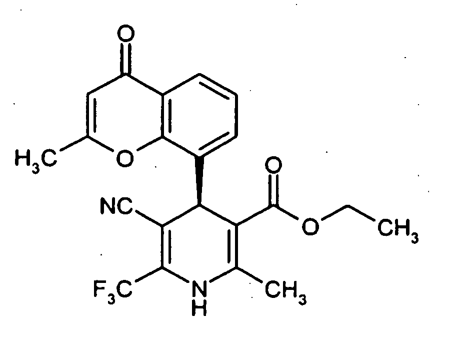

830 mg (1.98 mmol) racemisches Ethyl 5-cyano-2-methyl-4-(2-methyl-4-oxo-4*H*-chromen-8-yl)-6-(trifluormethyl)-1,4-dihydropyridin-3-carboxylat (Beispiel 29) werden mittels präparativer HPLC an chiraler Phase (Methode 7) in die Enantiomeren getrennt:

### Enantiomer 1 (mit 4R-Konfiguration):

Ausbeute: 377 mg
Rₜ = 4.28 min.

### Enantiomer 2 (mit 4S-Konfiguration):

Ausbeute: 339 mg
Rₜ = 5.69 min; >99.5% ee.

### Beispiel 31

### Ethyl 5-cyano-4-(6-fluor-2-methyl-4-oxo-4H-chromen-8-yl)-2-methyl-6-(trifluormethyl)-1,4-dihydropyridin-3-carboxylat

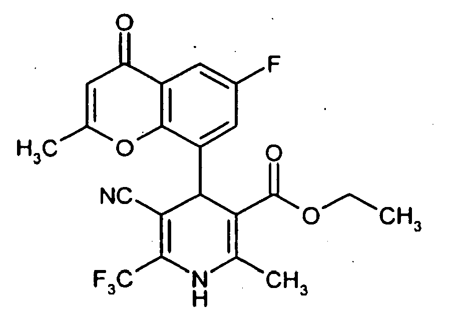

### Stufe 31a):

### 6-Fluor-2-methyl-4-oxo-4H-chromen-8-carbaldehyd

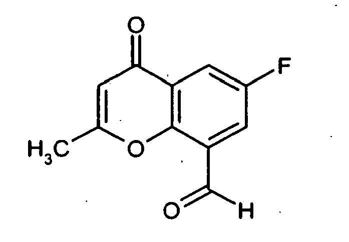

Die Titelverbindung wird in Analogie zu Beispiel 1, Stufe a-e, ausgehend von 1-(5-Fluor-2-hydroxyphenyl)ethanon erhalten.
LC-MS (Methode 1): Rₜ = 1.42 min; [M+H]⁺ = 207
¹H-NMR (300 MHz, CDCl₃): δ = 2.49 (s, 3H), 6.27 (s, 1H), 7.90 (dd, 1H), 8.08 (dd, 1H), 10.64 (s, 1H).

### Stufe 31b):

### Ethyl 2-[(6-fluor-2-methyl-4-oxo-4H-chromen-8-yl)methylen]-3-oxobutanoat

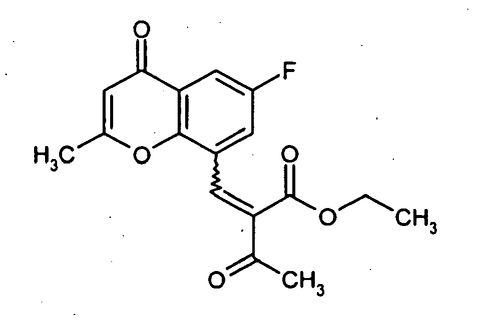

Die Titelverbindung wird in Analogie zu Beispiel 25 (Stufe 25a) ausgehend von 6-Fluor-2-methyl-4-oxo-4H-chromen-8-carbaldehyd (2.18 g, 10.62 mmol) und Acetessigsäureethylester (1.33 g, 10.62 mmol) hergestellt. Man erhält 3.30 g (98% d. Th.) der Titelverbindung als *E*/*Z*-Isomerengemisch.
LC-MS (Methode 1): Rₜ = 1.88 und 1.99 min; MS (ESIpos): m/z = 319 [M+H]⁺.

### Stufe 31c):

### Ethyl 5-cyano-4-(6-fluor-2-methyl-4-oxo-4H-chromen-8-yl)-2-methyl-6-(trifluormethyl)-1,4-dihydropyridin-3-carboxylat

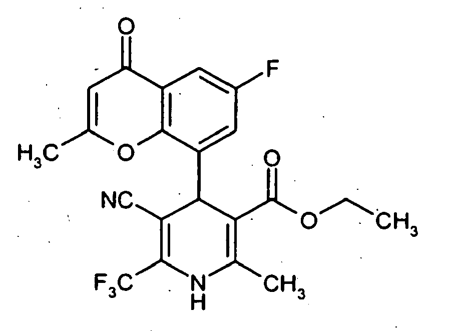

Eine Lösung von 230 mg (0.72 mmol) Ethyl 2-[(6-fluor-2-methyl-4-oxo-4H-chromen-8-yl)-methylen]-3-oxobutanoat in 10 ml 2-Propanol wird mit 98.34 mg (0.72 mmol) 3-Amino-4,4,4-trifluorbut-2-ennitril [Darstellung analog K. Krespan, J. Org. Chem. 34, 42-45 (1969)] und 12.2 mg (0.11 mmol) Kalium-*tert*.-butylat versetzt und 12 h lang unter Rückfluss gerührt. Nach dem Abkühlen wird der Ansatz eingeengt. Der Rückstand wird über eine Analogix-Kartusche (F12M) gereinigt (Laufmittel: Cyclohexan/Essigsäureethylester 2:1). Die erhaltenen Kristalle werden über präparative HPLC weiter gereinigt. Es werden 27.4 mg (8.69% d. Th.) der Titelverbindung als gelbe Kristalle erhalten.
¹H-NMR (300 MHz, DMSO-d₆): δ = 10.1 (1H, s), 7.65 (1H, dd), 7.55 (1H, dd), 6.33 (1H, s), 5.32 (1H, s), 3.9 (2H, q), 2.4 (6H, s), 0.95 (3H, t)
LC-MS (Methode 2): Rₜ = 2.41 min;
MS (ESIpos): m/z = 437 [M+H]⁺.

### Beispiel 32

### Isopropyl 5-cyano-2,6-dimethyl-4-(2-methyl-5-nitro-4-oxo-4H-chromen-8-yl)-1,4-dihydropyridin-3-carboxylat

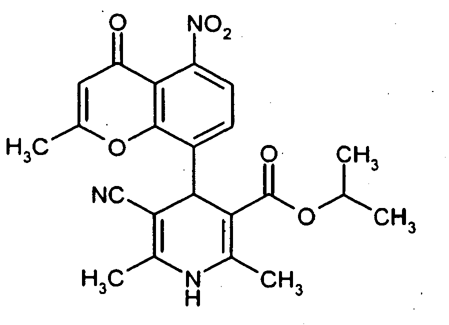

### Stufe 32a:

### 1-(2-Hydroxy-3-methylphenyl)-2-(triphenylphosphoranyliden)-ethanon

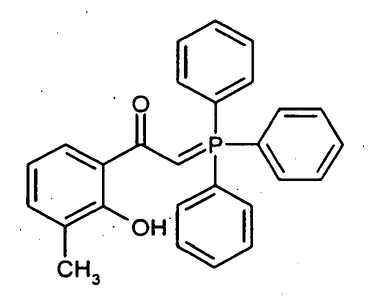

97.3 g (240.7 mmol) Methyltriphenylphosphoniumiodid werden unter Argon in 800 ml absolutem THF langsam mit 206.8 ml (330.9 mmol) einer 1.6 molaren n-Butyllithium-Lösung in n-Hexan versetzt. Man rührt 3 h bei Raumtemperatur. Anschließend werden zu der Reaktionsmischung 20.0 g (120.3 mmol) 2-Hydroxy-3-methylbenzoesäuremethylester in 200 ml absolutem THF getropft. Man rührt 3 h bei 60°C nach. Nach Abkühlen auf Raumtemperatur wird das ausgefallene Lithiumiodid abfiltriert. Das Filtrat wird im Vakuum eingeengt und der Rückstand aus Methanol umkristallisiert. Es werden 27 g (56% d. Th.) der Titelverbindung erhalten.
LC-MS (Methode 2): Rₜ = 2.12 min; [M+H]⁺ = 411.

### Stufe 32b):

### 2,8-Dimethyl-4H-chromen-4-on

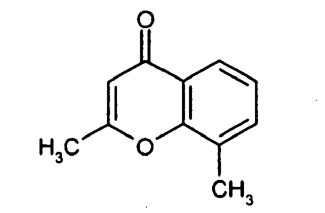

27.5 g (67 mmol) 1-(2-Hydroxy-3-methylphenyl)-2-(triphenylphosphoranyliden)-ethanon werden in 200 ml absolutem Toluol zum Rückfluß erhitzt. Man tropft langsam 13.7 g (134 mmol) Essigsäureanhydrid und 11.1 g (141 mmol) Pyridin zu dieser Lösung. Die Reaktionsmischung wird anschließend 6 h unter Rückfluß erhitzt. Nach Abkühlen auf Raumtemperatur wird die Lösung mit gesättigter Natriumcarbonat-Lösung gewaschen und über Natriumsulfat getrocknet. Das Lösungsmittel wird im Vakuum entfernt und der Rückstand säulenchromatogaphisch gereinigt (Laufmittel: Cyclohexan/Ethylacetat 7:3 → 4:6). Es werden 7.5 g (64% d. Th.) der Titelverbindung erhalten.
LC-MS (Methode 3): Rₜ =1.99 min; [M+H]⁺ = 175
¹H-NMR (300 MHz, DMSO-d₆): δ = 2.41 (s, 3H), 2.44 (s, 3H), 6.24 (s, 3H), 7.34 (t, 1H), 7.63 (dd, 1H), 7.83 (dd, 1H).

### Stufe 32c):

### 2,8-Dimethyl-5-nitro-4H-chromen-4-on

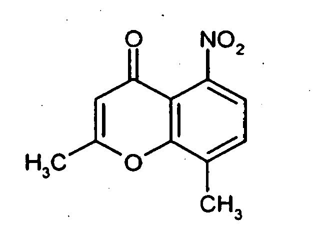

2 g (11.48 mmol) 2,8-Dimethyl-4*H*-chromen-4-on werden in 15 ml konzentrierter Schwefelsäure gelöst und bei 0°C mit 0.7 g (11.48 mmol) rauchender Salpetersäure versetzt, wobei die Temperatur 5°C nicht überschreiten sollte. Anschließend wird 1 h bei Raumtemperatur nachgerührt. Die Reaktionsmischung wird auf Eiswasser gegossen, wobei ein farbloser Feststoff ausfällt. Dieser wird abfiltriert und mehrfach mit Wasser und eiskaltem Methanol gewaschen. Es werden 2.3 g (90.8% d. Th.) der Titelverbindung erhalten.
LC-MS (Methode 2): Rₜ = 1.74 min; [M+H]⁺ = 220 ¹H-NMR (300 MHz, DMSO-d₆): δ = 2.41 (s, 3H), 2.48 (s, 3H), 6.34 (s, 1H), 7.65 (d, 1H), 7.80 (d, 1H).

### Stufe 32d):

### 8-(Dibrommethyl)-2-methyl-5-nitro-4H-chromen-4-on

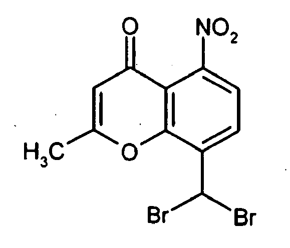

350 mg (1.59 mmol) 2,8-Dimethyl-5-nitro-4*H*-chiomen-4-on werden in 20 ml Tetrachlorkohlenstoff gelöst und mit 625 mg (3.51 mmol) *N*-Bromsuccinimid und 26.2 mg (0.16 mmol) 2,2'-Azobis-2-methylpropannitril über Nacht unter Rückfluß erhitzt. Nach Abkühlen auf Raumtemperatur wird der ausgefallene Feststoff abfiltriert und verworfen. Das Filtrat wird im Vakuum eingeengt und der Rückstand ohne Reinigung weiter umgesetzt.
LC-MS (Methode 1): Rₜ =2.21 min; [M+M]⁺ = 376.

### Stufe 32e):

### 2-Methyl-5-nitro-4-oxo-4H-chromen-8-carbaldehyd

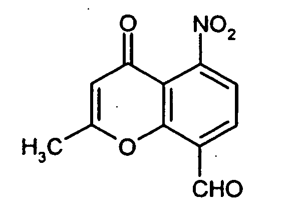

175 mg (0.47 mmol) 8-(Dibrommethyl)-2-methyl-5-nitro-4*H*-chromen-4-on werden mit 151 mg (1.29 mmol) *N*-Methylmorpholin-*N*-oxid unter Zusatz von Molekularsieb in 15 ml Acetonitril über Nacht unter Rückfluß erhitzt. Nach Filtration über Kieselgur wird das Lösungsmittel im Vakuum entfernt und der Rückstand mittels präparativer HPLC gereinigt. Es werden 23 mg (24% d. Th.) der Titelverbindung erhalten.
LC-MS (Methode 2): Rₜ =1.88 min; [M+H]⁺ = 234

### Stufe 32f):

### Isopropyl 5-cyano-2,6-dimethyl-4-(2-methyl-5-nitro-4-oxo-4H-chromen-8-yl)-1,4-dihydropyridin-3-carboxylat

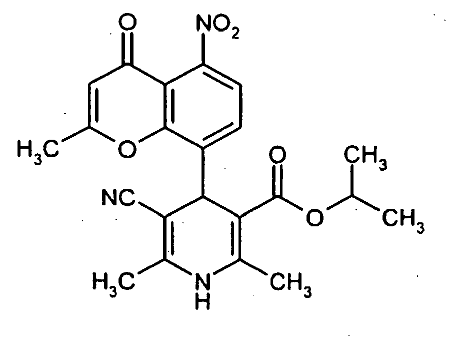

32 mg (0.137 mmol) 2-Methyl-5-nitro-4-oxo-4*H*-chromen-8-carbaldehyd werden mit 19.7 mg (0.137 mmol) Acetessigsäureisopropylester, 11.26 mg (0.137 mmol) 3-Aminocrotonsäurenitril und 8.24 mg (0.137 mmol) Essigsäure in 3 ml 2-Propanol gelöst und unter Argon 6 h lang unter Rückfluß erhitzt. Das Lösungsmittel wird im Vakuum entfernt und der Rückstand mittels präparativer HPLC gereinigt. Es werden 13 mg (22.3% d. Th.) der Titelverbindung als gelber Feststoff erhalten.
LC-MS (Methode 2): Rₜ = 2.38 min; [M+H]⁺ = 424
¹H-NMR (300 MHz, DMSO-d₆): δ = 0.71 (d, 3H), 1.07 (d, 3H), 2.00 (s, 3H), 2.32 (s, 3H), 2.43 (s, 3H), 4.68 (m, 1H), 5.19 (s, 1H), 6.40 (s, 1H), 7.74 (s, 2H), 9.38 (s, 1H).

### Beispiel 33

### Isopropyl 5-cyano-4-(5-cyano-2-methyl-4-oxo-4H-chromen-8-yl)-2,6-dimethyl-1,4-dihydropyridin-3-carboxylat

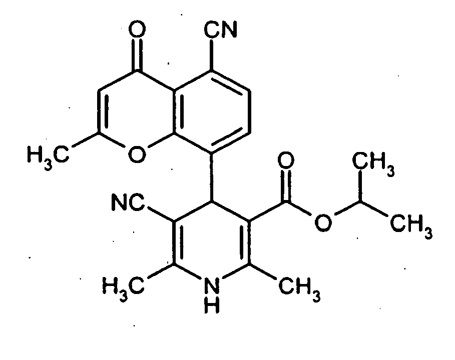

### Stufe 33a):

### 5-Amino-2,8-dimethyl-4H-chromen-4-on

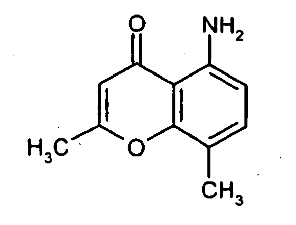

1.78 g (8.12 mmol) 2,8-Dimethyl-5-nitro-4*H*-chromen-4-on (Beispiel 32, Stufe c) werden mit 9.16 g (40.6 mmol) Zinn(II)chlorid-Dihydrat in 70 ml Ethylacetat über Nacht auf 70°C erhitzt. Nach Abkühlen auf Raumtemperatur wird die Reaktionsmischung mit gesättigter Natriumhydrogencarbonat-Lösung auf pH 9-10 gebracht. Nach Filtration über Kieselgur wird die organische Phase abgetrennt und die wässrige Phase mehrfach mit Ethylacetat extrahiert. Die vereinigten organischen Phasen werden mit gesättigter Natriumchlorid-Lösung gewaschen. Nach Trocknen über Natriumsulfat wird das Lösungsmittel im Vakuum entfernt. Es werden 1.5 g (99% d. Th.) der Titelverbindung erhalten.
LC-MS (Methode 1): Rₜ = 1.74 min; [M+H]⁺ = 190
¹H-NMR (300 MHz, DMSO-d₆): δ = 2.17 (s, 3H), 2.30 (s, 3H), 6.00 (s, 1H), 6.42 (d, 1H), 7.17 (br. s, 2H), 7.18 (d, 1H).

### Stufe 33b):

### 2,8-Dimethyl-4-oxo-4H-chromen-5-carbonitril

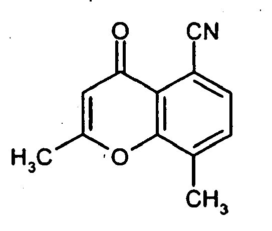

0.2 g (1.06 mmol) 5-Amino-2,8-dimethyl-4*H*-chromen-4-on werden in 5 ml 45%-iger Schwefelsäure gelöst und auf 0°C abgekühlt. Anschließend wird eine Lösung von 0.11 g (1.6 mmol) Natriumnitrit in 5 ml Wasser so zugetropft, daß die Temperatur 5°C nicht überschreitet. Man rührt 90 min bei 0°C nach und neutralisiert dann mit Natriumhydrogencarbonat. Anschließend wird eine 0°C kalte Lösung von 0.12 g (1.37 mmol) Kupfer(I)cyanid und 0.07 g (1.58 mmol) Natriumcyanid in 10 ml Wasser, überschichtet mit 50 ml Ethylacetat, zugesetzt. Man rührt 45 min bei 0°C nach. Anschließend wird die Reaktionsmischung über Kieselgur filtriert. Die organische Phase wird abgetrennt und die wässrige Phase mit Ethylacetat extrahiert. Die vereinigten organischen Phasen werden mit gesättigter Natriumchlorid-Lösung gewaschen. Nach Trocknen über Magnesiumsulfat wird das Lösungsmittel im Vakuum entfernt. Man erhält 0.14 g (67% d. Th.) der Titelverbindung.
LC-MS (Methode 3): Rₜ = 1.75 min; [M+H]⁺ = 200.

### Stufe 33c):

### 8-(Dibrommethyl)-2-methyl-4-oxo-4H-chromen-5-carbonitril

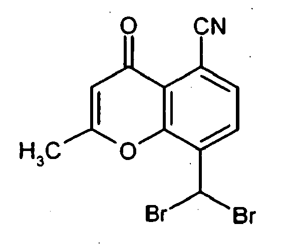

142 mg (0.71 mmol) 2,8-Dimethyl-4-oxo-4*H*-chromen-5-carbonitril werden in 20 ml Tetrachlorkohlenstoff gelöst und mit 279 mg (1.56 mmol) *N*-Bromsuccinimid und 11.7 mg (0.07 mmol) 2,2'-Azobis-2-methylpropannitril über Nacht unter Rückfluß erhitzt. Nach Abkühlen auf Raumtemperatur wird der ausgefallene Feststoff abfiltriert und verworfen. Das Filtrat wird im Vakuum eingeengt und der Rückstand ohne Reinigung weiter umgesetzt.

### Stufe 33d):

### 8-Formyl-2-methyl-4-oxo-4H-chromen-5-carbonitril

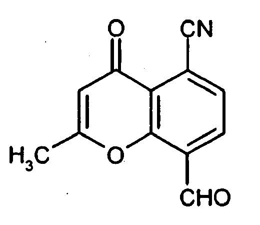

260 mg (0.72 mmol) 8-(Dibrommethyl)-2-methyl-4-oxo-4*H*-chromen-5-carbonitril werden mit 187 mg (1.6 mmol) *N*-Methylmorpholin-*N*-oxid unter Zusatz von Molekularsieb in 15 ml Acetonitril über Nacht unter Rückfluß erhitzt. Nach Filtration über Kieselgur wird das Lösungsmittel im Vakuum entfernt und der Rückstand mittels präparativer HPLC gereinigt. Es werden 23 mg (15% d. Th.) der Titelverbindung erhalten.
LC-MS (Methode 2): Rₜ = 1.58 min; [M+H]⁺ = 214.

### Stufe 33e):

### Isopropyl 5-cyano-4-(5-cyano-2-methyl-4-oxo-4H-chromen-8-yl)-2,6-dimethyl-1,4-dihydropyridin-3-carboxylat

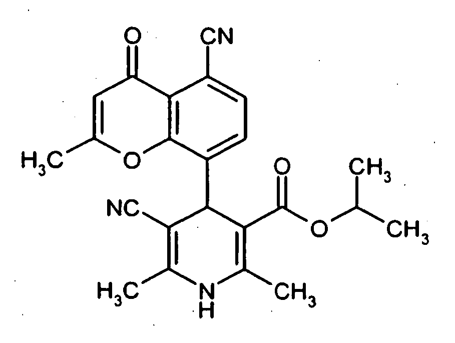

21 mg (0.09 mmol) 8-Formyl-2-methyl-4-oxo-4*H*-chromen-5-carbonitril werden mit 14 mg (0.09 mmol) Acetessigsäureisopropylester, 8 mg (0.09 mmol) 3-Aminocrotonsäurenitril und 6 mg (0.09 mmol) Essigsäure in 2 ml 2-Propanol gelöst und unter Argon 6 h lang unter Rückfluß erhitzt. Das Lösungsmittel wird im Vakuum entfernt und der Rückstand mittels präparativer HPLC gereinigt. Es werden 8.8 mg (22% d. Th.) der Titelverbindung als gelber Feststoff erhalten.
LC-MS (Methode 3): Rₜ = 2.03 min; [M+H]⁺ = 404.

### Beispiel 34

### Cyclobutylmethyl 5-cyano-2,6-dimethyl-4-(2-methyl-4-oxo-4H-chromen-8-yl)-1,4-dihydropyridin-3-carboxylat

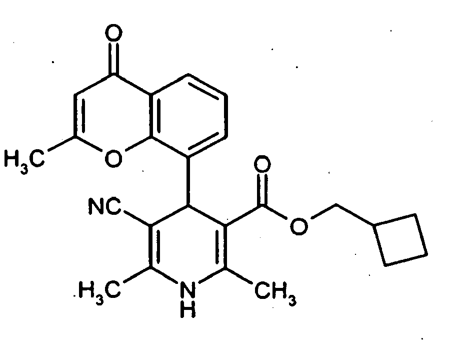

### Stufe 34a):

### Cyclobutylmethyl 3-oxobutanoat

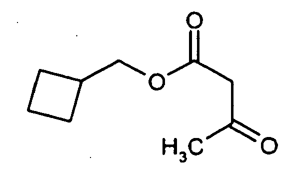

4.61 ml (35.17 mmol) 2,2,6-Trimethyl-1,3-dioxin-4-on und 3.32 ml (35.17 mmol) Cyclobutylmethanol werden in Toluol (20 ml) unter Argon 4 h lang unter Rückfluss gerührt. Anschließend wird das Lösungsmittel im Vakuum entfernt. Man erhält 7.51 g eines gelben Öls, das ohne weitere Reinigung eingesetzt wird.
¹H-NMR (300 MHz, DMSO-d₆): δ = 1.65-1.92 (m, 6H), 2.17 (s, 3H), 2.36 (m, 1H), 3.60 (s, 2H), 4.03 (d, 2H).

### Stufe 34b):

### Cyclobutylmethyl 2-[(2-methyl-4-oxo-4H-chromen-8-yl)methylen]-3-oxobutanoat

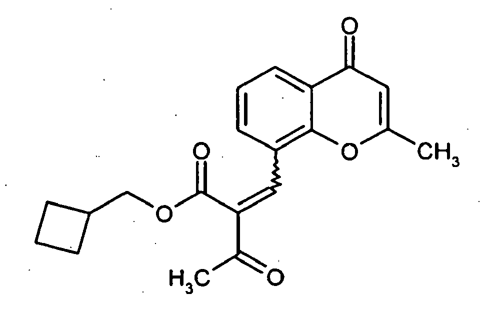

700 mg (3.72 mmol) 2-Methyl-4-oxo-4*H*-chromen-8-carbaldehyd, 760 mg (4.46 mmol) Cyclobutylmethyl 3-oxobutanoat, 53 µl (0.93 mmol) Essigsäure und 92 µl (0.93 mmol) Piperidin in 25 ml wasserfreiem Dichlormethan werden nach Zugabe von 4Å-Molekularsieb (1.5 g) 24 h lang unter Rückfluß erhitzt. Nach Abkühlen wird die Suspension abgesaugt und das Filtrat nacheinander mit gesättigter Natriumhydrogencarbonat- und Natriumchlorid-Lösung gewaschen. Die organische Phase wird über Magnesiumsulfat getrocknet und eingeengt. Der Rückstand wird durch präparative HPLC gereinigt. Man erhält 962 mg (76% d. Th.) der Titelverbindung als *E*/*Z*-Gemisch.
LC-MS (Methode 1): Rₜ = 2.12 und 2.29 min; [M+H]⁺ = 341.

### Stufe 34c):

### Cyclobutylmethyl 5-cyano-2,6-dimethyl-4-(2-methyl-4-oxo-4H-chromen-8-yl)-1,4-dihydropyridin-3-carboxylat

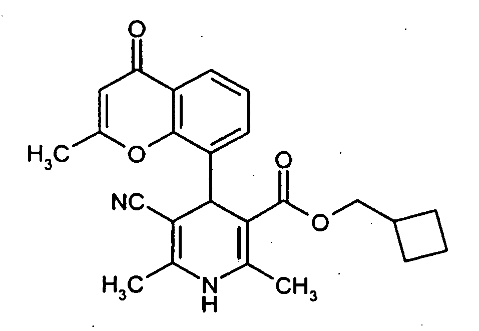

66 mg (0.19 mmol) Cyclobutylmethyl 2-[(2-methyl-4-oxo-4*H*-chromen-8-yl)methylen]-3-oxobutanoat werden mit 16 mg (0.19 mmol) 3-Aminobut-2-ennitril in 3 ml Ethanol gelöst und unter Argon 24 h lang unter Rückfluß erhitzt. Man lässt die Suspension abkühlen, saugt ab und wäscht den verbleibenden Feststoff mit Methanol. Es werden 45 mg (57% d. Th.) der Titelverbindung als weißer Feststoff erhalten.
LC-MS (Methode 2): Rₜ = 2.37 min; [M+H]⁺ = 404
¹H-NMR (300 MHz, DMSO-d₆): δ = 1.25 (m, 1H), 1.35 (m, 1H), 1.47 (m, 1H), 1.64 (m, 3H), 1.98 (s, 3H), 2.26 (m, 1H), 2.36 (s, 3H), 2.40 (s, 3H), 3.76 (m, 2H), 5.18 (s, 1H), 6.28 (s, 1H), 7.42 (t, 1H), 7.51 (t, 1H), 7.88 (d, 2H), 9.35 (s, 1H).

### Beispiel 35

### Isopropyl 5-cyano-6-methyl-4-(2-methyl-4-oxo-4H-chromen-8-yl)-2-(trifluormethyl)-1,4-dihydropyridin-3-carboxylat

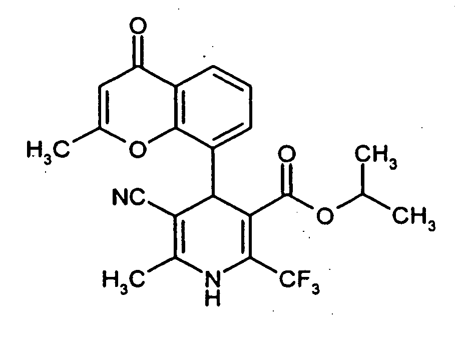

100 mg (0.53 mmol) 2-Methyl-4-oxo-4*H*-chromen-8-carbaldehyd werden mit 105 mg (0.53 mmol) Isopropyl 4,4,4-trifluor-3-oxobutanoat, 43.6 mg (0.53 mmol) 3-Aminocrotonsäurenitril und 46 µl (0.79 mmol) Essigsäure in 5 ml 2-Propanol gelöst und unter Argon 4 h lang unter Rückfluss erhitzt. Das Lösungsmittel wird im Vakuum entfernt und der Rückstand mittels präparativer HPLC aufgereinigt. Das hierbei erhaltene Hauptprodukt wird in Essigsäure über Nacht unter Rückfluss gerührt. Die Lösung wird eingeengt und der Rückstand aus Diethylether umkristallisiert. Es werden 75 mg (33% d. Th.) der Titelverbindung als weißer Feststoff erhalten.
LC-MS (Methode 1): Rₜ = 2.21 min; [M+H]⁺ = 433
¹H-NMR (300 MHz, CDCl₃): δ = 0.99 (d, 3H), 1.02 (d, 3H), 2.17 (s, 3H), 2.43 (s, 3H), 4.87 (m, 1H), 5.38 (s, 1H), 6.21 (s, 1H), 6.26 (br. s, 1H), 7.37 (t, 1H), 7.49 (dd, 1H), 8.14 (dd, 1H).

### Beispiel 36

### Isopropyl 5-cyano-2,6-dimethyl-4-(2-methyl-4-oxo-4H-chromen-8-yl)-1,4-dihydropyridin-3-carboxylat

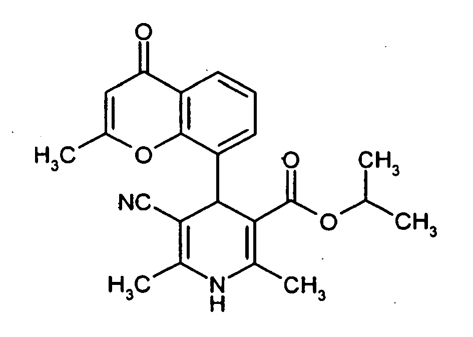

100 mg (0.53 mmol) 2-Methyl-4-oxo-4*H*-chromen-8-carbaldehyd werden mit 55.8 mg (0.53 mmol) Natrium-1-cyanoprop-1-en-2-olat, 76 mg (0.53 mmol) 3-Aminocrotonsäure-isopropylester und 30 µl (0.53 mmol) Essigsäure in 3 ml 2-Propanol gelöst und unter Argon 4 h lang unter Rückfluß erhitzt. Das Lösungsmittel wird im Vakuum entfernt und der Rückstand mittels präparativer HPLC gereinigt. Es werden 93 mg (46% d. Th.) der Titelverbindung als gelber Feststoff erhalten.
LC-MS (Methode 2): Rₜ = 2.28 min; [M+H]⁺ = 379
¹H-NMR (300 MHz, DMSO-d₆): δ= 0.64 (d, 3H), 1.04 (d, 3H), 1.99 (s, 3H), 2.31 (s, 3H), 2.39 (s, 3H), 4.65 (m, 1H), 5.12 (s, 1H), 6.27 (s, 1H), 7.43 (t, 1H), 7.53 (dd, 1H), 7.88 (dd, 1H), 9.18 (s, 1H).

### Beispiel 37

### 5-(3-Cyclobutylpropanoyl)-2,6-dimethyl-4-(2-methyl-4-oxo-4H-chromen-8-yl)-1,4-dihydropyridin-3-carbonitril

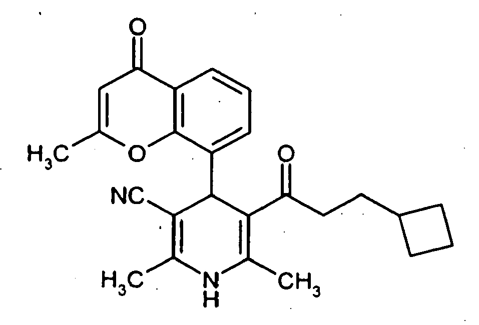

100 mg (0.53 mmol) 2-Methyl-4-oxo-4*H*-chromen-8-carbaldehyd werden mit 55.8 mg (0.53 mmol) Natrium-1-cyanoprop-1-en-2-olat, 88.8 mg (0.53 mmol) 5-Amino-1-cyclobutylhex-4-en-3-on (Beispiel 15, Stufe a) und 30 µl (0.53 mmol) Essigsäure in 3 ml 2-Propanol gelöst und unter Argon 4 h lang unter.Rückfluß erhitzt. Das Lösungsmittel wird im Vakuum entfernt und der Rückstand mittels präparativer HPLC gereinigt. Es werden 61 mg (28% d. Th.) der Titelverbindung als gelber Feststoff erhalten.
LC-MS (Methode 2): Rₜ = 2.39 min; [M+H]⁺ = 403
¹H-NMR (300 MHz, DMSO-d₆): δ = 1.22-1.46 (m, 5H), 1.60-1.85 (m, 5H), 1.95-2.14 (m, 4H), 2.31 (s, 3H), 2.39 (s, 3H), 5.25 (s, 1H), 6.28 (s, 1H), 7.42 (t, 1H), 7.49 (dd, 1H), 7.89 (dd, 1H), 9.28 (s, 1H).

### Beispiel 38

### 2,2,2-Trifluor-1-methylethyl 5-cyano-2,6-dimethyl-4-(2-methyl-4-oxo-4H-chromen-8-yl)-1,4-dihydropyridin-3-carboxylat

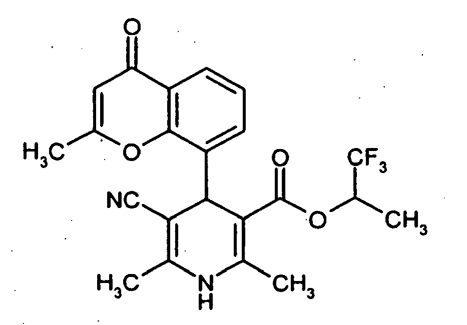

60 mg (0.31 mmol) 2-Methyl-4-oxo-4*H*-chromen-8-carbaldehyd werden mit 63 mg (0.32 mmol) 2,2,2-Trifluor-1-methylethyl-3-oxobutanoat, 26 mg (0.32 mmol) 3-Aminocrotonsäurenitril und 18 µl (0.32 mmol) Essigsäure in 2 ml 2-Propanol gelöst und unter Argon 4 h lang unter Rückfluß erhitzt. Das Lösungsmittel wird im Vakuum entfernt und der Rückstand mittels präparativer HPLC gereinigt. Es werden 89 mg (64% d. Th.) der Titelverbindung als gelber Feststoff erhalten.
LC-MS (Methode 3): Rₜ = 2.09 min; [M+H]⁺ = 433
¹H-NMR (300 MHz, DMSO-d₆): δ = 0.74 (d, 1.5H), 1.25 (d, 1.5H), 2.01 (s, 3H), 2.37 (t, 6H), 5.13 (s, 0.5H), 5.14-5.26 (m, 1H), 5.19 (s, 0.5H), 6.26 (s, 0.5H), 6.28 (s, 0.5H), 7.40 (t, 0.5H), 7.43 (t, 0.5H), 7.54 (dd, 0.5H), 7.57 (dd, 0.5H), 7.87 (dd, 0.5H), 7.90 (dd, 0.5H), 9.53 (s, 0.5H), 9.54 (s, 0.5H).

### Beispiel 39

### Ethyl (4S)-5-cyano-2,6-dimethyl-4-(2-methyl-4-oxo-4H-chromen-8-yl)-1,4-dihydropyridin-3-carboxylat

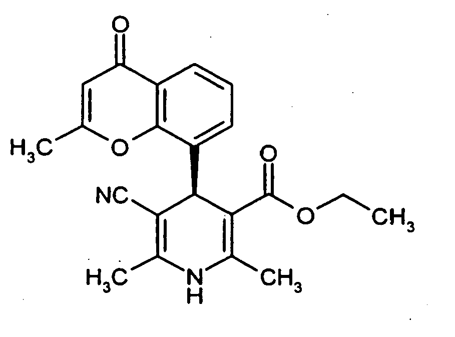

536 mg (1.45 mmol) racemisches Ethyl 5-cyano-2,6-dimethyl-4-(2-methyl-4-oxo-4*H*-chromen-8-yl)-1,4-dihydropyridin-3-carboxylat (Beispiel 7) werden mittels präparativer HPLC an chiraler Phase in die Enantiomeren getrennt [Säule: Chiralpak AS-H, 250 mm x 4.6 mm; Eluent: Isohexan/ Ethanol 3:1 (v/v) + 0.2% Diethylamin; Fluss: 1 ml/min; UV-Detektion: 220 nm]:

### Enantiomer 1 (mit 4R-Konfiguration):

Ausbeute: 197 mg
Rₜ =5.24 min.

### Enantiomer 2 (mit 4S-Konfiguration):

Ausbeute: 193 mg
Rₜ = 6.49 min; >99.5% ee.

### Beispiel 40

### Propyl (4S)-5-cyano-2,6-dimethyl-4-(2-methyl-4-oxo-4H-chromen-8-yl)-1,4-dihydropyridin-3-carboxylat

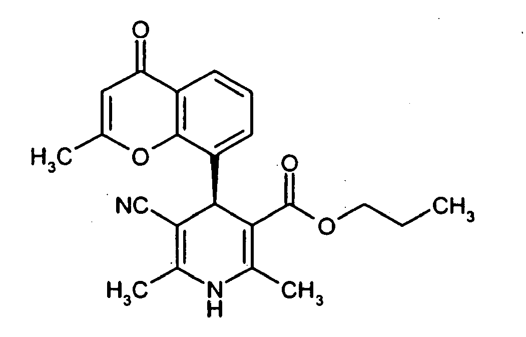

430 mg (1.13 mmol) racemisches Propyl 5-cyano-2,6-dimethyl-4-(2-methyl-4-oxo-4*H*-chromen-8-yl)-1,4-dihydropyridin-3-carboxylat (Beispiel 4) werden mittels präparativer HPLC an chiraler Phase in die Enantiomeren getrennt [Säule: Chiralpak AD-H, 250 mm x 4.6 mm; Eluent: Isohexan/Ethanol 3:1 (v/v) + 0.2% Diethylamin; Fluss: 1 ml/min; UV-Detektion: 220 nm]:

### Enantiomer 1 (mit 4R-Konfiguration):

Ausbeute: 151 mg
Rₜ = 4.19 min.

### Enantiomer 2 (mit 4S-Konfiguration):

Ausbeute: 140 mg
Rₜ = 6.00 min; >99.5% ee.

### Beispiel 41

### 5-(Cyclopentylacetyl)-2,6-dimethyl-4-(2-methyl-4-oxo-4H-chromen-8-yl)-1,4-dihydropyridin-3-carbonitril

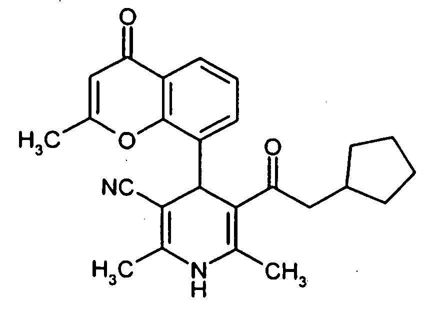

### Stufe 41a):

### 4-Amino-1-cyclopentylpent-3-en-2-on

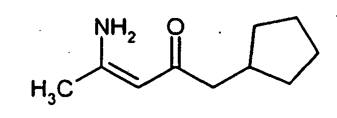

Die Herstellung erfolgt analog zu Beispiel 14 (Stufe 14a) ausgehend von 5-(Cyclopentylmethyl)-3-methylisoxazol [erhältlich analog zu C. Kashima et al., Bull. Chem. Soc. Jpn. 46, 310-313 (1973)].
¹H-NMR (300 MHz, DMSO-d₆): δ = 1.07 (m, 2H), 1.50 (m, 4H), 1.67 (m, 2H), 1.81 (s, 3H), 2.11 (m, 3H), 4.87 (s, 1H), 7.37 (br. s, 1H), 9.51 (br. s, 1H).

### Stufe 41b):

### 5-(Cyclopentylacetyl)-2,6-dimethyl-4-(2-methyl-4-oxo-4H-chromen-8-yl)-1,4-dihydropyridin-3-carbonitril

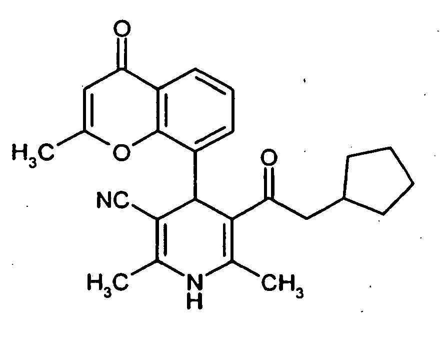

150 mg (0.79 mmol) 2-Methyl-4-oxo-4*H*-chromen-8-carbaldehyd werden mit 84 mg (0.79 mmol) Natrium-1-cyanoprop-1-en-2-olat, 133 mg (0.79 mmol) 4-Amino-1-cyclopentyl-pent-3-en-2-on und 68 µl (1.19 mmol) Essigsäure in 4 ml 2-Propanol gelöst und unter Argon 4 h lang unter Rückfluß erhitzt. Nach dem Abkühlen wird die Suspension abgesaugt und der verbleibende Feststoff mit Diethylether (20 ml) gewaschen. Es werden 240 mg (75% d. Th.) der Titelverbindung als weißer Feststoff erhalten.
LC-MS (Methode 2): Rₜ = 2.30 min; [M+H]⁺ = 403
¹H-NMR (300 MHz, CDCl₃): δ = 0.79 (m, 1H), 0.97 (m, 1H), 1.26 (m, 1H), 1.46 (m, 4H), 1.58 (m, 1H), 1.74 (m, 1H), 2.09 (s + m, 4H), 2.40 (s, 3H), 2.49 (s + m, 4H), 5.37 (s, 1H), 5.83 (br. s, 1H), 6.22 (s, 1H), 7.32 (t, 1H), 7.40 (dd, 1H), 8.10 (dd, 1H).

### B. Bewertung der pharmakotosischen Wirksamkeit

### Abkürzungen:

- DMEM: Dulbecco's Modified Eagle Medium
- DNA: Desoxyribo Nucleic Acid
- FCS: Fetal Calf Serum
- HEPES: 4-(2-Hydroxyethyl)-1-piperazin-ethansulfonsäure
- PCR: Polymerase Chain Reaction

Die vorteilhaften pharmakologischen Eigenschaften der erfindungsgemäßen Verbindungen können in folgenden Assays gezeigt werden:

### 1. Zellulärer in vitro-Test zur Bestimmung der inhibitorischen MR-Aktivität und MR-Selektivität gegenüber anderen Steroidhormon-Rezeptoren

Die Identifizierung von Antagonisten des humanen Mineralokorticoid-Rezeptors (MR) sowie die Quantifizierung der Wirksamkeit der hier beschriebenen Verbindungen erfolgt mit Hilfe einer rekombinanten Zelllinie. Die Zelle leitet sich ursprünglich von einer Ovarepithelzelle des Hamsters ab (Chinese Hamster Ovary, CHO K1, ATCC: American Type Culture Collection, VA 20108, USA).

In dieser CHO K1-Zelllinie wird ein etabliertes Chimärensystem verwendet, in dem die Liganden-Bindungsdomänen humaner Steroidhormon-Rezeptoren an die DNA-Bindungsdomäne des Hefe-Transkriptionsfaktors GAL4 fusioniert werden. Die so entstehenden GAL4-Steroidhormonrezeptor-Chimären werden in den CHO-Zellen mit einem Reporterkonstrukt co-transfiziert und stabil exprimiert.

### Klonierungen:

Zur Generierung der GAL4-Steroidhormonrezeptor-Chimären wird die GAL4-DNA-Bindungsdomäne (Aminosäuren 1-147) aus dem Vektor pFC2-dbd (Fa. Stratagene) mit den PCR-amplifizierten Liganden-Bindungsdomänen des Mineralokorticoid-Rezeptors (MR, Aminosäuren 734-985), des Glucokorticoid-Rezeptors (GR, Aminosäuren 443-777), des Progesteron-Rezeptors (PR, Aminosäuren 680-933) und des Androgen-Rezeptors (AR, Aminosäuren 667-919) in den Vektor pIRES2 (Fa. Clontech) kloniert. Das Reporterkonstrukt, welches fünf Kopien der GAL4-Bindestelle, vorgeschaltet vor einem Thymidinkinase-Promotor enthält, führt zur Expression der Firefly-Luciferase (*Photinus pyralis*) nach Aktivierung und Bindung der GAL4-Steroidhormonrezeptor-Chimären durch die jeweiligen spezifischen Agonisten Aldosteron (MR), Dexamethason (GR), Progesteron (PR) und Dihydrotestosteron (AR).

### Testablauf:

Die MR-, GR-, PR- und AR-Zellen werden am Tag vor dem Test in Medium (Optimem, 2.5% FCS, 2 mM Glutamin, 10 mM HEPES) in 96- (oder 384- bzw. 1536-) Loch-Mikrotiterplatten ausplattiert und in einem Zellinkubator (96% Luftfeuchtigkeit, 5% v/v CO₂, 37°C) gehalten. Am Testtag werden die zu prüfenden Substanzen in oben genanntem Medium aufgenommen und zu den Zellen hinzugegeben. Etwa 10 bis 30 Minuten nach Zugabe der Testsubstanzen werden die jeweiligen spezifischen Agonisten der Steroidhormon-Rezeptoren hinzugesetzt. Nach einer weiteren Inkubationszeit von 5 bis 6 Stunden wird die Luciferaseaktivität mit Hilfe einer Videokamera gemessen. Die gemessenen relativen Lichteinheiten ergeben in Abhängigkeit von der Substanzkonzentration eine sigmoide Stimulationskurve. Die Berechnung der IC₅₀-Werte erfolgt mit Hilfe des Computerprogramms GraphPad PRISM (Version 3.02).

Tabelle A zeigt die IC₅₀-Werte (MR) repräsentativer Beispielverbindungen:

**Tabelle A**

| **Beispiel-Nr.** | **MR IC₅₀ [nM]** |
|---|---|
| 13 | 127 |
| 14 | 195 |
| 15 | 93 |
| 18 | 22 |
| (Enantiomer 2) | |
| 29 | 193 |
| 30 | 89 |
| (Enantiomer 2) | |
| 32 | 105 |
| 41 | 290 |

### 2. In vitro-Test zur Bestimmung möglicher Bindungsaktivität am L-Typ Calcium-Kanal

Membranpräparationen des zerebralen Cortex von Wistar-Ratten dienen als Ausgangsmaterial für einen radioaktiven Bindungstest, der als Standardassay in der Literatur ausführlich beschrieben ist [Ehlert, F.J., Roeske, W.R., Itoga E., Yamamura, H.I., Life Sci. 30, 2191-2202 (1982); Gould, R.J., Murphy, K.M.M., Snyder, S.H., Proc. Natl. Acad. Sci. U.S.A. 79, 3656-3660] und von kommerziellen Anbietern (z.B. Fa. MDS Pharma Services) im Rahmen von Auftragsuntersuchungen verwendet wird. In diesem Bindungsassay werden Verdünnungsreihen der Testverbindungen in DMSO typischerweise für 90 Minuten bei 25°C in einem 50 mM TrisHCl-Puffer, pH 7.7, mit den Membranpräparationen und dem Tritium-markierten Liganden Nitrendipin (0.1 nM) inkubiert und die spezifische Bindung der Testverbindungen über Quantifizierung des spezifisch verdrängten, radioaktiv markierten Liganden bestimmt. IC₅₀-Werte werden über eine nicht-lineare Regressionsanalyse ermittelt.

In diesem L-Typ Calcium-Kanal-Bindungsassay wird für einen klassischen Calcium-Antagonisten vom Dihydropyridin-Typ, wie z.B. Nitrendipin, ein IC₅₀-Wert von 0.3 nM bestimmt, während untersuchte Beispiele der hier beschriebenen erfindungsgemäßen Verbindungen IC₅₀-Werte in der Grössenordnung von 0.8 bis 5 µM und somit eine mindestens um den Faktor 1000 verminderte Affinität zum L-Typ Calcium-Kanal aufweisen. Verbindungen mit einer solchen geringen Residualbindungsaffinität zum L-Typ Calcium-Kanal zeigen in vivo keine ausgeprägten hämodynamischen Effekte mehr, die über den L-Typ Calcium-Kanal vermittelt sind.

### 3. In vitro-Test zur funktionellen Charakterisierung möglicher Calcium-Kanalagonistischer oder -antagonistischer Wirkungen von Testverbindungen: Kaliumchlorid-induzierte Stimulation der isolierten Kaninchenaorta

Die frisch isolierte Thoraxaorta von männlichen New Zeeland White-Kaninchen wird entfernt und vom umgebenden Gewebe gereinigt. Dann werden Aortenringe von 2 mm Länge unter einer Anfangsspannung von 4 g in 10 ml-Organbäder mit auf 37°C erwärmter Krebs-Henseleit-Lösung gebracht. Kontraktionen von 40 mM KCl (submaximale Kontraktion) und 15 mM KCl (minimale Kontraktion) werden viermal im Abstand von 45 Minuten ausgelöst, um die Gefäße zu trainieren und eine stabile Ruhespannung zu erzeugen. Jeder Kontraktion folgt eine Serie von elf Spülzyklen und eine Ruhezeit von 30 Minuten bei vorheriger Nachspannung. Nach den vier Vorläufen erfolgt ohne weitere Nachspannung jeweils zu Beginn der Ruhephase die Zugabe der Testsubstanzen in die Organbäder. Die Konzentration der Testsubstanzen erhöht sich bei den vier folgenden Kontraktionen jeweils um den Faktor 10. Zur Wirkungsberechnung wird die Differenz zwischen der Basisspannung und dem vierten Vorlauf-Kontraktionswert als 100% gesetzt, die folgenden Kontraktionsspitzen beziehen sich auf diesen Wert. Diese Versuchsdurchführung ermöglicht die Differenzierung von Calcium-agonistischer (leichte Steigerung bei submaximaler Kontraktion, stärkere Steigerung bei minimaler Kontraktion) und Calcium-antagonistischer Substanzwirkung (Senkung bei submaximaler Kontraktion, stärkere Senkung bei minimaler Kontraktion).

In diesem funktionellen Assay am isolierten Organ wird für einen klassischen Calcium-Antagonisten vom Dihydropyridin-Typ, wie z.B. Nifedipin, ein IC₅₀-Wert von 0.1 nM bis 0.4 nM gemessen, während untersuchte Beispiele der hier beschriebenen erfindungsgemäßen Verbindungen IC₅₀-Werte in der Grössenordnung von 4 bis 25 µM und somit eine mindestens um den Faktor 10000 verminderte Affinität zum L-Typ Calcium-Kanal aufweisen. Verbindungen mit einer solchen geringen Residualbindungsaffinität zum L-Typ Calcium-Kanal zeigen *in vivo* keine ausgeprägten hämodynamischen Effekte mehr, die über den L-Typ Calcium-Kanal vermittelt sind.

### 4. In vivo-Test zum Nachweis der kardiovaskulären Wirkung: Diurese-Untersuchunegen an wachen Ratten in Stoffwechselkäfigen

Wistar-Ratten (250-350 g Körpergwicht) werden mit freiem Zugang zu Futter (Altromin) und Trinkwasser gehalten. Ab ca. 72 Stunden vor Versuchsbeginn erhalten die Tiere anstelle des normalen Futters ausschließlich Kochsalz-reduziertes Futter mit einem Gehalt von 0.02% Natriumchlorid (ssniff R/M-H, 10 mm mit 0.02% Na, S0602-E081, Fa. ssniff Spezialdiäten GmbH, D-59494 Soest). Während des Versuches werden die Tiere für ca. 24 Stunden einzeln in für Ratten dieser Gewichtsklasse geeigneten Stoffwechselkäfigen (Fa. Tecniplast Deutschland GmbH, D-82383 Hohenpeißenberg) mit freiem Zugang zu Kochsalz-reduziertem Futter und Trinkwasser gehalten. Am Versuchsbeginn wird den Tieren die zu prüfende Substanz in einem Volumen von 0.5 ml/kg Körpergewicht eines geeigneten Lösemittels mittels einer Schlundsonde in den Magen verabreicht. Als Kontrolle dienende Tiere erhalten nur Lösemittel. Kontrollen und Substanztestungen werden am selben Tag parallel durchgeführt. Kontrollgruppen und Substanz-Dosisgruppen bestehen aus jeweils 3 bis 6 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Aus den Messwerten wird der Natrium/Kalium-Quotient als ein Maß für die Substanzwirkung berechnet. Die Messintervalle betragen typischerweise den Zeitraum bis zu 8 Stunden nach Versuchsbeginn (Tagintervall) und den Zeitraum von 8 bis 24 Stunden nach Versuchsbeginn (Nachtintervall). In einer abgewandelten Versuchsanordnung wird der Urin während des Tagintervalls im Abstand von zwei Stunden gesammelt und gemessen. Um eine hierfür ausreichende Urinmenge zu erhalten, wird den Tieren zu Versuchsbeginn und dann im Abstand von zwei Stunden per Schlundsonde eine definierte Menge Wasser zugeführt.

### C. Ausführungsbeispiele für pharmazeutische Zusammensetzugen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 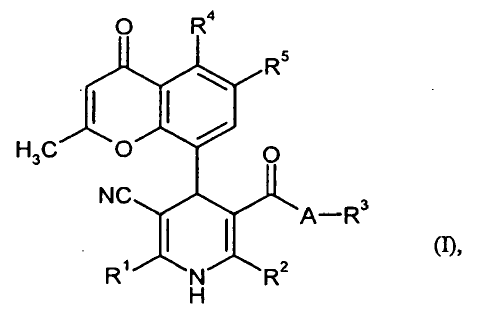 in welcher
R¹ und R² gleich oder verschieden sind und unabhängig voneinander für (C₁-C₄)-Alkyl, Trifluormethyl, Cyclopropyl oder Cyclobutyl stehen,
A für eine Bindung oder für O steht,
R³ für (C₃-C₇)-Cycloalkyl oder für (C₁-C₆)-Alkyl, das mit (C₃-C₇)-Cycloalkyl oder ein- bis dreifach mit Fluor substituiert sein kann, steht,
R⁴ für Wasserstoff, Halogen, Cyano, Nitro, Trifluormethyl, (C₁-C₄)-Alkyl oder (C₁- C₄)-Alkoxy steht
und
R⁵ für Wasserstoff oder Fluor steht,
sowie ihre pharmazeutisch verträglichen salze, Solvate und Solvate der Salze.

2. Verbindung der Formel (I) nach Anspruch 1, in welcher.
R¹ und R² gleich oder verschieden sind und für Methyl oder Trifluormethyl stehen,
A für eine Bindung oder für O steht,
R³ für (C₃-C₅)-Cycloalkyl oder für (C₁-C₆)-Alkyl, das mit (C₃-C₅)-Cycloalkyl oder ein- bis dreifach mit Fluor substituiert sein kann, steht,
R⁴ für Wasserstoff, Fluor, Chlor, Cyano, Nitro oder Methyl steht
und
R⁵ für Wasserstoff oder Fluor steht,
sowie ihre pharmazeutisch verträglichen Salze, Solvate und Solvate der Salze.

3. Verbindung der Formel (I) nach Anspruch 1 oder 2, in welcher
R¹ für Methyl oder Trifluormethyl steht,
R² für Methyl steht,
A für O steht,
R³ für Ethyl, 2,2,2-Trifluorethyl, n-Propyl, Isopropyl, 1-(Trifluormethyl)-ethyl, tert.- Butyl, Cyclobutyl, Cyclopentyl, Cyclopropylmethyl oder Cyclobutylmethyl steht,
R⁴ für Wasserstoff, Fluor, Chlor oder Nitro steht
und
R⁵ für Wasserstoff oder Fluor steht,
sowie ihre pharmazeutisch verträglichen Salze, Solvate und Solvate der Salze.

4. Verbindung der Formel (I) nach Anspruch 1 oder 2, in welcher
R¹ für Methyl oder Trifluormethyl steht,
R² für Methyl steht,
A für eine Bindung steht,
R³ für Isobutyl, Isopentyl, Cyclobutylmethyl, Cyclopentylmethyl, 2-(Cyclopropyl)- ethyl, 2-(Cyclobutyl)-ethyl oder 2-(Cyclopentyl)-ethyl steht,
R⁴ für Wasserstoff, Fluor, Chlor oder Nitro steht
und
R⁵ für Wasserstoff oder Fluor steht,
sowie ihre pharmazeutisch verträglichen Salze, Solvate und Solvate der Salze.

5. Verfahren zur Herstellung von Verbindungen der Formel (I), wie in den Ansprüchen 1 bis 4 definiert, **dadurch gekennzeichnet, dass** man Verbindungen der Formel (II) 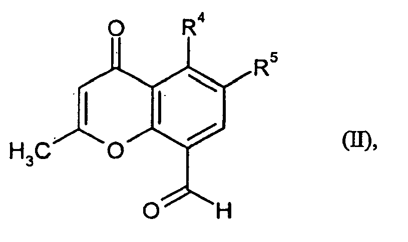 in welcher R⁴ und R⁵ jeweils die in den Ansprüchen 1 bis 4 angegebenen Bedeutungen haben,
entweder
[A] in einem einstufigen Verfahren (Eintopf-Reaktion) mit einer Verbindung der Formel (III) 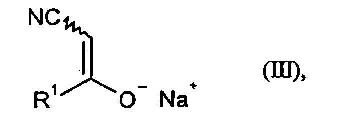 in welcher R¹ die in den Ansprüchen 1 bis 4 angegebenen Bedeutungen hat,
und einer Verbindung der Formel (IV) 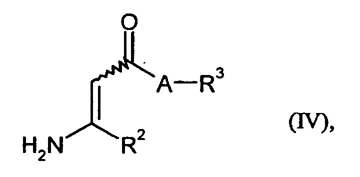 in welcher A, R² und R³ jeweils die in den Ansprüchen 1 bis 4 angegebenen Bedeutungen haben, umsetzt
oder
[B] in einem einstufigen Verfahren (Eintopf-Reaktion) mit einer Verbindung der Formel (V) 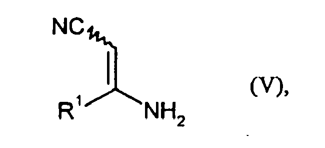 in welcher R¹ die in den Ansprüchen 1 bis 4 angegebenen Bedeutungen hat,
und einer Verbindung der Formel (VI) 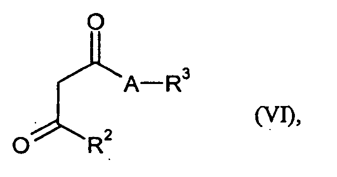 in welcher A, R² und R³ jeweils die in den Ansprüchen 1 bis 4 angegebenen Bedeutungen haben, umsetzt
oder
[C] in einem zweistufigen Verfahren zunächst mit einer Verbindung der Formel (III) in Verbindungen der Formel (VII) 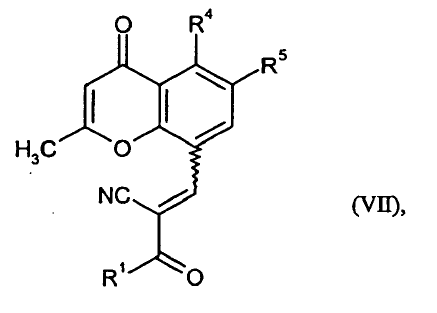 in welcher R¹, R⁴ und R⁵ jeweils die in den Ansprüchen 1 bis 4 angegebenen Bedeutungen haben,
überführt und diese dann in einem zweiten Schritt mit einer Verbindung der Formel (IV) umsetzt
oder
[D] in einem zweistufigen Verfahren zunächst mit einer Verbindung der Formel (VI) in Verbindungen der Formel (VIII) 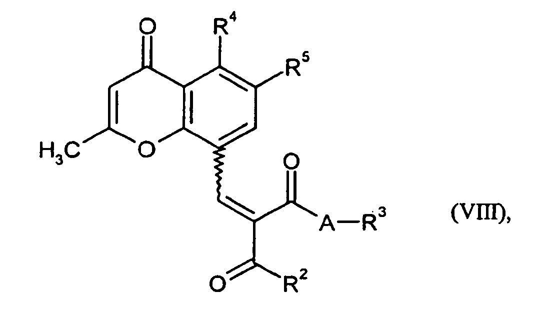 in welcher A, R², R³, R⁴ und R⁵ jeweils die in den Ansprüchen 1 bis 4 angegebenen Bedeutungen haben,
überführt und diese dann in einem zweiten Schritt mit einer Verbindung der Formel (V) umsetzt.

6. Verbindung der Formel (I), wie in einem der Ansprüche 1 bis 4 definiert, zur Behandlung und/oder Prophylaxe von Krankheiten.

7. Verwendung einer Verbindung der Formel (I), wie in einem der Ansprüche 1 bis 4 definiert, zur Herstellung eines Arzneimittels zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

8. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 4 definiert, in Kombination mit einem weiteren Wirkstoff ausgewählt aus der Gruppe bestehend aus ACE-Inhibitoren, Renin-Inhibitoren, Angiotensin II-Rezeptor-Antagonisten, Beta-Blocker, Acetylsalicylsäure, Diuretika, Kalium-Supplements, Calcium-Antagonisten, Statine, Digitalis (Digoxin)-Derivate, Calcium-Sensitizer, Nitrate, Antikoagulantien, Antiarrhythmika, Vasodilatatoren sowie Thrombolytika.

10. Arzneimittel nach Anspruch 8 oder 9 zur Behandlung und/oder Prophylaxe von Aldosteronismus, Bluthochdruck, chronischer Herzinsuffzienz, den Folgen eines Myokardinfarkts, Leberzirrhose, Niereninsuffizienz und Hirnschlag.

## Claims

1. Compound of the formula (I) 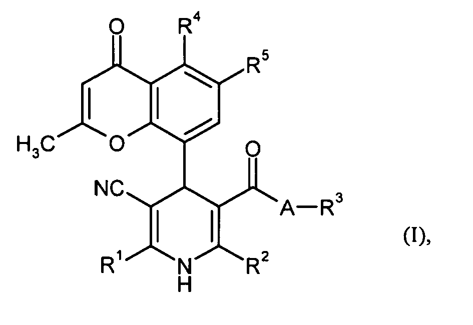 in which
R¹ and R² are identical or different and are independently of one another (C₁-C₄)- alkyl, trifluoromethyl, cyclopropyl or cyclobutyl,
A is a bond or is O,
R³ is (C₃-C₇)-cycloalkyl or is (C₁-C₆)-alkyl which may be substituted by (C₃-C₇)-cycloalkyl or once to three times by fluorine,
R⁴ is hydrogen, halogen, cyano, nitro, trifluoromethyl, (C₁-C₄)-alkyl or (C₁- C₄)-alkoxy
and
R⁵ is hydrogen or fluorine,
and the pharmaceutically acceptable salts, solvates and solvates of the salts thereof.

2. Compound of the formula (I) according to Claim 1, in which
R¹ and R² are identical or different and are methyl or trifluoromethyl,
A is a bond or is O,
R³ is (C₃-C₅)-cycloalkyl or is (C₁-C₆)-alkyl which may be substituted by (C₃-C₅)-cycloalkyl or once to three times by fluorine,
R⁴ is hydrogen, fluorine, chlorine, cyano, nitro or methyl
and
R⁵ is hydrogen or fluorine,
and the pharmaceutically acceptable salts, solvates and solvates of the salts thereof.

3. Compound of the formula (I) according to Claim 1 or 2, in which
R¹ is methyl or trifluoromethyl,
R² is methyl,
A is O,
R³ is ethyl, 2,2,2-trifluoroethyl, n-propyl, isopropyl, 1-(trifluoromethyl)ethyl, tert-butyl, cyclobutyl, cyclopentyl, cyclopropylmethyl or cyclobutylmethyl,
R⁴ is hydrogen, fluorine, chlorine or nitro
and
R⁵ is hydrogen or fluorine,
and the pharmaceutically acceptable salts, solvates and solvates of the salts thereof.

4. Compound of the formula (I) according to Claim 1 or 2, in which
R¹ is methyl or trifluoromethyl,
R² is methyl,
A is a bond,
R³ is isobutyl, isopentyl, cyclobutylmethyl, cyclopentylmethyl, 2-(cyclopropyl)ethyl, 2-(cyclobutyl)ethyl or 2-(cyclopentyl)ethyl,
R⁴ is hydrogen, fluorine, chlorine or nitro
and
R⁵ is hydrogen or fluorine,
and the pharmaceutically acceptable salts, solvates and solvates of the salts thereof.

5. Process for preparing compounds of the formula (I) as defined in Claims 1 to 4, **characterized in that** compounds of the formula (II) 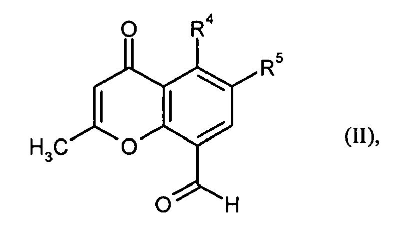 in which R⁴ and R⁵ each have the meanings indicated in Claims 1 to 4,
either
[A] are reacted in a one-stage process (one-pot reaction) with a compound of the formula (III) 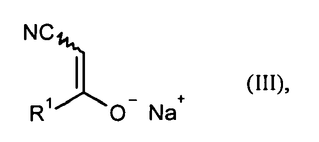 in which R¹ has the meanings indicated in Claims 1 to 4,
and a compound of the formula (IV) 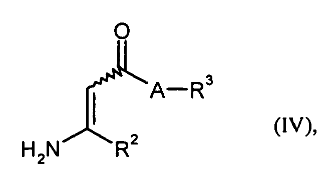 in which A, R² and R³ each have the meanings indicated in Claims 1 to 4
or
[B] are reacted in a one-stage process (one-pot reaction) with a compound of the formula (V) 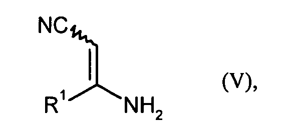 in which R¹ has the meanings indicated in Claims 1 to 4,
and a compound of the formula (VI) 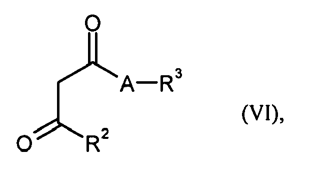 in which A, R² and R³ each have the meanings indicated in Claims 1 to 4
or
[C] are converted in a two-stage process firstly with a compound of the formula (III) into compounds of the formula (VII) 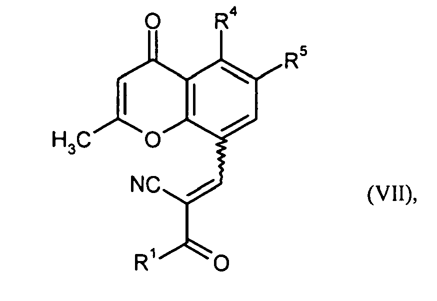 in which R¹, R⁴ and R⁵ each have the meanings indicated in Claims 1 to 4,
and the latter are then reacted in a second step with a compound of the formula (IV)
or
[D] are converted in a two-stage process firstly with a compound of the formula (VI) into compounds of the formula (VIII) 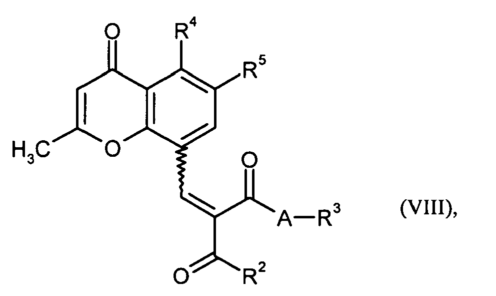 in which A, R², R³, R⁴ and R⁵ each have the meanings indicated in Claims 1 to 4,
and the latter are then reacted in a second step with a compound of the formula (V).

6. Compound of the formula (I) as defined in any of Claims 1 to 4 for the treatment and/or prophylaxis of diseases.

7. Use of a compound of the formula (I) as defined in any of Claims 1 to 4 for the manufacture of a medicament for the treatment and/or prophylaxis of aldosteronism, high blood pressure, chronic heart failure, the sequelae of a myocardial infarction, cirrhosis of the liver, renal failure and stroke.

8. Medicament comprising a compound of the formula (I) as defined in any of Claims I to 4 in combination with an inert, non-toxic, pharmaceutically suitable excipient.

9. Medicament comprising a compound of the formula (I) as defined in any of Claims 1 to 4 in combination with a further active ingredient selected from the group consisting of ACE inhibitors, renin inhibitors, angiotensin II receptor antagonists, beta blockers, acetylsalicylic acid, diuretics, potassium supplements, calcium antagonists, statins, digitalis (digoxin) derivatives, calcium sensitizers, nitrates, anticoagulants, antiarrhythmics, vasodilators, and thrombolytics.

10. Medicament according to Claim 8 or 9 for the treatment and/or prophylaxis of aldosteronism, high blood pressure, chronic heart failure, the sequelae of a myocardial infarction, cirrhosis of the liver, renal failure and stroke.

## Revendications

1. Composé de formule (I) 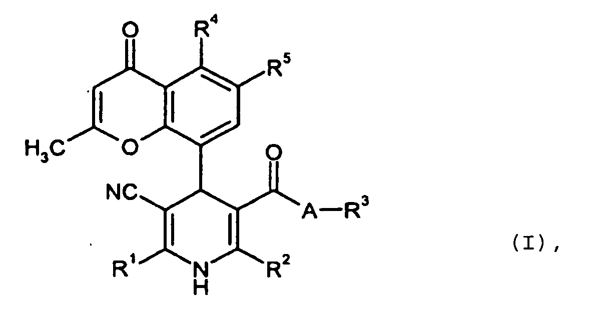 dans laquelle
R¹ et R² sont identiques ou différents et représentent, indépendamment l'un de l'autre, un groupe alkyle en C₁-C₄, trifluorométhyle, cyclopropyle ou cyclobutyle,
A représente une liaison ou O,
R³ représente un groupe cycloalkyle en C₃-C₇ ou alkyle en C₁-C₆, qui peut être substitué par cycloalkyle en C₃-C₇ ou une à trois fois par le fluor,
R⁴ représente un atome d'hydrogène ou d'halogène, un groupe cyano, nitro, trifluorométhyle, alkyle en C₁-C₄ ou alcoxy en C₁-C₄
et
R⁵ représente un atome d'hydrogène ou de fluor,
ainsi que ses sels, produits de solvatation et produits de solvatation des sels, pharmaceutiquement acceptables.

2. Composé de formule (I) selon la revendication 1, dans lequel
R¹ et R² sont identiques ou différents et représentent le groupe méthyle ou trifluorométhyle,
A représente une liaison ou O,
R³ représente un groupe cycloalkyle en C₃-C₅ ou alkyle en C₁-C₆, qui peut être substitué par cycloalkyle en C₃-C₅ ou une à trois fois par le fluor,
R⁴ représente un atome d'hydrogène, de fluor ou de chlore, le groupe cyano, nitro ou méthyle
et
R⁵ représente un atome d'hydrogène ou de fluor,
ainsi que ses sels, produits de solvatation et produits de solvatation des sels, pharmaceutiquement acceptables.

3. Composé de formule (I) selon la revendication 1 ou 2, dans lequel
R¹ représente le groupe méthyle ou trifluorométhyle,
R² représente le groupe méthyle,
A représente O,
R³ représente le groupe éthyle, 2,2,2-trifluoro- éthyle, n-propyle, isopropyle, 1-(trifluoro- méthyl)-éthyle, tert-butyle, cyclobutyle, cyclopentyle, cycopropylméthyle ou cyclobutyl- méthyle,
R⁴ représente un atome d'hydrogène, de fluor ou de chlore ou le groupe nitro
et
R⁵ représente un atome d'hydrogène ou de fluor,
ainsi que ses sels, produits de solvatation et produits de solvatation des sels, pharmaceutiquement acceptables.

4. Composé de formule (I) selon la revendication 1 ou 2, dans lequel
R¹ représente le groupe méthyle ou trifluorométhyle,
R² représente le groupe méthyle,
A représente une liaison,
R³ représente le groupe isobutyle, isopentyle, cyclobutylméthyle, cyclopentylméthyle, 2- (cyclopropyl) éthyle, 2-(cyclobutyl)-éthyle ou 2- (cyclopentyl)-éthyle,
R⁴ représente un atome d'hydrogène, de fluor ou de chlore ou le groupe nitro
et
R⁵ représente un atome d'hydrogène ou de fluor,
ainsi que ses sels, produits de solvatation et produits de solvatation des sels, pharmaceutiquement acceptables.

5. Procédé pour la préparation de composés de formule (I), tels que définis dans les revendications 1 à 4, **caractérisé en ce qu'**on fait réagir des composés de formule (II) 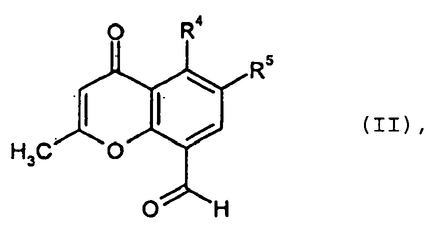 dans laquelle R⁴ et R⁵ ont chacun les significations indiquées dans les revendications 1 à 4,
soit
[A] dans une réaction en une étape (réaction en un seul récipient), avec un composé de formule (III) 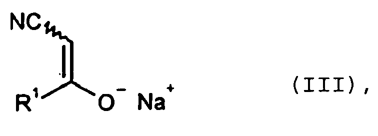 dans laquelle R¹ a les significations indiquées dans les revendications 1 à 4,
et un composé de formule (IV) 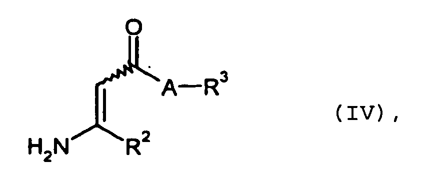 dans laquelle A, R² et R³ ont chacun les significations indiquées dans les revendications 1 à 4
soit
[B] dans un procédé en une étape (réaction en un seul récipient), avec un composé de formule (V) 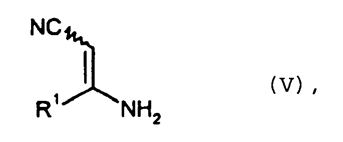 dans laquelle R¹ a les significations indiquées dans les revendications 1 à 4,
et un composé de formule (VI) 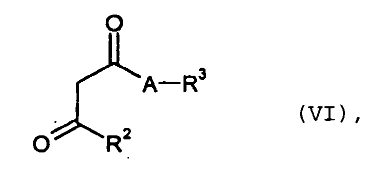 dans laquelle A, R² et R³ ont chacun les significations indiquées dans les revendications 1 à 4
ou
[C] dans un procédé en deux étapes, d'abord on les convertit, à l'aide d'un composé de formule (III), en des composés de formule (VII) 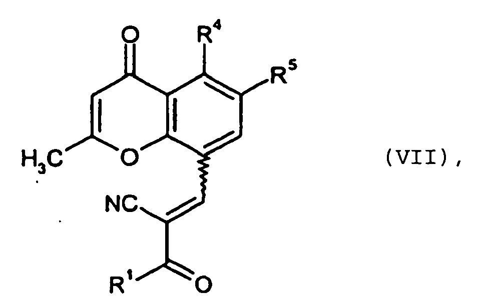 dans laquelle R¹, R⁴ et R⁵ ont chacun les significations indiquées dans les revendications 1 à 4,
et ensuite on fait réagir ceux-ci, dans une deuxième étape, avec un composé de formule (IV)
ou
[D] dans un procédé en deux étapes d'abord on les convertit, à l'aide d'un composé de formule (VI), en composés de formule (VIII) 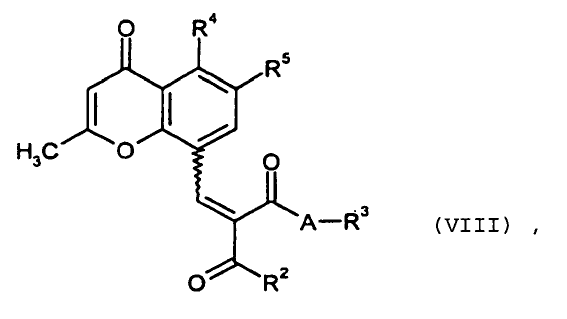 dans laquelle A, R², R³, R⁴ et R⁵ ont chacun les significations indiquées dans les revendications 1 à 4,
et ensuite on fait réagir ceux-ci, dans une deuxième étape, avec un composé de formule (V).

6. Composé de formule (I), tel que défini dans l'une quelconque des revendications 1 à 4, destiné au traitement et/ou à la prophylaxie de maladies.

7. Utilisation d'un composé de formule (I), tel que défini dans l'une quelconque des revendications 1 à 4, pour la fabrication d'un médicament destiné au traitement et/ou à la prophylaxie de l'hyperaldostéronisme, de l'hypertension artérielle, de l'insuffisance cardiaque chronique, des suites d'un infarctus du myocarde, de la cirrhose du foie, de l'insuffisance rénale et de l'accident vasculaire cérébral.

8. Médicament contenant un composé de formule (I), tel que défini dans l'une quelconque des revendications 1 à 4, en association avec un adjuvant inerte, non toxique, pharmaceutiquement approprié.

9. Médicament contenant un composé de formule (I), tel que défini dans l'une quelconque des revendications 1 à 4, en association avec une autre substance active choisie dans le groupe constitué par des inhibiteurs d'ACE, des inhibiteurs de rénine, des antagonistes de récepteurs de l'angiotensine II, des bêta-bloquants, l'acide acétylsalicylique, des diurétiques, des compléments de potassium, des antagonistes du calcium, des statines, des dérivés de digitale (digoxine), des sensibilisants au calcium, des nitrates, des anticoagulants, des antiarythmiques, des vasodilatateurs ainsi que des thrombolytiques.

10. Médicament selon la revendication 8 ou 9, destiné au traitement et/ou à la prophylaxie de l'hyperaldostéronisme, de l'hypertension artérielle, de l'insuffisance cardiaque chronique, des suites d'un infarctus du myocarde, de la cirrhose du foie, de l'insuffisance rénale et de l'accident vasculaire cérébral.
